# EUROPEAN PATENT APPLICATION

(11) **EP 3 763 389 A1**
(43) Date of publication of application: **13.01.2021**
(21) Application number: 20185577.2
(22) Date of filing: 13.07.2020
(51) Int. Cl.: A61K 47/54, A61P 35/00

(54) **TUMOR HOMING STATIN DERIVATIVES**

(30) Priority: 12.07.2019 US 201962873151 P; 12.07.2019 US 201962873293 P
(71) Applicant: Da Zen Theranostics Inc., San Jose, California 95123 (US)
(72) Inventor: CHUNG, Leland W.K., Beverly Hills, California 90210 (US); CHU, Gina C.Y., San Diego, California 92130 (US); ZHANG, Yi, Los Angeles, California 90063 (US)
(74) Representative: Ellis, Michael James

(57) **Abstract**

The present invention generally relates to tumor homing statin derivatives (THSD) and their use for therapy, in particular cancer therapy. These THSD comprise three moieties: a statin moiety which comprises a dihydroxyheptanoic acid unit (DHHA) fixated by linkage into its open chain form, a heptamethine carbocyanine dye (HMCD) moiety, and a linker that conjugates the DHHA of the statin to the dye moiety. The linker is linked to the DHHA via an ester bond (ester-linked statin derivative or ELSD), or via an amide bond (amide-linked statin derivative or ALSD). Thus linked to the DHHA, the linker provides a relatively stable link either for essentially no hydrolysis/statin release after administration, or preferably for very slow hydrolysis and statin release, as is the case for the ELSD. Embodiments include methods to provide the desired THSD, in particular the ELSD, with the DHHA in its open chain form. The invention also relates to methods wherein one or more ELSD is administered to a patient in a therapeutically effective amount, and methods wherein an ELSD and an ALSD are co-administered in a coordinated administration schedule. Advantages of the THSD and their use include, among others, improved efficacy and dose-response, and decreased statin-associated side effects.

## Description

### FIELD OF THE INVENTION

The present invention generally relates to tumor homing statin derivatives (THSD) and their use for therapy, in particular cancer therapy. These THSD comprise three moieties: a statin moiety which comprises a dihydroxyheptanoic acid unit (DHHA) fixated by linkage into its open chain form, a heptamethine carbocyanine dye (HMCD) moiety, and a linker that conjugates the DHHA of the statin to the dye moiety. The linker is linked to the DHHA via an ester bond (ester-linked statin derivative or ELSD), or via an amide bond (amide-linked statin derivative or ALSD). Thus linked to the DHHA, the linker provides a relatively stable link either for essentially no hydrolysis/statin release after administration, or preferably for very slow hydrolysis and statin release, as is the case for the ELSD. Embodiments include methods to provide the desired THSD, in particular the ELSD, with the DHHA in its open chain form. The invention also relates to methods wherein one or more ELSD is administered to a patient in a therapeutically effective amount, and methods wherein an ELSD and an ALSD are co-administered in a coordinated administration schedule. Advantages of the THSD and their use include, among others, improved efficacy and dose-response, and decreased statin-associated side effects.

### BACKGROUND OF THE INVENTION

Many cancer drugs are known and include various standard treatments such as tyrosine kinase inhibitors (TKI) and classic chemotherapeutics. Many TKI, e.g. Gefitinib or Icotinib, and many chemotherapeutics, e.g. cisplatin and its derivatives, gemcitabine, doxorubicin, paclitaxel, and docetaxel, are associated with significant side effects and/or subject to the development of drug resistance. For example, cisplatin or its combination therapies with other drugs are used in particular for treatment of advanced or metastatic cancers, or cancers that typically develop metastases. Treatment options for these cancers, in particular of their advanced/metastatic forms, are very limited, and these cancer types often develop resistance in response to treatment with a given chemotherapeutic. Various treatment approaches focus on combinations of traditional cancer drugs such as chemotherapeutics, or of a chemotherapeutic with another drug having some anti-cancer effects.

Statins are HMG-CoA analogues typically used for their cholesterol-lowering effect and while generally save can display a number of side effects. Statins have also been evaluated as potential anticancer therapeutics, with mixed findings. Positive responses have been observed in some subsets of patients, particularly in tumor type-specific clinical trials combining statins with traditional chemotherapeutic agents. In other studies, detrimental effects of statins for cancer risk or therapy have been reported, e.g. increasing the risk of cancer occurrence, or increasing the risk of cancer recurrence. Particular problems with statin drugs include that they tend to have short plasma circulation times and short tumor resident times, and are rapidly inactivated in vivo.

Heptamethine carbocyanine dyes (HMCD) are known for imaging e.g. of the human body for various diagnostic purposes; some of these dyes have been described for use in both cancer imaging as well as cancer therapy.

WO 2018/075996, WO 2018/075994 and WO 2018/075993 disclose certain drug-conjugated HMCD dyes ("DZ1") conjugated to Simvastatin or to certain resistance-prone drugs for drug-delivery to cancer cells, the ability of these drug-releasing conjugates to re-sensitize drug-resistant cancer cells, and their co-administration with various resistance-prone therapeutics, including, among others, tyrosine kinases (e.g. Gefitinib or Icotinib), Cisplatin, Gemcitabine, Paclitaxel, Docetaxel, and the anti-androgens Enzalutamide and Abiraterone. The dye-drug conjugates include, among others, a dye conjugated via alkylester to Simvastatin in a form that allows lactone formation in the linked statin moiety. This ester conjugate is referred to herein-below as Rapid Release Ester or "RRE".

According to WO2018075996, certain dye-simvastatin (NIR-SM) conjugates may reduce the expression of aggressiveness-related genes in prostate cancer cells, and lead to a reversal of the phenotype associated with treated/drug-resistant tumors (thus achieving re-sensitization of cells to a drug previously administered, and allowing it to kill the resensitized cells when again administered); e.g. Simvastatin released from an alkylester-linked NIR-SM conjugate (referred to herein-below as "RRE") may re-sensitize cancer cells thus allowing successful re-administration of Enzalutamide, Abiraterone or Docetaxel.

WO 2018/075994 discloses certain DZ1-drug conjugates linked to Cisplatin, Simvastatin, or Artemisinin, and their use for sensitization to various resistance-prone therapeutics, including, among others, Cisplatin, Gemcitabine, Paclitaxel, and Docetaxel.

WO 2018/075993 discloses DZ1-drug conjugates and their use in combination with administration of Tyrosine Kinase Inhibitors (TKI), such as Gefitinib or Icotinib, to sensitize cancer cells to TKI treatment and overcome TKI resistance.

Cancers treated with TKI include those particularly aggressive cancers that are prone to metastasize and/or develop drug resistance. However, a problem with TKI is the quick development of resistance to the TKI upon treatment. Another problem with some TKI is that despite the comparatively small size (MW of 300-600) the TKI may not, or not effectively, treat the brain. Other TKI that may treat the brain may have side effects or may be effective only in particular groups of patient.

There remains a need for improved cancer therapeutics and cancer treatments, including improved effectiveness. In particular there remains a need for cancer therapeutics that provide a more rapid growth inhibition/cell death of cancer cells, and avoid the development of drug resistance. Furthermore, there remains a need for effective therapeutics and treatments with less side effects. Still further there remains a need for therapeutics and treatments effective at a lower dose. Also there remains a need for effective therapeutics and treatments with a reduced future risk, such as risk for cancer, metastasis and chemotherapeutical induced disease. Still further there remains a need for effective therapeutics and treatments that provide a less frequent administration schedule. Yet further there remains a need for effective therapeutics and treatments with less side effects and a less frequent administration. Also there is a need for therapeutics with one or more of an improved, e.g. shorter plasma or elimination half-life, longer plasma circulation time, longer tumor residence time (e.g. 1-4 weeks or longer), slower inactivation time, and a more favorable dose response curve. Further there is a need for therapeutics and treatments suitable for highly aggressive cancers, including those reduce or avoid administration of drugs that cause side effects. Also there is a need for improved therapeutics and treatments that do not require co-administration of drugs with undesirable side effects. In particular there is a need for improved therapeutics and treatments that do not require co-administration of chemotherapeutics with undesirable side effects, including general cytotoxicity (including various non-cancer cells). Further there is a need for therapies and treatments that are suitable for a wide range of cancers, in particular including drug-resistant cancers, metastatic cancers, fast- growing cancers, and otherwise aggressive cancers. Still further there is a need for improved treatments and therapeutics for patients with prostate cancer or lung cancer. Also there is a need for improved therapeutics and treatments that cross the blood-brain-barrier (BBB) and allow to treat brain tumors and metastases in the brain. With regard cancers such as lung cancer traditionally treated with TKI, improved treatments and therapeutics are needed to avoid development of resistance, and for patients previously treated with TKI, a treatment is needed that overcomes or is not affected by the developed TKI resistance, in particular for patients of non-small cell lung cancer (NSCLC), including especially adenocarcinomas (AC) of the lung. With regard to cancers including lung cancers such as small cell lung cancer (SCLC) traditionally treated with chemotherapeutics, improved treatments and therapeutics are needed to avoid development of resistance, and for patients previously treated with a chemotherapeutic, therapeutics and treatments are needed that overcome or are not affected by the acquired resistance. These and other features and advantages of the present invention will be explained and will become apparent to one skilled in the art through the summary of the invention that follows.

### SUMMARY OF THE INVENTION

Aspects of the present invention are set out in the appended independent claims. Variations of these aspects are set out in the dependent claims.

Further aspects of the invention are described herein along with features and advantages of the invention which will become apparent from the following description, which is made with reference to the accompanying drawings.

### BRIEF DESCRIPTION OF THE DRAWINGS

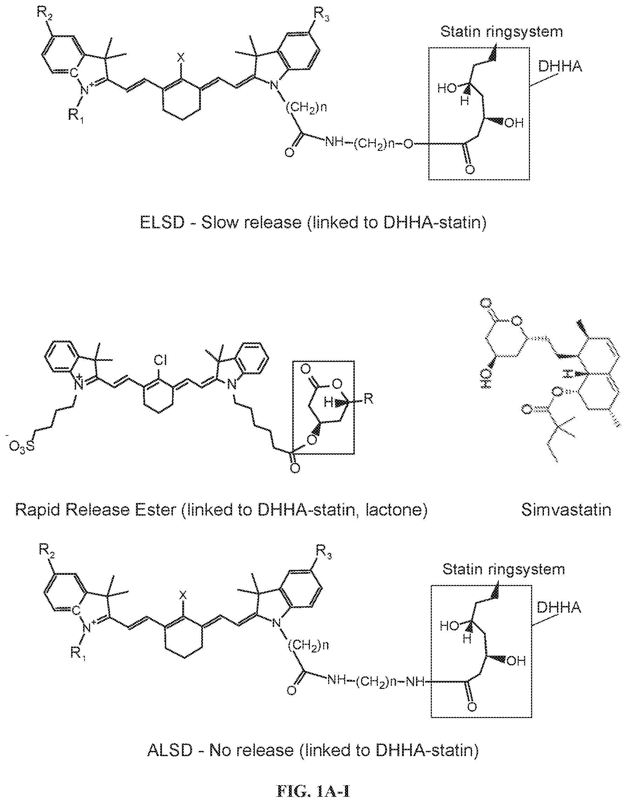

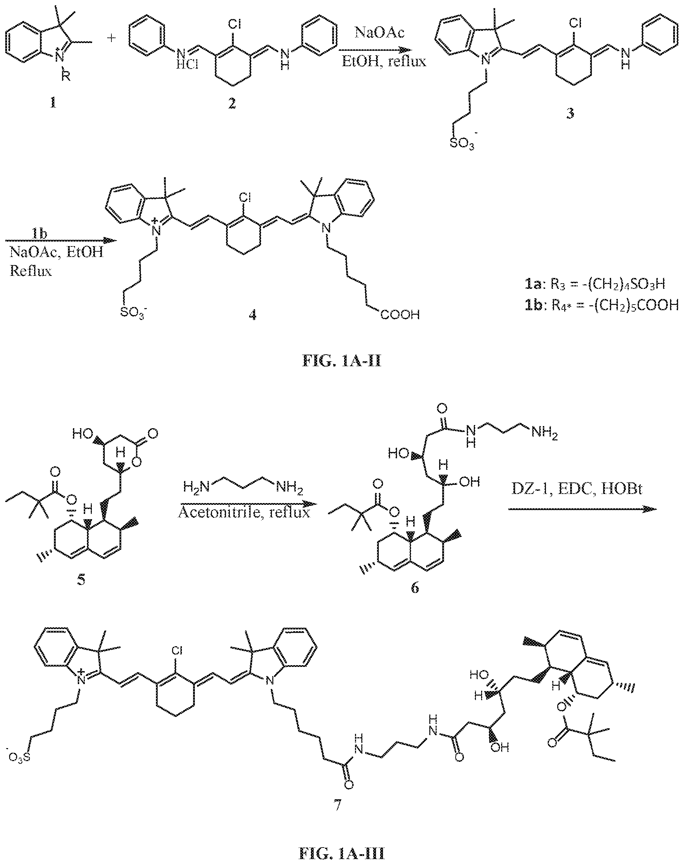

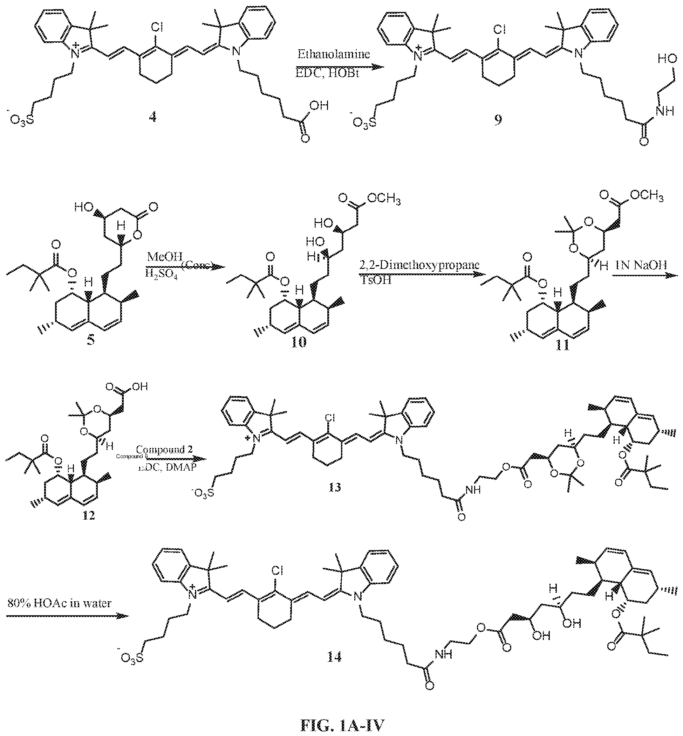

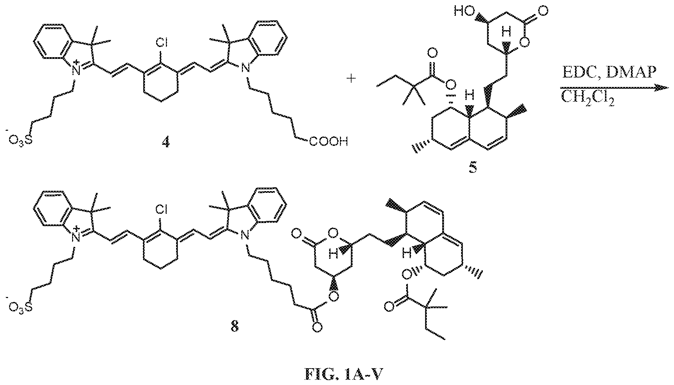

FIG. 1A-I shows ELSD, RRE and ALSD linked to the DHHA unit of a statin.
FIG. 1A-II shows the synthesis of a HMCD dye.
FIG. 1A-III shows the synthesis of a "no release" ALSD (DZ2a).
FIG. 1A-IV shows the synthesis of a "slow release" ELSD (DZ2b).
FIG. 1A-V shows the synthesis of a "Rapid Release Ester" (RRE) for comparison (DZ2c).

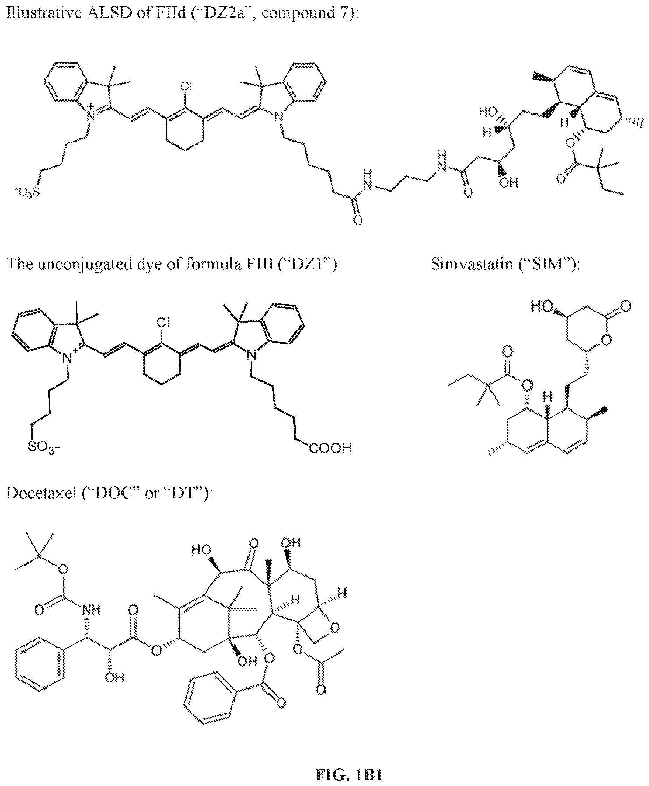

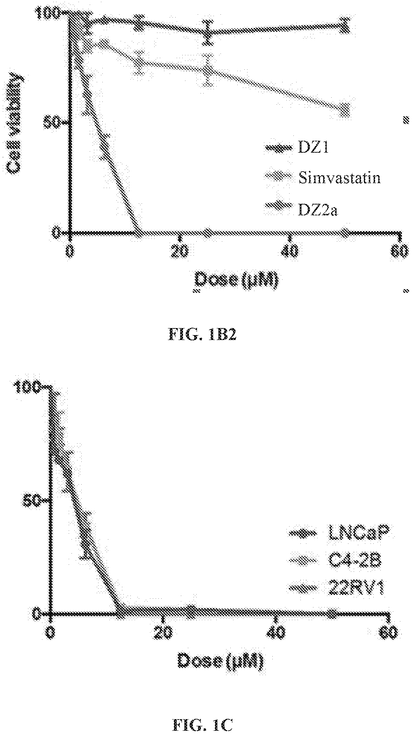

FIG. 1B1 shows the structures of DZ2a, DZ1, Simvastatin and docetaxel.
FIG. 1B2 shows growth inhibition of cancer cells by THSD.
FIG. 1C shows growth inhibition of different prostate cancer cells by THSD.
FIG. 1D shows uptake and retention of THSD in human tumors grown in mice.
FIG. 1E shows in vivo tumor shrinkage provided by THSD.
FIG. 2 shows co-localization of THSD with mitochondriae/lysosomes in cancer cells.
FIG. 3A shows lowering of the oxygen consumption rate OCR of cancer cells by THSD.
FIG. 3B shows less decrease of the Extracellular Acidification Rate (ECAR) for THSD.
FIG. 3C shows THSD bind proteins present in cytosol or mitochondriae.
FIG. 3D shows prevention of removal of polyubiquinated proteins by THSD.
FIG. 4A shows growth inhibition of cancer cells by THSD that is rapid and complete.
FIG. 4B shows growth inhibition of Abiraterone acetate-resistant cancer cells by THSD.
FIG. 4C shows growth inhibition of Enzalutamide (EZ)-resistant cancer cells by THSD.
FIG. 5 shows RhoA/B staining of human tumors in a lung cancer cell mouse model.
FIG. 6B1 shows the rapid decrease of THSD in rat plasma after systemic administration.

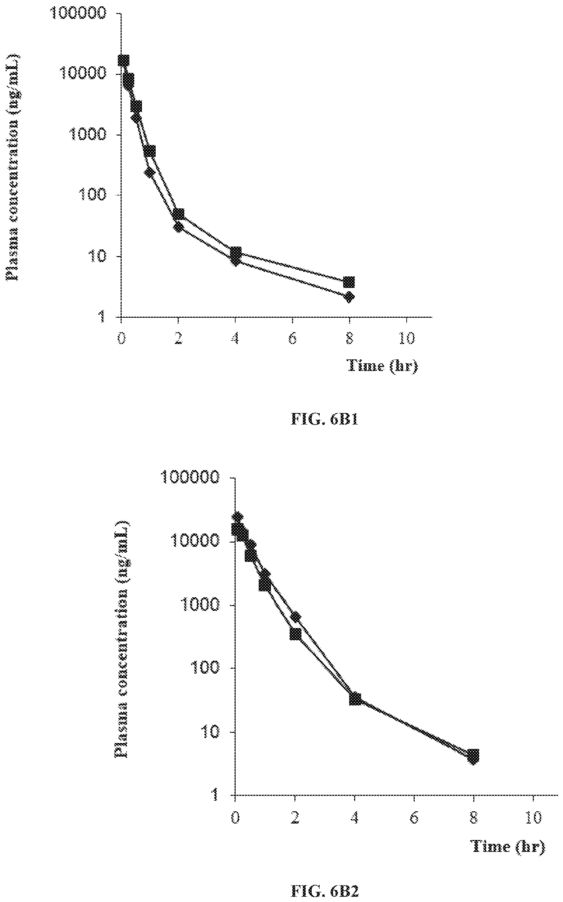

FIG. 6B2 shows the rapid decrease of THSD in dog plasma after systemic administration.

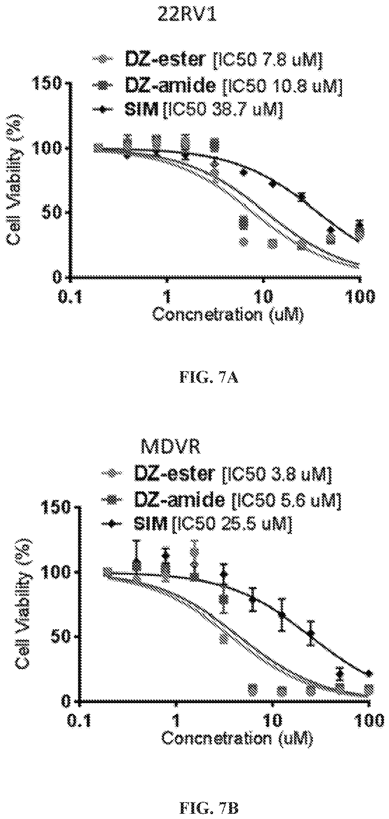

FIG. 7A shows that ALSD and ELSD inhibit growth of 22RV1 cancer cells.
FIG. 7B shows that ALSD and ELSD inhibit growth of EZ-resistant cancer cells.

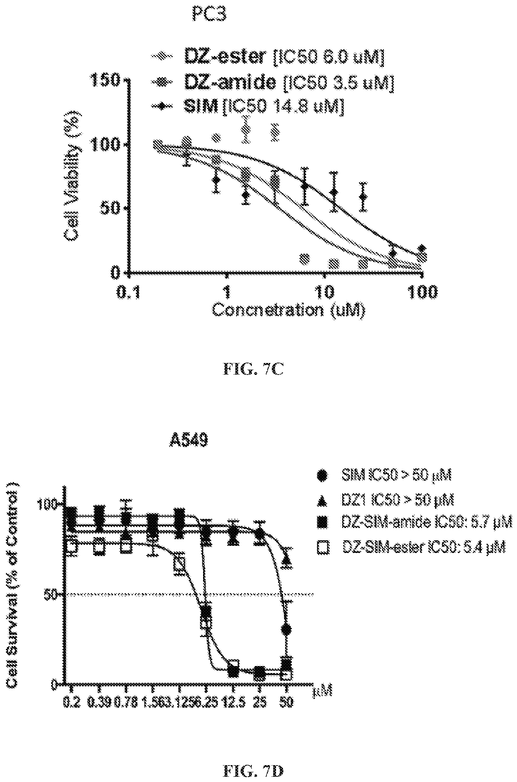

FIG. 7C shows that ALSD and ELSD inhibit growth of PC3 cancer cells.
FIG. 7D shows that ALSD and ELSD inhibit growth of A549 cancer cells.

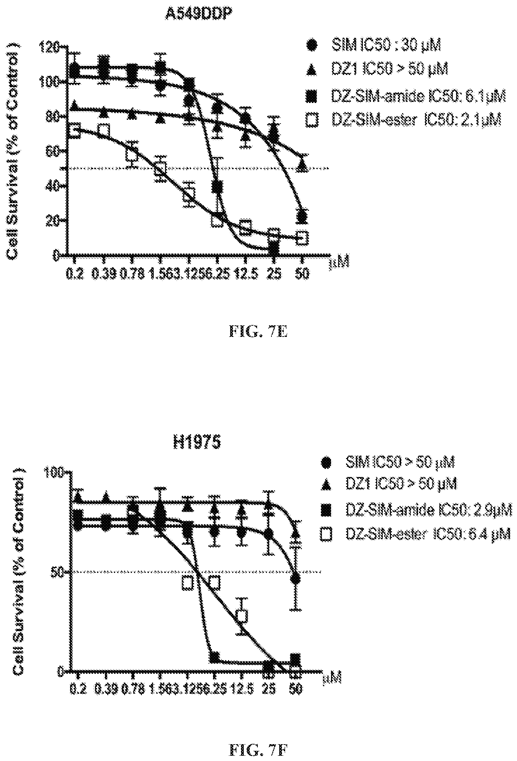

FIG. 7E shows that ALSD and ELSD inhibit growth of A549DDP cancer cells.
FIG. 7F shows that ALSD and ELSD inhibit growth of H1975 cancer cells.

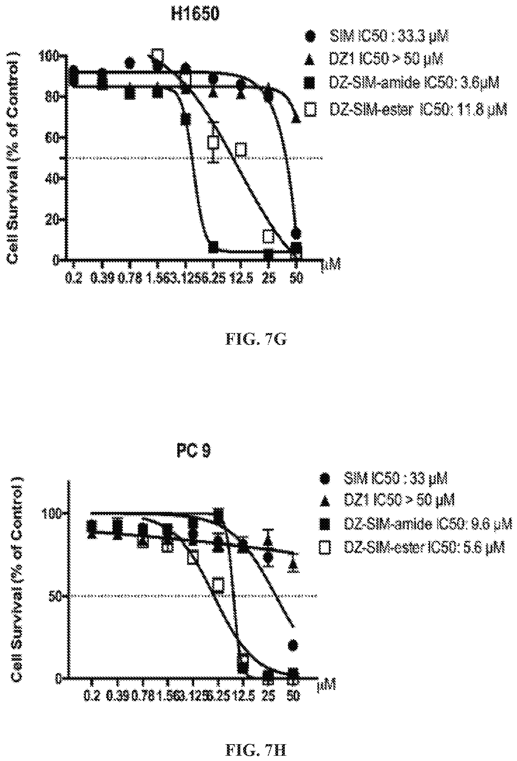

FIG. 7G shows that ALSD and ELSD inhibit growth of H1650 cancer cells.
FIG. 7H shows that ALSD and ELSD inhibit growth of PC9 cancer cells.

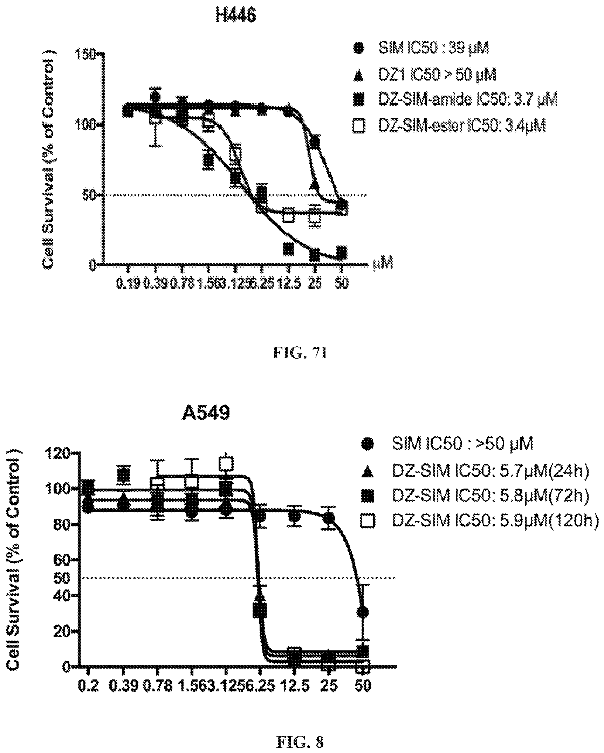

FIG. 7I shows that ALSD and ELSD inhibit growth of H446 cancer cells.
FIG. 8 shows that an ALSD is a stable/non-hydrolyzing drug rather than a pro-drug.

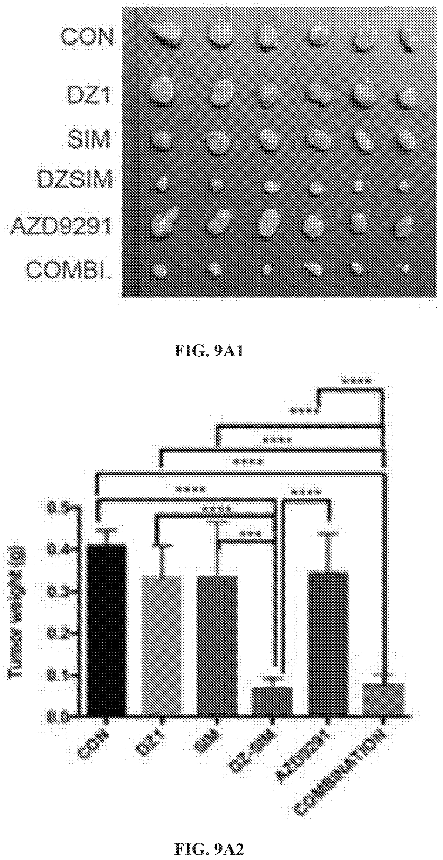

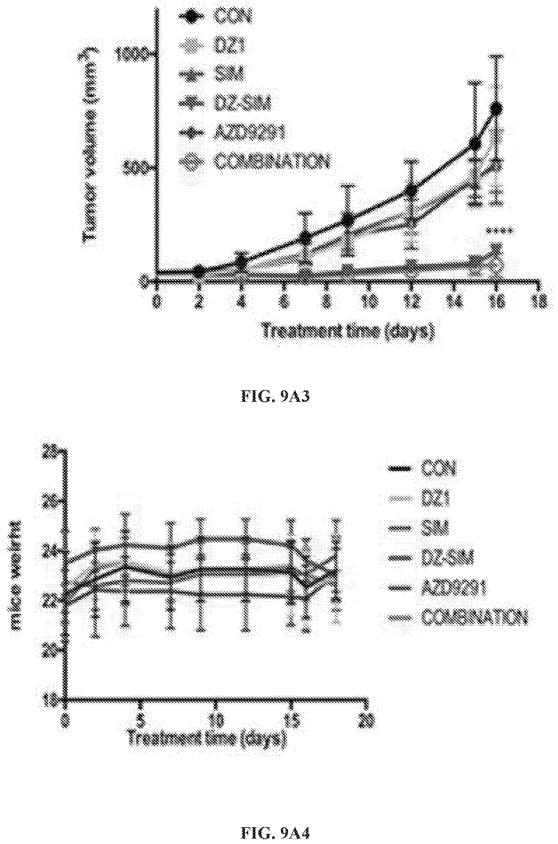

FIG. 9A1 shows growth inhibition by THSD in an in vivo model bearing 3rd generation EGFR-inhibitor-resistant cancer cells.
FIG. 9A2 shows a chart comparing tumor weights showing in vivo inhibition by THSD.
FIG. 9A3 shows graphs comparing tumor volumes showing in vivo inhibition by THSD.
FIG. 9A4 shows graphs comparing weight of mice, and lack of weight loss/acute toxicity.

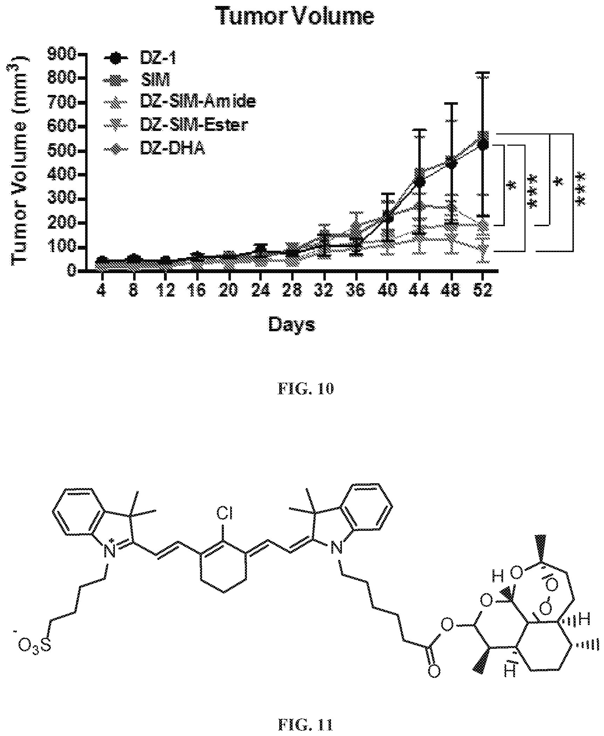

FIG. 10 shows growth inhibition by THSD in an in vivo model based on H446 human cancer cells.
FIG. 11 shows a non-statin conjugate DZ 1-DHA for comparative purposes.

### DETAILED DESCRIPTION OF THE INVENTION

The present invention generally relates to tumor homing statin derivatives (THSD) and their use for therapy, in particular cancer therapy. The THSD comprises a heptamethine carbocyanine (HMCD) dye moiety linked to the DHHA of a statin by a linker that fixates the DHHA into its open chain form; the linker may be attached to the open-chain DHHA either via an ester bond ("ELSD") or via an amide bond ("ALSD"). Embodiments also include methods to make the THSD and methods of their administration. In particular, methods include administration of ELSD, or a coordinated administration schedule wherein an ALSD is co-administered together with an ELSD, optionally with one or more further drug, e.g. a chemotherapeutic drug or a hormonal antagonist/anti-androgenic drug.

Surprisingly it has now been found that, unlike pro-drugs, the tumor homing statin derivates (THSD) of the present invention are essentially stably linked and achieve various advantages including e.g. improved growth inhibition, dose-response and improved and/or additional therapeutic effects, while being less rapidly inactivated and avoiding or reducing side effects. The ALSD essentially does not release the statin; similarly the ELSD provides a very slow release, in contrast to a RRE (see e.g. **example 8** and **Fig. 8**). Compared to the ALSD, the ELSD provides an improved dose response with a curve that is less steep (compare e.g. **FIG. 7D-E****,** and **FIG. 7H**).

Despite the lack of release (or in case of the ELSD the slow release), as shown in comparative examples (see e.g. **examples 1B, 1E and 7,** also see figures **FIG. 1A-E** and **FIG. 7A-I** **and** **8**), the THSD outperform not only the unconjugated statin and the unconjugated dye, but also chemotherapeutics including e.g. Docetaxel, and other drugs used for cancer therapy, such as hormonal antagonists including anti-androgens (e.g. Abiraterone and Enzalutamide), and do so without requiring co-administration of these therapeutics (thus avoiding their significant side-effects).

Thus the THSD in its conjugated form provides the active principle, and surprisingly, appears to be able to bind despite its much larger size into the relevant binding pockets including those of the statin, to achieve its various effects including anti-cancer effects and effects provided by statins. In contrast, the rapid release ester ("RRE") quickly hydrolyzes to release the statin (see e.g. **comparative examples 1A-IV** and **4A** and corresponding figures **FIG. 1A-IV** herein-below). Unlike the RRE, the THSD are thus much more stable and do not release the statin systemically to a significant degree for a prolonged duration (e.g. hours, days, weeks or even months), yet provide their effects to cancer cells in their conjugated form.

Also, surprisingly, the ELSD appears to have an improved dose-response curve compared to the ALSD that is less steep, i.e. providing an initial effect at a lower dose and/or more quickly (compare e.g. **FIG. 7D****,** **FIG. 7E****,** and **FIG. 7H**.). Without wishing to be bound by theory, it is believed that in ELSD an early systemic release is avoided yet the anti-cancer effects and some statin effects are provided immediately within the first few hours, as these effects are mediated by the conjugate and/or the released statin portion after cancer cell entry of the ELSD. Furthermore, these statin derivatives may also provide one or more statin effects to cancer cells while decreasing or avoiding side effects typically associated with statin drugs administered systemically or via rapid-release conjugates such as the RRE

Further, surprisingly, THSD are able to effectively treat the most aggressive cancers that tend to develop resistance and other resistance-prone forms of cancer, and are able to do so without requiring co-administration of cancer drugs such as TKI or chemotherapeutics; thus THSD may be effective in cancers such as, without limitation, prostate cancer, lung cancer including non-small cell lung cancer (NSCLC), adenocarcinomas (AC) of the lung, and small cell lung cancer (SCLC). This is shown for both ELSD and ALSD e.g. in **FIG. 7 A-I**, **FIG. 8** and **FIG. 10** which illustrate growth inhibition of cells lines representing prostate cancer, prostate carcinoma, prostate adenocarcinoma, lung cancer, lung carcinoma, NSCLC, lung adenocarcinoma, SCLC, and including various drug resistant forms, and in mouse models for the human tumors formed in mice implanted with these human cancer cells. Treatment of these cancer forms with THSD allows to avoids the development of resistance that is often encountered with standard treatments such as TKI or chemotherapeutics, or allows to overcomes resistance once developed, e.g. resistance due to prior cancer drug treatment, for example with tyrosine kinase inhibitors (TKI) such as Gefitinib or standard chemotherapeutics such as Docetaxel and Cisplatin (DDP).

Furthermore, surprisingly, the THSD cross the blood-brain-barrier and are thus able to treat brain tumors and brain metastases even when administered systemically, rather than locally.

Without wishing to be bound by theory, at least some of the effects and advantages of THSD seem mediated by the particular type of conjugate and in particular the type of linker and its linkage to the conjugate moieties and their particular form, i.e. DZ1-backbone, the L_{E}/L_{A} linker and its amide or ester linkage that connects it to the statin via the statin's DHHA unit (which is the statin's key pharmacophore), and, importantly, the form of the DHHA unit when connected to the linker. Three types of dye-statin conjugates that can be distinguished based on their linker and/or linkage-structure (and thus their effects) essentially include 1) ALSD, 2) ELSD, and 3) RRE. According to linker/linkage, these may either 1) prevent release of the statin (i.e. in the ALSD having a L_{A} linker with amide linkage), or 2) provide for a slow release of the statin (i.e. in the ELSD with ester linkage to the open chain form of the DHHA), and 3) provide for a rapid release of the statin from the pro-drug conjugate (i.e. in the RRE with ester linkage to the lactone form of the DHHA). In contrast to the pro-drug rapid release type, the THSD are either of type 1) or 2), and do not release the statin, or provide a very slow release.

Without wishing to be bound by theory, it appears that the THSD, especially the "no release" amide linked statin derivates ("ALSD"), but also the "slow release" ester-linked statin derivates ("ELSD"), tend to tightly bind to cellular proteins and nucleic acids, which causes the THSD to avoid inactivation and remain inside in the cancer cells for prolonged periods, e.g. days, weeks or even months, thus allowing a much less frequent administration schedule, while reducing or even avoiding side effects, e.g. compared to those of conjugated or unconjugated chemotherapeutics, unconjugated statins, and conjugated statins of the "fast release" pro-drug type. In particular in case of the ELSD, the tight binding may be a contributing factor to avoid the rapid post-release inactivation of the statin seen e.g. in the "fast release" type ester-bound HMCD statin conjugates, and may this contribute to improved therapeutic effects (e.g. growth suppression/death of cancer cells and tumor shrinkage), while avoiding one or more side effects.

Without wishing to be bound by theory, it appears that ELSD, when their effect such as growth inhibition is compared to the respective ALSD, have a dose response curve that is less steep and start to be effective at a lower concentration and provide an initial non-maximal effect. For example, the dose of the ELSD or the total THSD may be, e.g., about 0.1 mg to a maximum about 6 mg/kg or less (e.g. up to about 2.5 mg or even about 1.0 mg/kg). Thus advantageously, one or more ELSD may be used for treatment of cancer either on its own (e.g. treating with a lower dose which due to its long tumor residence time may continue to have a non-maximal but sufficient therapeutic effect), or in combination with one or more ALSD (e.g. to thus provide a sufficient therapeutic effect and/or lower the ALSD or total THSD dose at which a sufficient or maximum effect is achieved). Illustrative administration schedules are described herein-below.

In embodiments, provided is an ester-linked statin derivative shown in formula FIa below: 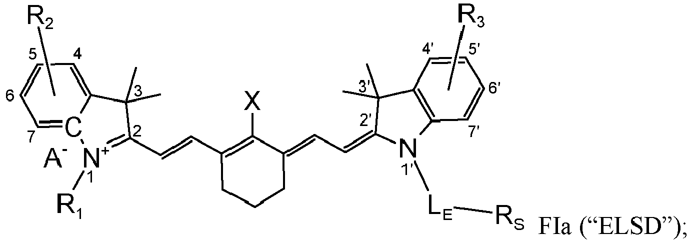 wherein X is a halogen residue; wherein R₁ is a residue selected from the group consisting of C₁-C₂₅ alkyl, C₅-C₂₅ aryl, C₅-C₂₅ indolyl, C₅-C₂₅ thienyl, C₅-C₂₅ phenyl, C₅-C₂₅ napththyl, C₁-C₂₅ aralkyl, C₁-C₂₅ alkylsulphonate, C₁-C₂₅ alkylcarboxyl, C₁-C₂₅ alkylamino, C₁-C₂₅ ω-alkylaminium, C₁-C₂₅ ω-alkynyl, a PEGyl polyethylene chain with (-CH₂-CH₂-O-)₂₋₂₀, a PEGylcarboxylate with (-CH₂-CH₂-O-)₂₋₂₀, a ω-PEGylaminium with (-CH₂-CH₂-O-)₂₋₂₀, a ω-acyl-NH, a ω- acyl-lysinyl-, a ω -acyl-triazole, a ω- PEGylcarboxyl-NH- with (-CH₂-CH₂-O-)₂₋₂₀, a ω-PEGylcarboxyl-lysinyl with (-CH₂-CH₂-O-)₂₋₂₀, and a ω- PEGylcarboxyl-triazole with (-CH₂-CH₂-O-)₂₋₂₀; wherein R₂ and R₃ are residues independently selected from the group consisting of: a hydrogen, an C₁-C₂₀ alkyl, a sulphonate, a C₁-C₂₀ alkylcarboxyl, C₁-C₂₀ alkylamino, C₁-C₂₀ aryl, -SO₃H, -PO₃H, -OH, -NH₂, and a halogen residue; and wherein R₂ and R₃ are attached at a ring position selected from the group consisting of 3, 3', 4, 4', 5, 5', 6, and 6'; wherein the A⁻ group is a pharmaceutically acceptable negatively charged anion; wherein an ester linker L_{E} is linked to a statin residue R_{S} by an ester bond; wherein L_{E} is selected from the group consisting of L_{E1} and L_{E2}, wherein L_{E1} is -(CH₂)ₙ-O-, and wherein L_{E2} is -(CH₂)ₙ-CO-NH-(CH₂)ₘ-O-, and wherein n=4-9, and m=1-4; wherein Rs is a residue of a statin or of a statin derivative, wherein R_{S} comprises a dihydroxyheptanoic acid unit (DHHA) that is connected to residue R_{S*} which is the remainder of the statin; and wherein Rs is linked to L_{E} via its DHHA in its open chain form which is -CO-CH₂-COH-CH₂-CHOH-R_{S*}.

In embodiments, provided is an ELSD wherein the statin is selected from the group consisting of: Simvastatin, Mevastatin, Lovastatin, Pravastatin, Atorvastatin, Fluvastatin, Rosuvastatin, Cerivastatin, Pitavastatin, and derivatives thereof which retain binding to the statin's active binding site.

In embodiments, provided is an ELSD wherein the statin is Simvastatin and wherein the ester linker L_{E} is L_{E2}, as shown in Formula FIb below: 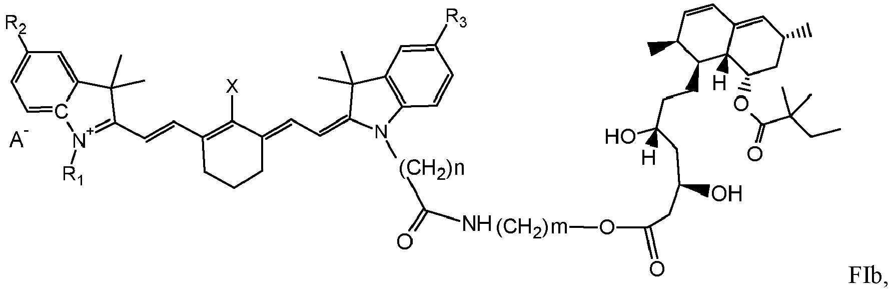 and wherein A⁻, X, R₁, R₂, R₃, and n and m of the L_{E2} linker are defined as for FIa herein.

In embodiments, provided is an ELSD of FIa or FIb wherein X is Cl. Further embodiments are provided wherein R₁ is a -(CH₂)n-SO₃- alkylsulphonate residue, and wherein n of R₁ is selected from 2, 3, 4, 5, 6, 7 and 8. Still further embodiments are provided wherein R₁ is a -(CH₂)₄-SO₃- alkylsulphonate residue. Also, embodiments are provided wherein R₂ and R₃ are H.

In embodiments, provided is a pharmaceutical composition comprising an ELSD and one or more pharmaceutical excipient, wherein the ELSD is selected from the group consisting of: an ELSD of formula FIa above; the ELSD of FIa wherein the statin to form R_{S} is selected from the group consisting of: Simvastatin, Mevastatin, Lovastatin, Pravastatin, Atorvastatin, Fluvastatin, Rosuvastatin, Cerivastatin, Pitavastatin, and derivatives thereof which retain binding to the statin's active binding site; an ELSD of formula FIb above, wherein the statin is Simvastatin and the ester linker L_{E} is L_{E2} (-(CH₂)ₙ-CO-NH-(CH₂)ₘ-O-, and wherein A⁻, X, R₁, R₂, R₃, and n and m of the L_{E2} linker, are defined as for FIa in (i) above; an ELSD as defined herein wherein X is Cl; an ELSD as defined herein wherein R₁ is a -(CH₂)ₙ-SO₃- alkylsulphonate residue and wherein n of R₁ is selected from 2, 3, 4, 5, 6, 7 and 8; an ELSD as defined herein wherein R₁ is a -(CH₂)₄-SO₃⁻ alkylsulphonate residue.

In embodiments, provided is a pharmaceutical composition comprising one or more ELSD and further comprising an ALSD formula FIIa below: 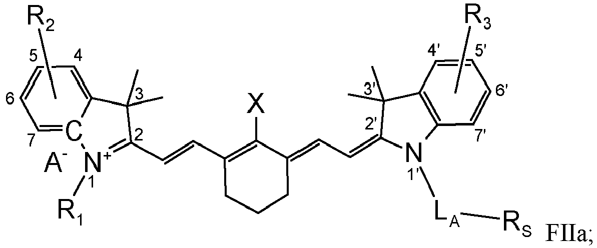 wherein A⁻, X, R₁, R₂, and R₃ are defined as described for FIa herein; and wherein an amide linker L_{A} is linked to a statin residue R_{S} via an amide bond; wherein L_{A} is a linker selected from the group consisting of L_{A1} and L_{A2}, wherein L_{A1} is -(CH₂)ₙ-NH-, and wherein L_{A2} is -(CH₂)ₙ-CO-NH-(CH₂)ₘ-NH- and wherein n=4-9, and m=1-4; wherein Rs is a residue of a statin or of a statin derivative, wherein R_{S} comprises a dihydroxyheptanoic acid unit (DHHA) that is connected to residue R_{S*} which is the remainder of the statin, and wherein Rs is linked to L_{A} via its DHHA in its open chain form which is -CO-CH₂-COH-CH₂-CHOH-R_{S*}.

In embodiments, provided is a method of treating cancer wherein one or more ELSD, and optionally one or more ALSD, are administered to a patient in need thereof in amounts sufficient to inhibit cancer cell or pre-cancerous cell growth or induce apoptosis in cancer or pre-cancerous cells in the patient, wherein the one or more ELSD is selected from the group consisting of: an ELSD of formula FIa above; the ELSD of FIa wherein the statin to form RS is selected from the group consisting of: Simvastatin, Mevastatin, Lovastatin, Pravastatin, Atorvastatin, Fluvastatin, Rosuvastatin, Cerivastatin, Pitavastatin, and derivatives thereof which retain binding to the statin's active binding site; an ELSD of formula FIb above, wherein the statin is Simvastatin and the ester linker LE is L_{E2} is-(CH₂)ₙ-CO-NH-(CH₂)ₘ-O-, and wherein A⁻, X, R₁, R₂, R₃ and n and m of the L_{E2} linker are defined as for FIa in (i) above; an ELSD as defined herein wherein X is Cl; an ELSD as defined herein wherein R₁ is a -(CH₂)ₙ-SO₃⁻ alkylsulphonate residue and wherein n of R₁ is selected from 2, 3, 4, 5, 6, 7 and 8; an ELSD as defined herein wherein R₁ is a -(CH₂)₄-SO₃⁻ alkylsulphonate residue; and wherein the optional one or more ALSD is selected from the group consisting of: an ALSD of formula FIIa as described herein; and an ALSD of formula FIIb as shown below and wherein the statin is Simvastatin and the linker is L_{A2} (-(CH₂)ₙ-CO-NH-(CH₂)ₘ-NH-): 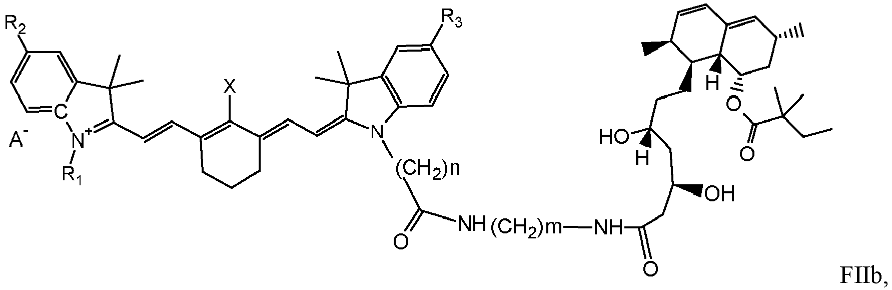 and wherein A⁻, X, R₁, R₂, R₃, and n and m of the L_{A2} linker, are as defined herein-above for FIa.

In embodiments, provided is a method wherein the one or more ELSD and the one or more ALSD are co-administered to the patient in a coordinated administration schedule of one or more combined dosage forms, wherein each dosage form comprises the one or more ELSD and the one or more ALSD and one or more pharmaceutical excipient.

In embodiments, provided is a method wherein the one or more ELSD is co-administered in a coordinated administration schedule with the one or more ALSD; wherein the schedule includes administration of a loading dose administered at least one hour or more prior to administration of one or more maintenance dose; wherein the loading dose consists of a separate dosage form that comprises the one or more ELSD and one or more pharmaceutical excipient, and does not comprise the one or more ALSD; and wherein the one or more maintenance dose consists of a dosage form that comprises the one or more ALSD and one or more pharmaceutical excipient, and optionally comprises the ELSD.

In embodiments, provided is a method wherein the one or more ELSD is co-administered in a coordinated administration schedule together with one or more secondary drug, and wherein the one or more secondary drug is selected from the group consisting of: a hormonal antagonist, an anti-androgenic drug, Abiraterone, Enzalutamid, a chemotherapeutic drug, Docetaxel, Paclitaxel, and Cabazitaxel.

In embodiments, provided is a method wherein one or more combined dosage form, or one or more separate dosage forms of a loading dose and one or more maintenance doses, are co-administered in a coordinated administration schedule; wherein the combined dosage form comprises the one or more ELSD, the one or more ALSD, and the one or more pharmaceutical excipient, wherein the loading dose consists of a separate dosage form that comprises the one or more ELSD and one or more pharmaceutical excipient, and does not comprise the one or more ALSD; and wherein the one or more maintenance dose consists of a dosage form that comprises the one or more ALSD and one or more pharmaceutical excipient, and optionally comprises the ELSD; wherein the co-administered dosage forms are further co-administered in a coordinated administration schedule together with one or more secondary drug, and wherein the one or more secondary drug is selected from the group consisting of: a hormonal antagonist, an anti-androgenic drug, Abiraterone, Enzalutamid, a chemotherapeutic drug, Docetaxel, Paclitaxel, and Cabazitaxel.

In embodiments, provided is a method wherein the one or more ELSD is administered to a patient whose cancer cells, pre-cancerous lesions, tissues, tumors or metastases are identified to carry one or more genetic aberration in one or more gene encoding for one or more tyrosine kinase receptor, selected from the group comprising: epidermal growth factor receptor tyrosine kinase (EGFR), Anaplastic lymphoma kinase receptor (ALF), and Proto-oncogene tyrosine-protein kinase (ROS or ROS1).

In embodiments, provided is a method wherein the one or more ELSD is administered to a patient whose cancer cells, pre-cancerous lesions, tumors or metastases have acquired resistance to one or more tyrosine kinase inhibitor (TKI), including a patient who received prior TKI treatment with one or more TKI prior to ELSD administration and whose response to the prior TKI treatment is therapeutically insufficient.

In embodiments, provided is a method wherein the TKI is selected from the group consisting of an epidermal growth factor receptor tyrosine kinase inhibitor (EGFR-TKI), an ALK tyrosine kinase receptor inhibitor (ALK-TKI), and an inhibitor to Proto-oncogene tyrosine-protein kinase ROS (ROS-TKI).

In embodiments, provided is a method wherein the EGFR-TKI is selected from the group consisting of: Gefitinib, Icotinib, Erlotinib, Brigatinib, Dacomitinib, Lapatinib, Vandetanib, Afatinib, Osimertinib (AZD9291), CO-1686, HM61713, Nazartinib (EGF816), Olmutinib, PF-06747775, YH5448, Avitinib (AC0010), Rociletinib, and Cetuximab.

In embodiments, provided is a method wherein the patient is suffering from a drug-resistant cancer as determined by drug exposure or genetic testing, the drug-resistant cancer selected from the group comprising: prostate cancer, pancreatic cancer, lung cancer, non-small cell lung carcinoma (NSCLC; NSCLC may include squamous-cell carcinoma, adenocarcinoma (mucinous cystadenocarcinoma), large-cell lung carcinoma, rhabdoid carcinoma, sarcomatoid carcinoma, carcinoid, salivary gland-like carcinoma, adenosquamous carcinoma, papillary adenocarcinoma, giant-cell carcinoma), SCLC (small cell lung carcinoma), combined small-cell carcinoma, non-carcinoma cancers of the lung (sarcoma, lymphoma, immature teratoma, and melanoma), kidney cancer, lymphoma, colorectal cancer, skin cancer, HCC cancer, and breast cancer, squamous-cell carcinoma of the lung, anal cancers, glioblastoma, epithelian tumors of the head and neck, and other cancers.

In embodiments, provided is a method wherein the patient is a patient suffering from a drug-resistant lung cancer as determined by drug exposure or genetic testing, the drug resistant lung cancer selected from the group comprising: small cell carcinoma lung cancer (SCCLC), non-small cell lung carcinoma (NSCLC), combined small-cell carcinoma, squamous-cell carcinoma, adenocarcinoma (AC, mucinous cystadenocarcinoma, MCACL), large-cell lung carcinoma, rhabdoid carcinoma, sarcomatoid carcinoma, carcinoid, salivary gland-like carcinoma, adenosquamous carcinoma, papillary adenocarcinoma, giant-cell carcinoma, non-carcinoma cancer of the lung, sarcoma, lymphoma, immature teratoma, and melanoma.

In embodiments, the THSD essentially comprise three moieties: a statin moiety, a heptamethine carbocyanine (HMCD) dye moiety, and a linker moiety that conjugates the DHHA of the statin to the dye moiety via an ester or amide bond ("L_{E}/L_{A} linker" or "linker"). The dye is a NIR (near infrared) dye of the heptamethine carbocyanine type that comprises a central halogen-cyclohexyl moiety. The linker may be thought of as comprising an alkyl chain of the dye. In the conjugated statin, the DHHA is fixated into its open chain form by attachment of the linker and dye.

Embodiments include methods to make the THSD; for example, without limitation, the dihydroxyheptanoic acid (DHHA) unit of a statin or statin precursor can be reacted with a hydroxylamine, and the hydroxyl group of the statin reaction product can be reacted with the carboxyl group of the HMCD, forming a L_{E} linker with ester-bond. Alternatively, a dye moiety precursor can be reacted with a diamine, and the dye reaction product's amine group can be reacted with the carboxyl group of the statin's DHHA, thus forming an amide bond.

For example, in case of the ELSD, the linker may be an alkyl linker (-(CH₂)ₙ-O-, also referred to herein as "L_{E1}"), or may be a carboxamide linker comprising a carboxamide (-(CH₂)n-CO-NH-(CH₂)m-O-, also referred to herein as "L_{E2}") which may result from e.g. a reaction of an hydroxyl group of an HMCD derivative (the amine group of the derivative resulting e.g. from previous reaction of a carboxyl group of the HMCD with a hydroxylamine, e.g. ethanolamine), with a carboxyl group of the DHHA unit of a statin or statin derivative, thus forming an in-chain carboxamide of the L_{E} linker that is linked to the DHHA unit of the statin via an ester bond (compare e.g. FIG. 1A-IV for an illustrative embodiment how to form the ELSD).

In case of the ALSD, similarly, the linker may be an alkyl linker (-(CH₂)ₙ-NH-, also referred to herein as "L_{A1}"), or may be a carboxamide linker comprising a carboxamide (-(CH₂)n-CO-NH-(CH₂)m-NH-, also referred to herein as "L_{A2}"). The carboxamide of L_{A2} may result from, without limitation, e.g. a reaction of an amine group of a statin derivative (the amine group of the derivative e.g. resulting from previous reaction of the DHHA unit of the statin with the amine residue of a diamine, e.g. propane-1,3-diamine) with a carboxyl group of the HMCD, thus forming a THSD with a carboxamide linker (L_{A2}) that is linked to the statin via an amide bond (compare e.g. **FIG. 1A-III** for an illustrative embodiment how to form the ALSD).

Thus the linker of the THSD may be connected to the statin either via an amide bond or an ester bond (L_{E} or L_{A}, respectively).

The linking amide or ester bond connect the dye part of the THSD to the statin residue via the statin's open chain DHHA; without wishing to be bound by theory, this particular linkage may contribute to the enhanced efficacy in both the ELSD and the ALSD, as well as result in a slow release ester in cases of the ester bond. Thus both ELSD and ALSD are relatively stable: one is characterized by essentially no release of the statin (Amide Linked SD or "ALSD"), and the other provides a very slow release (Ester Linked SD or "ELSD"), much slower than other rapid release conjugates, in particular rapid release esters ("RRE") that are linked to a DHHA that allows taking the lactone form.

Carboxamide linkers (L_{E2}/L_{A2}) may be preferred for superior efficacy and in case of the ester further improved stability/slower release. A general formula for the L_{E2}/L_{A2} linkers may be -(CH₂)n-CO-NH-(CH₂)m-NH-, wherein n=4-9 and m=1-4 (or "L_{E}") for the ALSD, and -(CH₂)n-CO-NH-(CH2)m-O-, wherein wherein n=4-9 and m=1-4 (or "L_{A}") for the ELSD.

More preferably, n and m may be chosen for L_{E} and L_{A} as follows: n=4-6 and m=1-4; most preferably with the total of n (n+m) not exceeding 10, 9, 8, 7, or 6, e.g. for n=5 a total of 6-9 (i.e. DZ1-based THSD), i.e. without limitation: -(CH₂)₅-CO-NH-(CH₂)₁-NH-, -(CH₂)₅-CO-NH-(CH₂)₂-NH-, -(CH₂)₅-CO-NH-(CH₂)₃-NH-, -(CH₂)₅-CO-NH-(CH₂)₄-NH- for L_{E}; and -(CH₂)₅-CO-NH-(CH₂)₁-O-, -(CH₂)₅-CO-NH-(CH₂)₂-O-,-(CH₂)₅-CO-NH-(CH₂)₃-O-, -(CH₂)₅-CO-NH-(CH₂)₄-O- for L_{A}.

Surprisingly, it has been found that improvements are provided by the THSD as such, i.e. in form of the conjugate, rather than acting as a prodrug (where the drug moiety upon release would provide its effects). In case of the ALSD there is essentially no or minimal hydrolysis of the statin, and surprisingly the amide bond was found to be substantially stable under *in vivo* conditions, with essentially no released statin detected. Similarly, in case of the ELSD, hydrolysis occurs only very slowly under "in vivo" conditions, and thus after the ELSD reaches the cancer cells or tissue.

As there is no significant release of the statin from THSD under *in vivo* conditions as shown e.g. in serum of various mammals, see e.g. **examples 1A-V** (ELSD is stable in mouse blood serum at 37° C, in contrast to RRE), and **example 6** (rat/dog; ALSD is stable *in vivo* for at least 24h) herein-below, the improved effects shown for the THSD are substantially those of the conjugate itself, avoiding side effects, including those associated with systemic statin administration.

Without wishing to be bound by theory, it is believed that the structure of the THSD as described herein, and in particular, the open chain form of the DHHA residue of the statin, contributes to the various therapeutically relevant effects including effects neither the dye nor the much smaller statin itself would achieve. This may be further improved by the presence of a carboxamide in the linker.

Surprisingly, the therapeutic effects of the THSD are not only improved over those of the unconjugated statin and the unconjugated dye, but also over those of various chemotherapeutic drugs, some of which are highly cytotoxic and provided at high dosage, both in resistant and in non-resistant cancer cells or tumors. Such effects of the THSD are shown e.g. in comparative examples **1A-IV** herein-below, and corresponding figures **FIG. 1A-IV**.

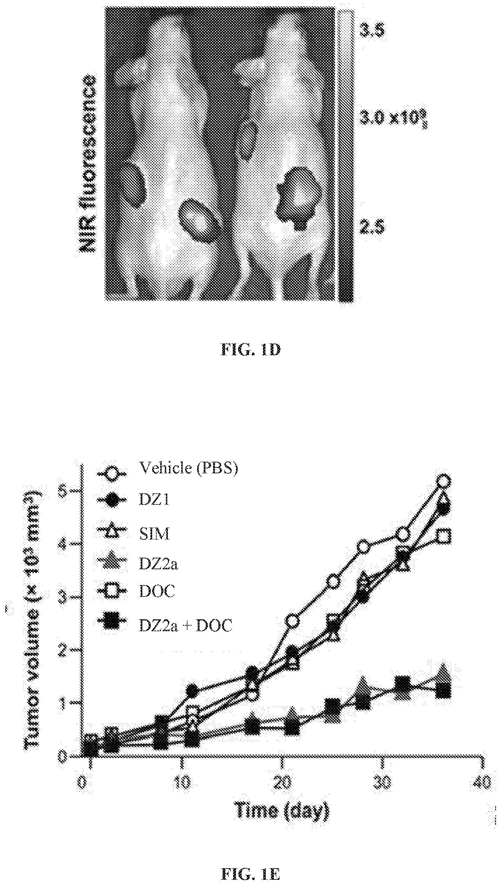

Improvements of the THSD are illustrated in **examples 1A-1E** and **FIG. 1A-1E** that show that THSD can provide improved cancer cell growth inhibition and *in vivo* tumor shrinkage compared to both the unconjugated statin drug itself, and compared to the unconjugated dye (see e.g. **FIG. 1A-C**). These improvements can be found in different cancer cell lines and human tumors grown in mice (see e.g. **FIG. 1D**), and outperform cancer drugs such as Docetaxel (see e.g. **FIG. 1E**), which even when administered in high amounts of 8 mg/kg body weight essentially had no or very mild tumor suppressive activity when compared to the THSD, which exhibited a strong long lasting tumor suppressive effect at a lower amount of 5 mg/kg. Thus Docetaxel can be replaced with a THSD, avoiding its side effects and providing better activity/growth inhibition and tumor shrinkage.

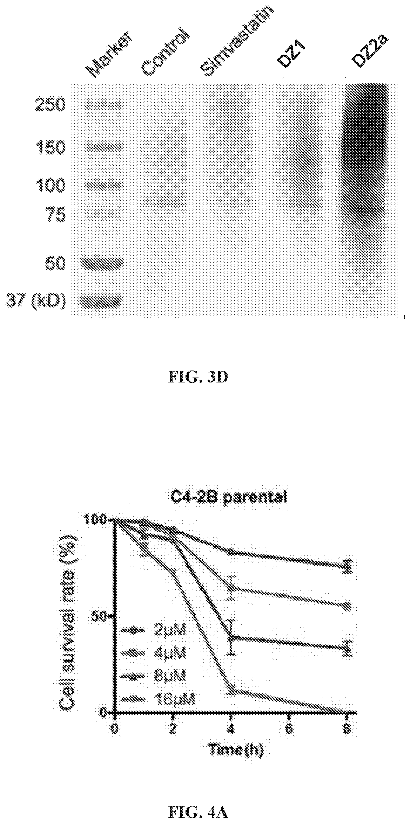

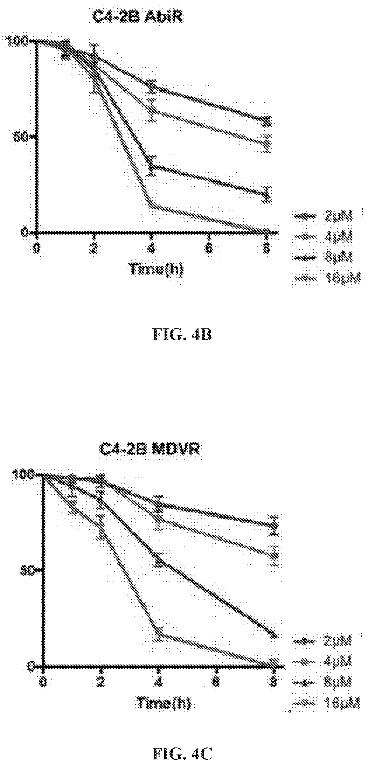

Also, as illustrated in **examples 4A1-3** and **B1-3** and **FIG. 4A-C** which show that THSD provide improvements in treating drug-resistant cancer cells. Various cancer drugs (Abiraterone acetate, Enzalutamide, Docetaxel) are known to have only very moderate effects on lowering the cell survival rate and thus suppressing the growth of the resistant cancer cells. However, when exposed to a THSD, compare **FIG. 4A-C**, at moderate concentrations of about 16 µM or less and within only 8 hours, not only can the growth of parental (i.e. before induction of resistance) cancer cells be suppressed completely (0% cell survival rate after 8h, **see** **FIG. 4A**), but in resistant cells, using the same THSD concentration and within the same short time, complete growth suppression is achieved (0% cell survival rate after 8h, **see** **FIG. 4B** and **FIG. 4C**).

Further, **examples 7A-7I** and **FIG. 9A1-4** illustrate that THSD (including ELSD) provide improvements in inhibiting cell growth of various different human cancer cell lines, including various types of prostate and lung cancer cell lines, including both those established from NSCLC and SCLC, and including drug resistant ones. This similarly applies to ALSD and ELSD, as reduction of cell viability in various human cancer cell lines shown for both illustrates, compare e.g. graphs for 22RV1, MDVR (EZ-resistant C4-2B), and PC3 cancer cells shown in **FIG. 7A****-7C.** Growth inhibition and tumor shrinkage is also shown *in vivo* in a model for human drug-resistant lung cancer (e.g. PC9AR AC/NSCLC cells which are resistant to EGFR-TKI), with the THSD showing no acute toxicity (**FIG. 9A1-4**). A combination with an EGFR-TKI may slightly improve anti-cancer activity further.

IC₅₀ and other cancer cell based data as well as tumor weight/volume and other preliminary data support that the THSD cause stronger cytotoxicity in cancer cells and less side effects (see e.g. results shown in **FIG. 1B-E****,** **FIG. 4A-C**, **FIG. 7A-I**).

Without wishing to be bound by theory, it is believed that the HMCD moiety and the statin moiety when linked as described (and in particular, via the statin's DHHA in open chain form) are able to act in concert to achieve effects that are at least additive and possibly synergistic, and may provide or contribute to improved therapeutic effects of the THSD. This may be due to one or more of the following three types of actions or functions of the THSD conjugate, but not the unconjugated HMCD dye: 1) mitochondrial functions, 2) lysosomal functions, and 3) cell-cell communication through protein prenylation. These three functions or actions occur independently, but collectively contribute to multiple mechanisms of THSD each of which contributes to improved inhibition of the growth of cancer cells.

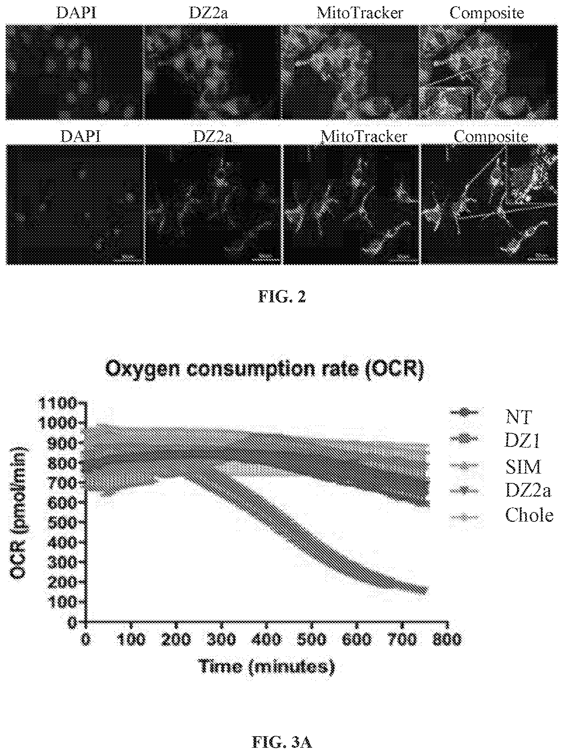

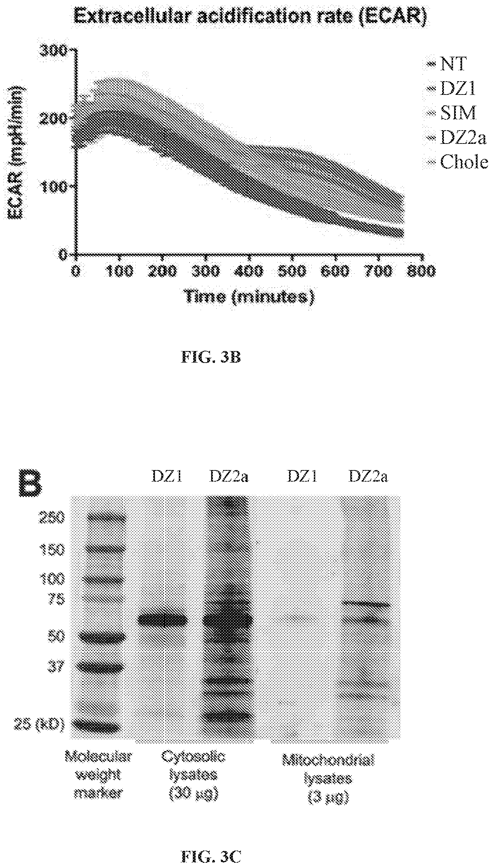

The three functions of the THSD (unlike the unconjugated HMCD dye or the unconjugated statin) are believed to display are supported by the results of **example 2** shown in **FIG. 2****.** With regard to 1) and 2) a staining of cancer cells shows that THSD co-localize with mitochondria and lysosomes, and thus are able to interfere with various mitochondrial and lysosomal functions in cancer cells. While some proteins overlap, the proteins bound differ from those bound by the unconjugated dye both in strength and/or identity, as shown by additional bands appearing in **FIG. 3C****. Examples 3A-D** and **FIG. 3A-D** show an incredible improvement in lowering the oxygen consumption rate (OCR) of cancer cells by THSD when compared to the unconjugated dye/statin, or controls (down from about 800-900 pmol/min to less than 200 for the THSD, with the unconjugated drugs and controls stay above 700, see e.g. **FIG. 3A**).

Also with regard to 1) and corresponding with a strongly decreased OCR, the THSD also show a decrease of the extracellular acidification rate ("ECAR"), as shown in **FIG. 3B****.** The ECAR corresponds to the use of anaerobic glycolysis, which in its anaerobic form produces and accumulates lactic acid extracellularly, thus the higher rate of extracellular acidity build-up by lactic acid. While all samples show a decrease in ECAR (anaerobic ATP production), the least decrease is exhibited by the THSD-exposed cells ("DZ2a"), which also show an upwards bump in the curve at around 500 minutes before the decrease is resumed; all other samples show a gradual decrease in ECAR, with the curve of the negative control ("NT") being lowest, followed by that of the unconjugated statin; the other curves (not visible) show a similar decrease and gradual trend; all these samples show a high OCR (i.e. have a high aerobic respiration), in contrast to THSD treated cells. A lowering of the ECAR typically indicates less anaerobic glycolysis (and more aerobic respiration instead). The anaerobic production of ATP, i.e. via the anaerobic glycolysis/lactic acid system, is an alternative means of cell survival that is particularly important for cancer cells and especially in solid tumors where oxygen may not be readily available. As shown, THSD effects physiological changes in cancer cells which include a strong reduction of aerobic but (after an initial attempt to switch) also anaerobic ATP production, which contributes to inhibit the growth of cancer cells exposed to THSD.

Further with regard to 2), THSD prevent the removal/lysosomal clearance of polyubiquitinated cell proteins by cancer cells, as illustrated in **FIG. 3D****.**

With regard to 3), as demonstrated by the results described in **example 5,** THSD can prevent the anchoring of RhoA/B onto cell membranes by inhibiting their prenylation and thus block their downstream signalling. Protein prenylation is a post-translational mechanism of protein modification of e.g. RhoA/B to help anchor these proteins onto cancer cell membranes (e.g. in lung and prostate cancer cells) for downstream cellular communication. THSD can inhibit HMG CoA reductase, thus inhibiting its production of both cholesterol and isoprenoids. Cholesterol and isoprenoids are required for protein prenylation but missing due to THSD-mediated HGM CoA inhibition, thus blocking downstream signaling of the prenylated/anchored proteins.

Thus unlike the unconjugated statins which primarily act on host liver HMG-CoA reductase (and only have limited if any effect on cancer cells), THSD may have at least three independent actions/functions (mitochondrial, lysosomal and cell-cell communication). These may occur independently, but may collectively contribute to inhibiting the growth of cancer cells.

Without wishing to be bound by theory, THSD thus may provide various advantages compared statins in their unconjugated form, compared to unconjugated HMCD, and/or compared to conjugated statin pro-drugs that rapidly release the statin. These fast-release conjugates include in particular fast-release ester conjugates that are directly linked to a lactone form of the DHHA, such as the fast-release ester conjugates (see e.g. DZ1-SIM in **FIG. 1A-V**). Compared to the unconjugated dyes/statins, RRE, and/or other cancer drugs typically used, the THSD have a multitude of advantages that may include one or more of: avoiding or reducing inactivation of the statin (especially *in vivo*), a reduced general cytotoxicity in normal cells combined with an increased cytotoxicity in cancer cells (as shown e.g. by the IC₅₀), increased anti-cancer effectiveness (e.g. growth inhibition, tumor shrinkage, more rapid growth inhibition/cell death of cancer cells, e.g. less than 16, 12, 10, or 8 hours, even when tested on drug-resistant cells (see e.g. **example 4** and **examples B1-B3** and **FIG. 4A-C**), thus avoiding the development of drug resistance), shorter duration of THSD administration, less frequent administration schedule of the THSD (including e.g. just once, once-weekly, bi-weekly, monthly etc.), decreased side effects (particularly when administered systemically), reduced future risk (such as risk for cancer, metastasis and chemotherapeutical induced disease), decreased and/or slower drug inactivation (particularly when administered systemically), an improved plasma and/or elimination half-life (e.g. of less than 8, 4, 2, 1 hour or 30 minutes, e.g. about 1-2 hours), increased plasma circulation time, increased tumor residence time (e.g. longer than 1, 2, 3, 4 weeks, or longer), and an improved dose-response curve, in particular, a less steep sigmoidal dose response curve.

Without wishing to be bound by theory, it is believed that ELSD may have a longer tumor residence time compared to the rapid release ester (RRE). The increased tumor residence time together with a less steep sigmoidal dose-response curve (and thus lower dose at which an initial non-maximal effect occurs) may provide a sufficient therapeutic effect for the THSD. Thus the ELSD may be administered either alone, or may be administered together with a dose of ALSD that is lower than the dose that provides the maximal effect when administered on its own, or may be co-administered as described herein, e.g. as one or more subsequent maintenance dose following one or more initial ELSD loading dose.

In addition, specific advantages of the ELSD may include the slow continuous non-systemic statin release in cancer cells and/or tumor tissue that allows systemic administration in form of the ELSD while avoiding or reducing statin side effects associated with systemic administration of an unconjugated statin. Without wishing to be bound by theory, this may provide additional or improved anti-cancer effects not provided by a "no release" type HMCD conjugate, including an ALSD. Again without wishing to be bound by theory, additional or improved anti-cancer effects may be provided by the particular L_{E} linker, in combination with the dye and statin moieties linked as described, including the DHHA of the statin fixated in open chain form.

Furthermore, surprisingly, the ELSD seems to provide a sigmoidal dose-response curve that is much less steep compared to the ALSD, as indicated by preliminary data and e.g. in **FIG. 7D****,** **FIG. 7E****,** and **FIG. 7H****.** This means for the range of doses which provide an initial effect, the ELSD provides this initial effect at a lower dosages, at which the ALSD does not have any effect yet (but may cause side effects). Similarly, looking at the range of higher doses that provide maximum effect, the ALSD provides the maximum effect at a lower dose while more of the ELSD is needed. Thus administration of the ELSD alone or in combination with an ALSD, in particular according to the dosing and administration schedules described herein, may provide an improved therapy that provides increased cancer growth inhibition and/or reduced side effects, and allows to use a reduced dose of more toxic drug alternatives, including the ALSD, and/or less frequent drug doses e.g. co-administering ELSD and ALSD in a particular ratio, amount and frequency adjusted to capture both the initial effect of the ELSD and the maximum effect of the ALSD.

For example, the advantages and improvements of the THSD allow for an improved administration schedule with less frequent instances of drug administration, including a co-administration schedule wherein an ALSD and an ELSD are combined. For example, without limitation, the ELSD may be administered first, providing a loading dose, which is followed by one or more subsequent maintenance doses of an ALSD.

In embodiments, provided is a THSD compound of formula FIa shown below (also designated "ELSD" herein), wherein the A⁻ group is a pharmaceutically acceptable negatively charged anion; wherein X is a halogen; wherein R₁ is an alkyl residue optionally terminally substituted with a residue selected from SO₃, CO₂ or NH₂, and wherein each R₁ alkyl chain may optionally be branched, and the branch may constitute one or more of an alkyl chain, aryl ring, heteroaryl group, aralkyl group, and wherein one or more positions of the chain or of a branch thereof may be unsaturated; wherein R₂ and R₃ are independently selected from H, an electron withdrawing group (EWG), or an electron donating group (EDG); wherein L_{E} is selected from the group consisting of an alkyl linker L_{E1} and a carboxamide linker L_{E2} and wherein L_{E1} is -(CH₂)ₙ-O- and wherein L_{E2} is -(CH₂)ₙ-CO-NH-(CH₂)ₘ-O-, wherein n=4-9, e.g. 4-6, and m=1-4, and wherein L_{E} and the statin residue R_{S} are thus linked by an ester bond, and wherein R_{S} is a statin residue linked via its -CO-CH₂-COH-CH₂-CHOH-R moiety:

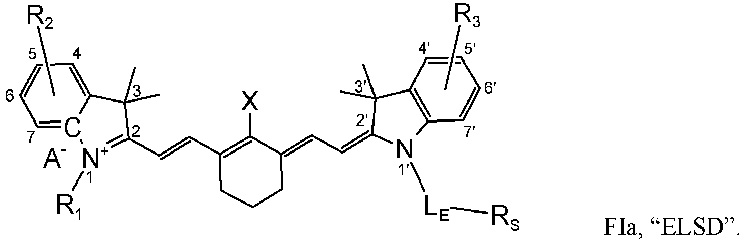

In embodiments, methods are provided that use a THSD compound of formula FIIa shown below (also designated "ALSD"), wherein the residues are as designated for formula FIa above, and wherein L_{A} is selected from the group consisting of an alkyl linker L_{A1} and a carboxamide linker L_{A2} and wherein L_{A1} is -(CH₂)ₙ-NH- and wherein L_{A2} is -(CH₂)ₙ-CO-NH-(CH₂)ₘ-NH-, wherein n=4-9, e.g. 4-6, and m=1-4, and wherein L_{A} and the statin residue R_{S} are thus linked by an amide bond, and wherein R_{S} is a statin residue linked via its -CO-CH₂-COH-CH₂-CHOH-R moiety:

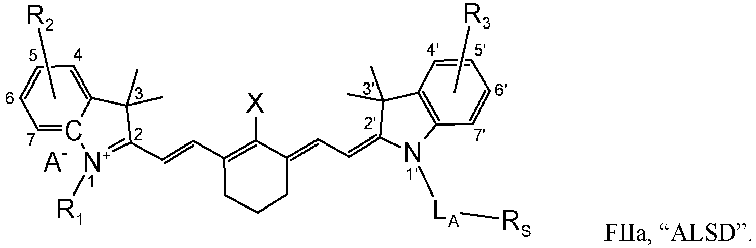

In embodiments, THSD are provided wherein n=5, and preferably the linker is an L_{E2} or L_{A2} linker with n=5 and m=2; optionally, X, R₁, R₂ and R₃ may be chosen as described above or selected as follows: X=Cl, R₁ = (CH₂)₄-SO₃⁻, R₂ =H, R₃ = H (e.g. DZ1-based THSD).

In embodiments, THSD are provided wherein the A⁻ group may be selected from the group comprising I⁻, Cl⁻, Br⁻, OSO₂R⁻, BF₄⁻, ClO₄⁻.

In embodiments, THSD are provided wherein R_{S} is a residue of a statin, or a derivative thereof which retains binding to the statin's active binding site. Statins comprise a dihydroxyheptanoic acid unit (DHHA), typically a 3,5-dihydroxy HA unit, more specifically a 3R,5R-dihydroxy HA unit, which is the statin's key pharmacophore; the DHHA may be present in a statin either in form of its inactive closed ring lactone form, or its active open chain carboxylic acid form, i.e. COOH-CH₂-CHOH-CH₂-CHOH-CH₂-CH₂-Rs* (or when linked: HMCD-L_{E/A}-CO-CH₂-CHOH-CH₂-CHOH-CH₂-CH₂-R_{s*}), with R_{S*} being the remainder of the statin. Upon forming the THSD as described herein, the open chain form is present and linked, or formed and fixated upon linkage.

In embodiments of the THSD, the R_{S} statin residue may be linked via a residue of its DHHA unit in its open chain carboxylic acid form to the L_{E}/L_{A} linker, via an ester bond (L_{E}) or an amide bond (L_{A}), respectively.

In embodiments, ELSD of formula FIb are provided wherein an HMCD dye residue is conjugated to a statin via an L_{E2} linker (-(CH₂)ₙ-CO-NH-(CH₂)ₘ-O-) as shown below for statins having a dihydroxyheptanoic acid unit (DHHA), i.e. a statin residue -CO-CH₂-CO-CH₂-CHOH-R_{s*} with R_{s*} designating the statin residue minus the DHHA residue, and thus remainder of the statin molecule:

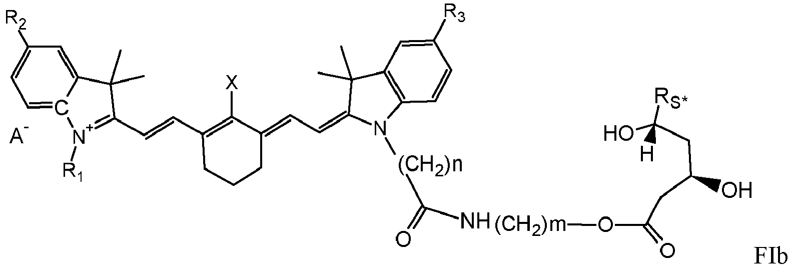

In embodiments, ELSD of formula FIc are provided wherein the linker is an L_{E2} linker as above and the statin is Simvastatin:

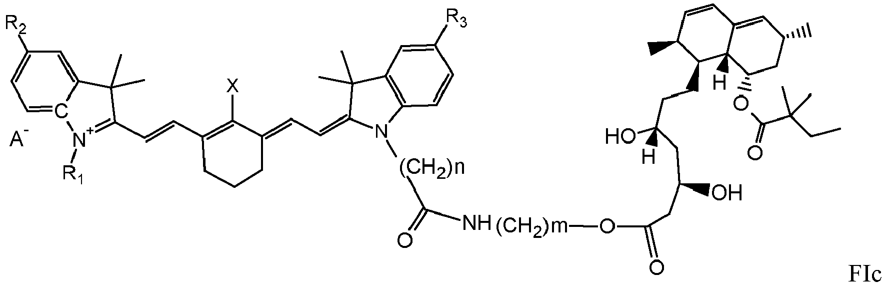

In embodiments, ELSD of formula FId are provided wherein the statin is Simvastatin as above, and the dye is a DZ1-based dye wherein R₁ is (CH₂)₄-SO₃⁻, wherein the linker is an L_{E2} linker with n=5 and m=2, wherein X=Cl, and wherein R₂ and R₃ are H: 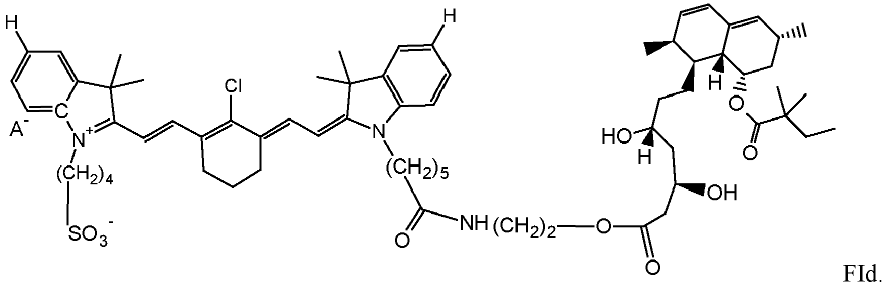 The synthesis of the compound of formula FId is described herein below in example 1A-IV, compare compound 14 (DZ2b).

In embodiments, ALSD of formula FIIb are provided wherein an HMCD dye residue is conjugated to the DHHA of a statin via an L_{A2} linker (-(CH₂)ₙ-CO-NH-(CH₂)ₘ-NH-) as shown in FIIb below:

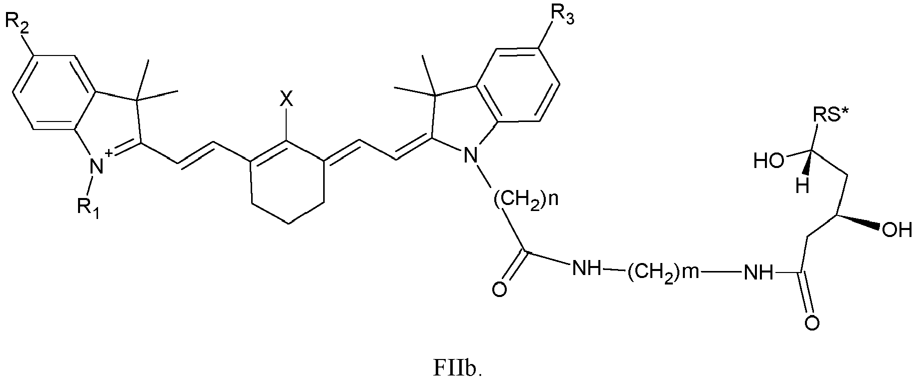

In embodiments, ALSD of formula FIIb are provided wherein an HMCD residue is conjugated to Simvastatin via the L_{A2} linker as shown in FIIc below:

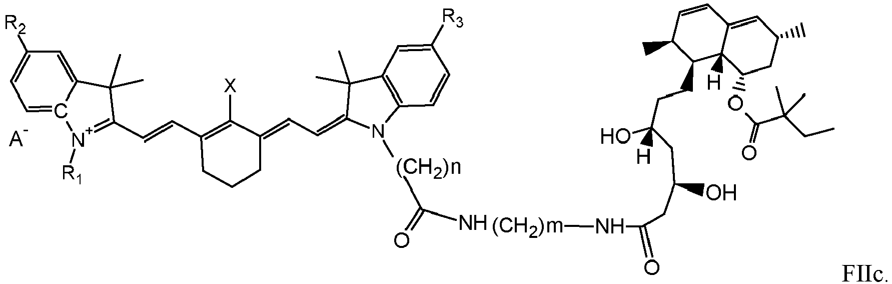

In embodiments, ALSD of formula FIb or FIIb as described herein are provided wherein an HMCD residue is conjugated to Simvastatin via an L_{A} linker, e.g. L_{A1} or L_{A2}, and wherein X=Cl.

In embodiments, THSD of formula FIb or FIIb as described herein are provided wherein n=5.

In embodiments, THSD of formula FIb or FIIb as described herein are provided wherein R₁ is (CH₂)₄-SO₃⁻.

In embodiments, THSD of formula FIb or FIIb as described herein are provided wherein R_{2/3} are H.

In embodiments, THSD of formula FIb or FIIb as described herein are provided wherein n=5, R₁ is (CH₂)₄-SO₃⁻ , and R_{2/3} are H (DZ1-based THSD).

In embodiments, THSD of formula FIb or FIIb as described herein are provided wherein n=5 and m=3.

As an illustrative example, formula FIId below shows an ALSD wherein an HMCD is conjugated to Simvastatin via an L_{A2} linker wherein n=5 and m=3 (-(CH₂)₅-CO-NH-(CH₂)₃-NH-), wherein X=C1, R₁ is (CH₂)₄-SO₃⁻, and R_{2/3} are H): 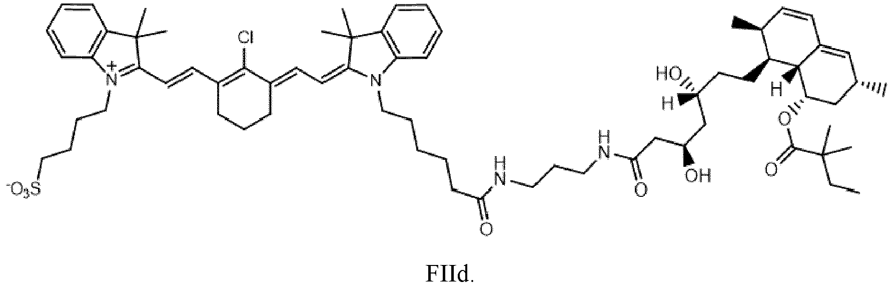 The synthesis of the compound of formula FIId is described herein below in example 1A-**IV,** compare compound 7 ("DZ2a").

For comparative purposes, Formulae FIII and FIV below show HMCD-statin conjugates of the Rapid Release Ester (RRE) type (FIV is compound **8** of comparative **example 1A-IV,** also designated "DZ2c" herein). The RRE are missing the more stable L_{E}/L_{A} linkers, and instead comprise a labile L_{RR} linker (e.g. of general formula -(CH₂)ₙ-OC-O-), forming a labile ester bond to a statin, e.g. with a residue of the statin's DHHA unit in its lactone form. This labile ester rapidly releases the statin under typical *in vivo* conditions, and significant release may occur during a short period of time such as one hour or less, e.g. 30 minutes or less, or even 10 minutes or less.

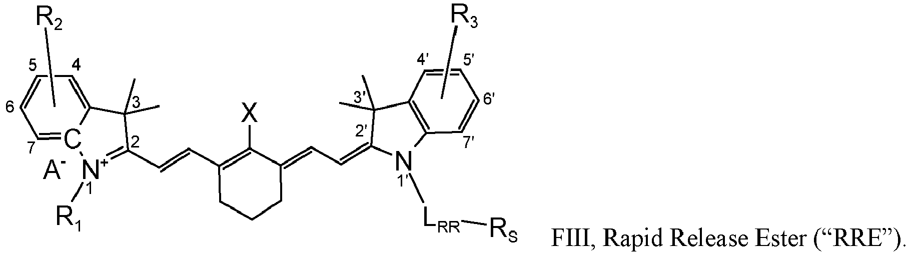

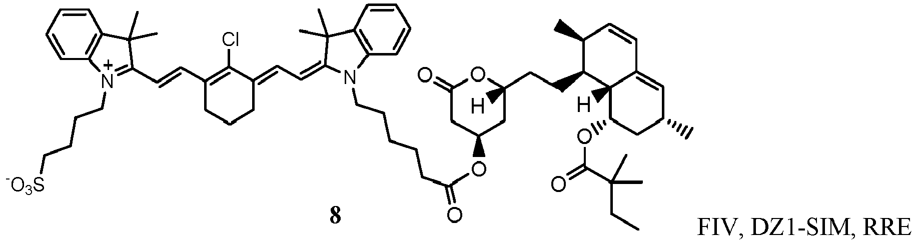

In embodiments, to form a THSD, the method may start from a HMCD such as DZ1, comprising a carboxyl group. An amine or diamine may be reacted with either the carboxyl group of the HMCD or with a carboxyl group of the DHHA unit of the statin, to form part of the L_{E}/L_{A} linker to be formed, and then reacting a resulting amide group with a carboxyl group of the remaining moiety (i.e. that of the dye or of the statin), to provide the desired THSD with the L_{E}/L_{A} linker linking a HMCD to a statin.

For example, to form an **ELSD,** a hydroxyalkylamine may be reacted with the HMCD, to provide the desired L_{E} linker upon reaction with the carboxyl group of the DHHA unit of a statin. The statin may be activated accordingly, as illustrated in **FIG. 1A-IV** which illustrates a DZ1-based method to form an exemplary ELSD (compound **14,** DZ2b). For example, as a first step, a hydroxyethylamide derivate of a HMCD is formed, e.g. by reacting the HMCD with ethanolamine to form an HMCD-hydroxyethylamide. In a subsequent step, the HMCD-hydroxyethylamide is reacted with a methyl derivate of the desired statin. Also compare the exemplary method illustrated in **FIG.1** **AIII** for the DZ1 and Simvastatin.

To form an **ALSD,** for example, an alkyl-diamine may be reacted with the desired statin to provide a statin-amid derivate, to provide the desired L_{A} linker upon reaction with a carboxyl group of the HMCD located in a position corresponding to L_{A} as illustrated in **FIG. 1A-III** for DZ1 and Simvastatin. For example, as a first step, an amine derivate of a statin is formed, e.g. by reacting the statin with an alkyl-diamine such as propane-1,3-diamine to form the amine derivate. In a subsequent step, the introduced amine group of the statin derivate is reacted with the carboxyl group of the HMCD, in particular a terminal carboxyl group in the R_{4*} residue position (which upon reaction with the alkyl-diamine is to become part of the L_{A} linker of the THSD). Also compare the exemplary method illustrated in **FIG. 1A**-**III** for DZ1 and Simvastatin.

In embodiments, the THSD may be a THSD of formula FIa or FIIa, and R₁ may be selected, for example, as indicated in the illustrative list below, and optionally, X may be a halogen selected from, e.g., without limitation, Cl and Br.

| | | |
|---|---|---|
| 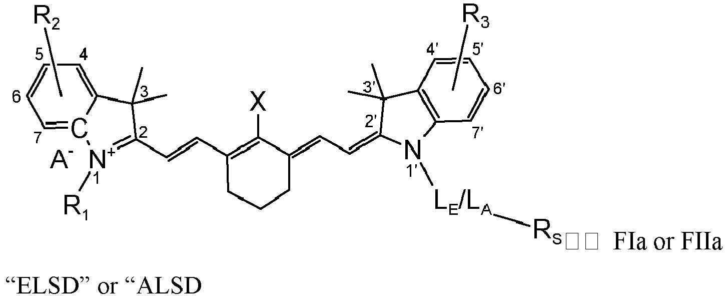 | | |

| X | R₁ | R₂/R₃** |
|---|---|---|
| Halogen, Cl, Br | Methyl | H, EDG, EWG |
| Halogen, Cl, Br | Ethyl | H, EDG, EWG |
| Halogen, Cl, Br | Propyl | H, EDG, EWG |
| Halogen, Cl, Br | Butyl * | H, EDG, EWG |
| Halogen, Cl, Br | Pentyl* | H, EDG, EWG |
| Halogen, Cl, Br | Hexyl* | H, EDG, EWG |
| Halogen, Cl, Br | Heptyl* | H, EDG, EWG |
| Halogen, Cl, Br | Octyl* | H, EDG, EWG |
| Halogen, Cl, Br | Nonyl* | H, EDG, EWG |
| Halogen, Cl, Br | Decyl* | H, EDG, EWG |
| Halogen, Cl, Br | Undecyl * | H, EDG, EWG |
| Halogen, Cl, Br | Dodecyl* | H, EDG, EWG |
| Halogen, Cl, Br | Tridecyl * | H, EDG, EWG |
| Halogen, Cl, Br | Tetradecyl* | H, EDG, EWG |
| Halogen, Cl, Br | Pentadecyl* | H, EDG, EWG |
| Halogen, Cl, Br | Hexadecyl* | H, EDG, EWG |
| Halogen, Cl, Br | Heptadecyl* | H, EDG, EWG |
| Halogen, Cl, Br | Octadecyl* | H, EDG, EWG |
| Halogen, Cl, Br | CH₂-SO₃⁻ | H, EDG, EWG |
| Halogen, Cl, Br | (CH₂)₂-SO₃⁻ | H, EDG, EWG |
| Halogen, Cl, Br | (CH₂)₃-SO₃⁻ | H, EDG, EWG |
| Halogen, Cl, Br | (CH₂)₄-SO₃⁻ | H, EDG, EWG |
| Halogen, Cl, Br | (CH₂)₅-SO₃⁻ | H, EDG, EWG |
| Halogen, Cl, Br | (CH₂)₆-SO₃⁻ | H, EDG, EWG |
| Halogen, Cl, Br | (CH₂)₇-SO₃⁻ | H, EDG, EWG |
| Halogen, Cl, Br | (CH₂)₈-SO₃⁻ | H, EDG, EWG |
| Halogen, Cl, Br | (CH₂)₉-SO₃⁻ | H, EDG, EWG |
| Halogen, Cl, Br | (CH₂)₁₀-SO₃⁻ | H, EDG, EWG |
| Halogen, Cl, Br | (CH₂)₁₁-SO₃⁻ | H, EDG, EWG |
| Halogen, Cl, Br | (CH₂)₁₂-SO₃⁻ | H, EDG, EWG |
| Halogen, Cl, Br | (CH₂)₁₃-SO₃⁻ | H, EDG, EWG |
| Halogen, Cl, Br | (CH₂)₁₄-SO₃⁻ | H, EDG, EWG |
| Halogen, Cl, Br | (CH₂)₁₅-SO₃⁻ | H, EDG, EWG |
| Halogen, Cl, Br | (CH₂)₁₆-SO₃⁻ | H, EDG, EWG |
| Halogen, Cl, Br | (CH₂)₁₇-SO₃⁻ | H, EDG, EWG |
| Halogen, Cl, Br | (CH₂)₁₈-SO₃⁻ | H, EDG, EWG |
| Halogen, Cl, Br | CH₂-CO₂⁻ | H, EDG, EWG |
| Halogen, Cl, Br | (CH₂)₂-CO₂⁻ | H, EDG, EWG |
| Halogen, Cl, Br | (CH₂)₃-CO₂⁻ | H, EDG, EWG |
| Halogen, Cl, Br | (CH₂)₄-CO₂⁻ | H, EDG, EWG |
| Halogen, Cl, Br | (CH₂)₅-CO₂⁻ | H, EDG, EWG |
| Halogen, Cl, Br | (CH₂)₆-CO₂⁻ | H, EDG, EWG |
| Halogen, Cl, Br | (CH₂)₇-CO₂⁻ | H, EDG, EWG |
| Halogen, Cl, Br | (CH₂)₈-CO₂⁻ | H, EDG, EWG |
| Halogen, Cl, Br | (CH₂)₉-CO₂⁻ | H, EDG, EWG |
| Halogen, Cl, Br | (CH₂)₁₀-CO₂⁻ | H, EDG, EWG |
| Halogen, Cl, Br | (CH₂)₁₁-CO₂⁻ | H, EDG, EWG |
| Halogen, Cl, Br | (CH₂)₁₂-CO₂⁻ | H, EDG, EWG |
| Halogen, Cl, Br | (CH₂)₁₃-CO₂⁻ | H, EDG, EWG |
| Halogen, Cl, Br | (CH₂)₁₄-CO₂⁻ | H, EDG, EWG |
| Halogen, Cl, Br | (CH₂)₁₅-CO₂⁻ | H, EDG, EWG |
| Halogen, Cl, Br | (CH₂)₁₆-CO₂⁻ | H, EDG, EWG |
| Halogen, Cl, Br | (CH₂)₁₇-CO₂⁻ | H, EDG, EWG |
| Halogen, Cl, Br | (CH₂)₁₈-CO₂⁻ | H, EDG, EWG |
| Halogen, Cl, Br | CH₂-NH₂ | H, EDG, EWG |
| Halogen, Cl, Br | (CH₂)₂- NH₂ | H, EDG, EWG |
| Halogen, Cl, Br | (CH₂)₃- NH₂ | H, EDG, EWG |
| Halogen, Cl, Br | (CH₂)₄- NH₂ | H, EDG, EWG |
| Halogen, Cl, Br | (CH₂)₅- NH₂ | H, EDG, EWG |
| Halogen, Cl, Br | (CH₂)₆- NH₂ | H, EDG, EWG |
| Halogen, Cl, Br | (CH₂)₇- NH₂ | H, EDG, EWG |
| Halogen, Cl, Br | (CH₂)₈- NH₂ | H, EDG, EWG |
| Halogen, Cl, Br | (CH₂)₉- NH₂ | H, EDG, EWG |
| Halogen, Cl, Br | (CH₂)₁₀-NH₂ | H, EDG, EWG |
| Halogen, Cl, Br | (CH₂)₁₁-NH₂ | H, EDG, EWG |
| Halogen, Cl, Br | (CH₂)₁₂-NH₂ | H, EDG, EWG |
| Halogen, Cl, Br | (CH₂)₁₃-NH₂ | H, EDG, EWG |
| Halogen, Cl, Br | (CH₂)₁₄-NH₂ | H, EDG, EWG |
| Halogen, Cl, Br | (CH₂)₁₅-NH₂ | H, EDG, EWG |
| Halogen, Cl, Br | (CH₂)₁₆-NH₂ | H, EDG, EWG |
| Halogen, Cl, Br | (CH₂)₁₇-NH₂ | H, EDG, EWG |
| Halogen, Cl, Br | (CH₂)₁₈-NH₂ | H, EDG, EWG |

| | | |
|---|---|---|
| * Each alkyl chain may optionally be branched, and the branch may constitute one or more of an alkyl chain, aryl ring, heteroaryl group, aralkyl group; one or more positions of the chain or of a branch may be unsaturated. ** The R₂ and R₃ group may be independently selected from H, an electron withdrawing group (EWG), or an electron donating group (EDG). Example R₂/R₃ groups are indicated in the table further below. | | |

In embodiments, THSD of formula FIa or FIIa are provided wherein R₁ = (CH₂)₄-SO₃⁻ and X=Cl. In embodiments, THSD of formula FIa or FIIa are provided wherein R₁ = (CH₂)₄-SO₃⁻ , X=C1, and R₂/R₃ are independently selected as indicated below.

| | |
|---|---|
| 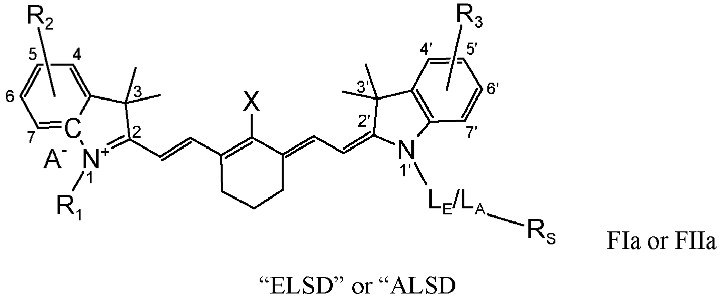 | |

| X, R₁ | R₂ /R₃ |
|---|---|
| see table above | H |
| see table above | OCH₃ |
| see table above | SCH₃ |
| see table above | NH₂ |
| see table above | NHCH₃ |
| see table above | N(CH₃)₂ |
| see table above | NHCOCH₃ |
| see table above | SH |
| see table above | OH |
| see table above | F |
| see table above | Cl |
| see table above | Br |
| see table above | I |
| see table above | CH₃ |
| see table above | CH₂CH₃ |
| see table above | (CH₂)₂CH₃ |
| see table above | NO₂ |
| see table above | CN |
| see table above | COOH |
| see table above | COOCH₃ |
| see table above | COOCH₂CH₃ |
| see table above | CF₃ |
| see table above | CCl₃ |
| see table above | SO₃H |
| see table above | PO₃H |

In embodiments, THSD of formula FIa or FIIa are provided wherein the statin is conjugated to a L_{E} or L_{A} linker residue of the THSD. Preferably, the statin is connected via its dihydroxyheptanoic acid unit in open chain form as shown e.g. in **FIG. 1A-I****.**

In embodiments, a statin residue is connected as residue R_{S} to the linker (L_{E} or L_{A}, respectively), via its dihydroxyheptanoic acid unit (DHHA), typically a 3R,5R-dihydroxyheptanoic acid unit, which is the key pharmacophore. The DHHA is a modified hydroxyglutaric acid component that is structurally similar to the endogenous substrate HMG CoA. Some statins comprise this unit in open-ring acid form (e.g. Atorvastatin, Fluvastatin, Rosuvastatin, Cerivastatin, Pravastatin, Pitavastatin), others comprise it as a closed ring lactone form (e.g. Simvastatin, Lovastatin, Mevastatin). In the THSD of the present invention, the statin is connected to the linker via the terminal carboxyl residue of its DHHA unit and fixated into a form corresponding to the DHHA's "open acid" form (compare e.g. in **FIG. 1A-I**), unlike the linkage occurring in the RRE ester.

In embodiments, the statin may be selected as indicated in the left column of the table below, to provide the R_{S*} residue as indicated in the right column.

| **Statin** | **R_{S*}** | **R_{S}** (incl. DHHA) |
|---|---|---|
| Simvastatin (lactone DHHA) | | 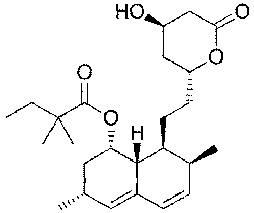 |
| Mevastatin (lactone DHHA) | 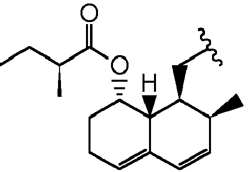 | 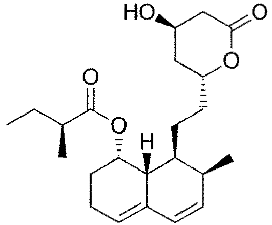 |
| Lovastatin (lactone DHHA) | 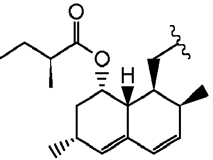 | 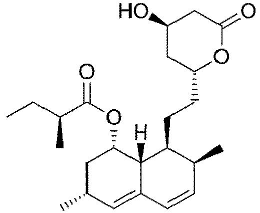 |
| Pravastatin | | 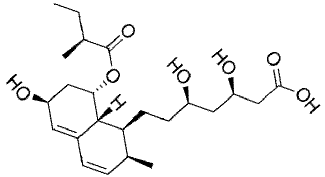 |
| Atorvastatin | | 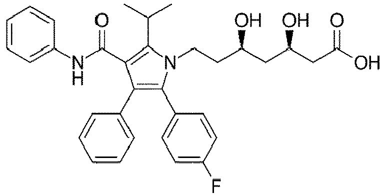 |
| Fluvastatin | | 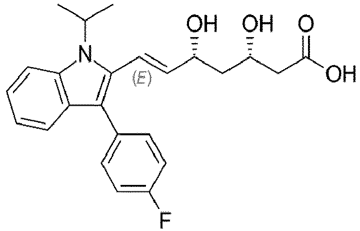 |
| Rosuvastatin | 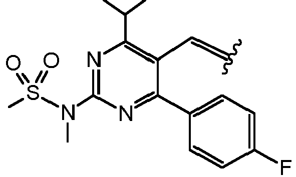 | 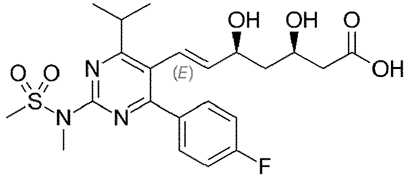 |
| Cerivastatin | 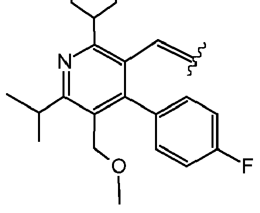 | 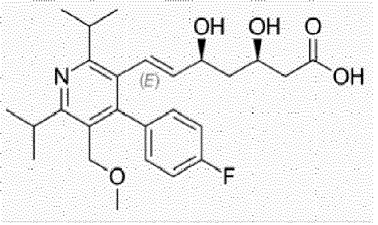 |
| Pitavastatin | 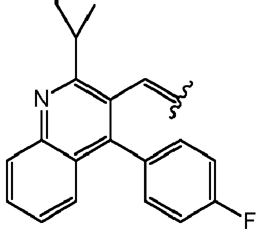 | 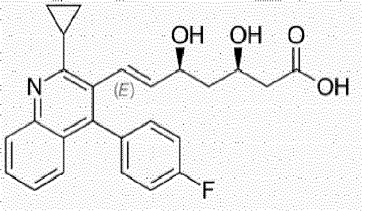 |

In embodiments, the THSD comprise a statin residue (R_{S}), wherein the corresponding statin to form said residue as described herein may be selected from the group comprising Simvastatin, Mevastatin, Lovastatin, Pravastatin, Atorvastatin, Fluvastatin, Rosuvastatin, Cerivastatin, Pitavastatin, and derivatives thereof which retain binding to the statin's active binding site. Statins are a well-known group of structurally related HMG-CoA analogues that competitively inhibit HMG-CoA reductase enzyme with respect to the binding of the substrate, HMG-CoA. Statins generally act by binding to the active site of the enzyme, sterically preventing the substrate from binding, i.e. the HMG-CoA-like moieties of the respective statin binds to the HMG-CoA-binding portion of the enzyme active site, while the enzyme undergoes a rearrangement that enables the rigid, hydrophobic ring structures of the statins to be accommodated.

The structure of a statin generally comprises three parts: 1) HMG-CoA analogue, 2) Hydrophobic ring structure that is involved in enzyme binding, 3) Side groups on the rings that influence the solubility and pharmacokinetic properties (for example, Simvastatin, Atorvastatin, Fluvastatin, and Lovastatin are relatively lipophilic, while Pravastatin and Rosuvastatin are more hydrophilic).

Surprisingly it has been found that the much larger THSD are able to provide one or more of the effects provided by statin drugs (lowering cholesterol, reducing the risk of certain cancers), and/or additional effects as described herein, while avoiding one or more side effects typically associated with the statin, or with a combination of the statin and one or more other drug/secondary active (in particular chemotherapeutic drugs such as, e.g., cisplatin and its derivatives, gemcitabine, doxorubicin, taxane drugs such as, e.g., paclitaxel and docetaxel, and anti-androgenic drugs, such as, e.g., Abiraterone and Enzalutamide).

Side effects associated with statins include for example, without limitation: headache, pins and needles, nausea, dizziness, drowsiness, stomach upset, vomiting, abdominal pain, abdominal cramping, bloating, diarrhea, difficulty sleeping, feeling sick, rashes and rarely, j oint pain, j oint inflammation, muscle aches, tenderness, pains or weakness (myalgia), tenderness, muscle cramps, muscle inflammation, flushing of the skin, increased risk of cataracts, abnormal liver function tests, liver problems (increased levels of liver enzymes), liver failure, increased risk of diabetes, skeletal muscle damage, rhabdomyolysis (especially when statins are used in combination with other drugs that carry high rhabdomyolysis risk). The common side effects of statins include e.g. joint pain, diarrhea, nausea, vomiting, abdominal cramping or pain, dizziness, drowsiness, stomach upset, headaches, difficulty sleeping, abnormal liver function tests, muscle aches, tenderness, pains or weakness (myalgia) and muscle cramps, flushing of the skin. Serious side effects of statins include e.g. rhabdomyolysis, liver problems/failure, and diabetes.

Drug-Drug Interactions (DDI) associated with the combination of statins with other drugs include for example, without limitation: rhabdomyolysis, e.g. due to a combination with other drugs that carry high rhabdomyolysis risk, and/or in combination with drugs that raise the concentration of statins in the blood.

In embodiments, methods are provided wherein one or more THSD is administered to patients or patient groups at elevated risk for development of tumors or cancer, to lower their risk to develop cancers or tumors, or to slow the growth of existing tumors or prevent existing tumors from growing and/or spreading. These may include patients/patient groups who have or are at increased risk for cancer (in particular e.g. lung cancer and breast cancer), as determined by biomarkers or risk profile, including e.g. environmental risks (smoking, exposure to chemicals, occupational exposure to dust) or a family history for one or more particular cancer, e.g. breast cancer, and/or detection of potential pre-cancerous lesions, e.g. breast nodules.

In embodiments, methods are provided wherein one or more THSD is administered to a patient group typically prescribed statins for lowering the risk of developing one or more cancer, the cancer including, without limitation: esophageal cancer, colorectal cancer, gastric cancer, hepatocellular carcinoma, and prostate cancer. For these cancers, statins and similarly THSD may be associated with a lowering of their cancer risk, and THSD may avoid statin side effects while lowering risk.

In embodiments, methods are provided wherein one or more THSD is administered to a cancer patient experiencing (or at higher risk for) statin side effects, e.g. patients having liver disease, pregnant women, and breast-feeding women.

In embodiments, methods are provided wherein one or more THSD is administered to a cancer patient experiencing (or at higher risk for) side effects due to drug-drug interactions of statins with other drugs. Such patients further include those taking one or more other drug that may interact with statins, including for example, without limitation: protease inhibitors (AIDS treatment), clarithromycin, erythromycin, itraconazole, clarithromycin, diltiazem, verapamil, grapefruit juice, niacin, or fibrate drugs (that also lower cholesterol LDL levels).

In embodiments, methods for treating cancer or decreasing future risk of cancer (for example by, e.g., improving mitochondrial or lysosomal function, such as, e.g., lowering the mitochondrial oxygen consumption rate (OCR), improving the extracellular acidification rate ("ECAR"), and reducing or preventing the removal of polyubiquinated proteins) are provided.

In embodiments, THSD and methods described herein may be suitable for treatment or risk reduction relating to one or more of the following cancers: prostate cancer, pancreatic cancer, lung cancer, NSCLC (non-small cell lung carcinoma), SCLC (small cell lung carcinoma), kidney cancer, lymphoma, colorectal cancer, skin cancer, HCC cancer, and breast cancer, squamous-cell carcinoma of the lung, anal cancers, glioblastoma, epithelian tumors of the head and neck, and other cancers. Non-small-cell lung carcinoma may include Squamous-cell carcinoma, Adenocarcinoma (Mucinous cystadenocarcinoma), Large-cell lung carcinoma, Rhabdoid carcinoma, Sarcomatoid carcinoma, Carcinoid, Salivary gland-like carcinoma, Adenosquamous carcinoma, Papillary adenocarcinoma, and Giant-cell carcinoma. Small-cell lung carcinoma may include Combined small-cell carcinoma. Non-carcinoma of the lung may include Sarcoma, Lymphoma, Immature teratoma, and Melanoma. Without wishing to bound by theory, it is believed that the THSD have broad applicability for different types of cancers, tumors, and their metastases.

In embodiments, THSD may be particularly beneficial to treat patients with lung cancer, or patients at higher than average risk to develop lung cancer (e.g. smokers). Lung cancers may include the two main types of cancer i.e. non-small cell lung cancers (NSCLC) and small cell lung cancer. The most common lung cancer is adenocarcinoma of the lung (AC) which is one of the three lung cancers of the NSCLC type (the other two NSCLC cancers being squamous cell carcinoma and large cell carcinoma). In contrast to small cell lung cancer (SCLC), NSCL incl. AC typically respond to various milder non-chemotherapeutic treatment options, including TKI. SCLC is the form most strongly related to cigarette smoking and is more difficult to treat, typically requires chemotherapy, and is prone to develop resistance upon chemotherapy administration. THSD may allow treatment of patients despite them having acquired resistance (e.g. against TKI and/or chemotherapeutics), or may avoid development of resistance that occurs in these cancers upon treatment altogether.

In embodiments, methods are provided for treating cancer by administering one or more THSD to a patient presenting with a brain tumor or brain metastase. The THSD may cross the blood-brain barrier (BBB), thus it may provide its anti-cancer effects to brain tumors or brain metastases of various cancers, in particular of the cancers as described herein-above, which may be treated according to the methods of administration as described herein, including in particular systemic administration methods.

In embodiments, a pharmaceutical composition comprising one or more THSD is provided. A plurality of THSD may be administered in a single or in a corresponding plurality of pharmaceutical compositions. A plurality of compositions may be co-administered in a coordinated administration schedule as described herein below. The pharmaceutical composition may be for human or for veterinary use, and comprise one or more compound of the invention (or a salt, solvate, metabolite, or derivative thereof) with one or more pharmaceutically acceptable carrier and/or one or more excipient and/or one or more active. The one or more carrier, excipient and/or active may be selected for compatibility with the other ingredients of the formulation and not unduly deleterious to the recipient thereof. Such carriers are known in the art and may be selected as will be apparent to a person of ordinary skill in the art.

In embodiments, pharmaceutical kits are provided. Such kits can comprise one or more THSD or composition comprising a THSD, preferably in form of its salt, and, typically, a pharmaceutically acceptable carrier. The kit can also further comprise conventional kit components, such as needles for use in injecting the composition(s), one or more vials for mixing the composition components, and the like, as are apparent to those of ordinary skill in the art. In addition, instructions, e.g. as inserts or as labels, indicating quantities of the components, guidelines for mixing the components, and protocols for administration/co-administration, can be included in the kit. In particular, the kit may comprise instructions for a co-administration schedule of a plurality of THSD in a coordinated administration schedule as described herein below.

In embodiments, routes of administration for the THSD and pharmaceutical compositions may be systemic (administration into the circulatory system so that the entire body is affected) or local/tissue-specific, and may be include, but are not limited to: oral, intraperitoneal, subcutaneous, intramuscular, transdermal, rectal, vaginal, sublingual, intravenous, buccal, or inhalational. In some embodiments, the pharmaceutical compositions of the invention contain a pharmaceutically acceptable excipient suitable for rendering the compound or mixture administrable via the above routes of administration. Alternatively, the compounds and pharmaceutical compositions may be administered through urogenital routes, e.g. via internal organs, topical lesions, skin patches, or access by instillation (such as urinary bladder, vaginal cannel), or vaginal channel through implantation, e.g. ring implantation.

Advantageously, in embodiments, the THSD may be administered to patients at a low frequency, avoiding the requirement of multiple daily or daily dosing. Depending on the amount, for example, doses may be administered e.g. every 2, 3, 4, 5, 6, or 7 days, or at longer intervals. Preferably, administration may be once a week. Even longer intervals such as once every 2, 3 or 4 weeks may be possible depending on the amount of THSD administered with each dose and individual patient requirements, including e.g. the degree of desired anti-cancer effects, the patient's cancer type and rapidity of tumor growth or/or spreading of metastases, and the level of side effects considered acceptable. Without wishing to be bound by theory, it is believed this is due to tight HMCD binding of this new statin derivative, which keeps it in the cancer cells for extended periods of time for multiple days, likely weeks or months, thus providing prolonged anti-cancer activity while preventing side effects.

Advantageously, in embodiments, the ELSD and ALSD may be co-administered, e.g. administered in a coordinated administration schedule. In particular, administration of a first loading dose of an ELSD may be followed by one or more subsequent sustaining doses of an ALSD. Further ALSD doses may then be administered as described above for THSD administration, e.g. once every 2-7 days, once weekly, or every 2-4 weeks.

In embodiments, the ELSD and ALSD may be co-administered in a coordinated administration schedule either concurrently or subsequently. For example, a loading dose of the ELSD may be administered first in a first time interval, followed by one or more subsequent maintenance dose(s) of the ALSD at the start of the second time interval. For example, a first amount of about 0.1 mg to about 10 mg ELSD/kg body weight, preferably about 0.1 to about 6 mg/kg, e.g. about 0.1 to about 2.5 mg or about 0.1 mg to about 1.0 mg/kg may be administered to a patient, depending on the route of administration, e.g. either intravenously or orally or by any convenient administration means (loading dose). The ELSD loading dose may be separated into multiple doses during the first time interval, for example, 4 to 72 hours, e.g. about 4 h, 8 h, 16 h, 24 h, 32 h, 48 h, or 72 h. Advantageously, the loading dose of the ELSD is lower than the maintenance dose of the ALSD. Without wishing to be bound by theory, it is believed that the ELSD is effective at a lower concentration compared to the ALSD, and allows to prime to body to experience the THSD effects, while the ALSD, especially when used on its own, may require a higher dose (but at a higher dose may provide a better maximum effect compared to the ELSD).

In embodiments, during a subsequent second and optional further subsequent time intervals, one or more maintenance doses of an ALSD may be administered. For example, the one or more maintenance dose may be administered in an amount, per dose, of about 0.1 mg to about 10 mg ALSD/kg body weight, preferably about 1 to about 10 mg/kg, e.g. about 1 to about 8 mg, about 1 to about 6 mg, about 1 mg to about 4 mg, or about 1 to about 2 mg/kg may be administered to a patient, depending on the route of administration and frequency of administration. The subsequent dose(s) may be a single subsequent dose, or multiple subsequent doses at the same or different intervals, e.g. bi-daily, daily, over 2-7 days, weekly, etc. Preferably, the ELSD loading dose may be administered on day 1, and after about 24 hours on day 2, one or more higher ALSD maintenance dose(s) may be administered as described herein, e.g. in daily or in less frequent time intervals. Each maintenance dose of the ALSD may be higher or lower compared to the loading dose of the ELSD. Suitable ratios of ELSD:ALSD may include, for example, from about 20:1 to about 1:20 (ELSD:ALSD), for example about 10:1 to about 1:10 (ELSD:ALSD), e.g. about 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, or about 10:1 (ELSD:ALSD). Advantageously, the loading dose of the ELSD is lower than the maintenance dose of the ALSD.

Alternatively, the ELSD loading dose may be co-administered concurrently with an ALSD maintenance dose within a first time interval, and at the start of the second time interval may be followed by one or more ALSD maintenance doses as described above. [0002] Still alternatively, the ELSD and ALSD may be co-administered in a particular ratio, either for each dose (including the first dose in the first time interval), or for the maintenance dose(s) starting at the second time interval only. This ratio may be from about 20:1 to about 1:20 (ELSD:ALSD), for example about 10:1 to about 1:10 (ELSD:ALSD), e.g. about 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, or about 10:1 (ELSD:ALSD).

Surprisingly, it was found that THSD have a similar or better growth inhibitory effect on tumors *in vivo* compared to tyrosine kinase inhibitors (TKI), compare e.g. **example 9** and **FIG. 9A1-4****.** Thus, advantageously, THSD may be administered to patients who were administered TKI, which are often associated with rapid development of drug resistance by the TKI-treated cancer cells; alternatively, THSD may be administered instead of a TKI (thus avoiding development of resistance while providing a similar or better growth inhibitory effect compared to the TKI), to patient groups typically benefiting from administration of TKI.

A TKI is an agent or pharmaceutical drug that inhibits tyrosine kinases. Tyrosine kinases are enzymes responsible for the activation of many proteins by signal transduction cascades, including in particular EGFR (EGFR-TKI), ALK (ALK-TKI), and ROS1 (ROS-TKI). For example, the proteins are activated by adding a phosphate group to the protein (phosphorylation), a step that the TKI inhibits. TKI are typically used as anticancer drugs against various cancers to inhibit the growth of the cancer cells (and stop or slow down tumor growth), and/or to induce the cells to undergo apoptosis (cell death), typically resulting in tumor shrinkage. Gene rearrangement events involving the relevant tyrosine kinase receptor gene, e.g. the EGFR, ALK, and ROS1 gene, have been described to occur in various cancers, including lung cancers. Various cancers and tumors, including of the lung (e.g. NSCLC, NSCLC/AC), were found to be responsive to TKI, including one or more of EGFR-TKI, ALK-TKI, and ROS-1-TKI. In all TKI, the emergence of resistance upon TKI administration to cancer patients is common.

TKI, including in particular EGFR-TKI, are used treat various cancers including, in particular, without limitation, non-small cell lung carcinoma (NSCLC).

Epidermal growth factor receptor (EGFR) is a member of the ErbB family of receptors, a subfamily of four closely related receptor tyrosine kinases: EGFR (ErbB-1), HER2/neu (ErbB-2), Her 3 (ErbB-3) and Her 4 (ErbB-4). Mutations affecting EGFR expression or activity may result in many types of cancer, including, e.g., NSCLC, adenocarcinoma of the lung (AC), anal cancers, glioblastoma, epithelial tumors of the head and neck; Cancer-causing mutations include e.g. EGFRvIII (e.g. glioblastoma), other aberrations include amplification or dysregulation. The main activating EGFR-mutations include, without limitation, L858R mutation, deletions (Del) in exon 19 (Del19), and T790M; further mutations include, e.g., E746-A750 deletion, L747-E749 deletion, A750P mutation, and C797S mutation, among others. MET amplification is another mechanism of resistance to both EGFR-TKIs including e.g. AZD9291 and CO1686.

Anaplastic lymphoma kinase (ALK) also known as ALK tyrosine kinase receptor is an enzyme that in humans is encoded by the ALK gene. ALK aberrations play a role in cancers including, e.g., anaplastic large-cell lymphoma, NSCLC, adenocarcinoma of the lung (AC), neuroblastoma, inflammatory myofibroblastic tumor, renal cell carcinomas, esophageal squamous cell carcinoma, breast cancer (in particular the inflammatory subtype), colonic adenocarcinoma, glioblastoma multiforme, and anaplastic thyroid cancer, among others.

Proto-oncogene tyrosine-protein kinase (ROS or ROS1) is an enzyme that in humans is encoded by the ROS1 gene with structural similarity to the ALK protein. ROS is a receptor tyrosine kinase with structural similarity to the anaplastic lymphoma kinase (ALK) protein. Gene rearrangement events involving ROS1 have been described in lung and other cancers, and such tumors have been found to be responsive to TKI.

THSD may be administered to cancer patients, in particular to cancer patient groups that respond to, or are likely to respond to, treatment with tyrosine kinase inhibitors (TKI), based on e.g. tumor type/grading, tumor histology, prior treatment, resistance to one or more TKI, or various biomarkers, including mutations or aberrations in one or more gene that effects the activity of cellular tyrosine kinase receptors (including, e.g., EGFR, ALK, ROS1 and BRAF). Such patient groups are typically treated with the corresponding inhibitor(s), including, e.g., EGFR-TKI, ALK-TKI, ROS-TKI, and BRAF-TKI. Such patient groups include those with, for example, without limitation, non-small cell lung cancer (NSCLC), and in particular NSCLC with adenocarcinoma (AC), which are common types of lung cancer with increasing incidence. A subgroup of NSCLC patients harbors a particular oncogenic driver, namely activating EGFR, ALK-, or ROS1-aberrations (including chromosomal rearrangements, translocations, or mutations); and these oncogenic drivers appear to be almost exclusively present in AC.

EGFR-TKI include, for example, without limitation: Gefitinib, Icotinib, Erlotinib, Brigatinib, Dacomitinib, Lapatinib, Vandetanib, Afatinib, Osimertinib (AZD9291), CO-1686, HM61713, Nazartinib (EGF816), Olmutinib, PF-06747775, YH5448, Avitinib (AC0010), Rociletinib, and Cetuximab; they are used to treat various cancers including in particular lung cancer, NSCLC, colon cancer, metastatic colorectal cancer, and head and neck cancer, among others.

Based on their mechanism, EGFR-TKI can be grouped into three groups: 1st, 2nd or 3rd generation TKI/EGFR-TKI. Examples for 1st generation EGFR-TKI include, e.g., Gefitinib, Icotinib, and Erlotinib. Examples for 2nd generation EGFR-TKI include, e.g., Afatinib and Dacomitinib. 2nd generation EGFR-TKI typically irreversibly bind to the tyrosine kinase of EGFR and other ErbB-family members. Uses for 2nd generation EGFR-TKI include, e.g., first-line treatment of advanced NSCLC harboring activating EGFR mutations. Examples for 3rd generation EGFR-TKI include, e.g., Osimertinib (AZD9291), CO-1686, HM61713, Nazartinib (EGF816), Olmutinib, PF-06747775, YH5448, Afatinib Avitinib (AC0010), and Rociletinib.

All generations of TKI exhibit a tendency for the treated tumor/cancer cells to develop resistance, with 2nd and 3rd generation drugs typically used to treat cancers that harbor mutations in the relevant gene, in particular the epidermal growth factor receptor (EGFR) gene, and/or display resistance to a 1st generation TKI. The grouping is based on mechanism and correspondingly patient group that may best benefit from the drug, and may also determine the risk of developing resistance, e.g. developing one or more of various mutations, in particular EGFR mutations, or mutations that allow to bypass the EGFR-related mechanism. 1st generation EGFR-TKI are effective, e.g., as first-line treatment of advanced NSCLC harboring activating EGFR mutations (deletions in exon 19 (Del19), and exon 21 L858R mutation); further mutations including in particular EGFR T790M resistance mutation (EGFR T790M) emerged in a large number of these patients. The 2nd and 3rd generation EGFR-TKI are designed for improvements over the 1st generation drugs, and in particular, to more potently inhibit EGFR and/or to overcome various mutations developed by patients, in particular after 1st generation treatment, such as EGFR T790M.

Surprisingly, THSD are able to circumvent TKI resistance and provide growth inhibition, as shown e.g. in **example 9** and **FIG. 9A1-4** for TKI resistant tumors established *in vivo* from TKI resistant human cancer cells; thus THSD may be effective in patient groups that exhibit such resistance, in particular, patients and patient groups having undergone therapy with a TKI inhibitor, including a TKI inhibitor of the 1st, 2nd or third generation, and in particular patients suitable for or having undergone therapy with a 3rd generation TKI inhibitor, or with a tumor already TKI or 3rd generation TKI resistant. As shown in **example 9,** this circumvention of TKI resistance may not require co-administration of a TKI (though for a given TKI, co-administration may be beneficial). Administration of THSD alone may thus be effective to provide growth inhibition in cancers prone to development of drug resistance, in particular lung cancers including, without limitation, NSCLC. In particular, THSD alone (i.e. without a TKI) may be effective to provide growth inhibition in NSCLC as an alternative to treatment with a 3rd generation EGFR inhibitor, in particular, osimertinib (AZD9291), or after treatment with Osimertinib, especially after resistance was acquired.

Examples for 1st generation EGFR-TKI include Gefitinib and Erlotinib. Examples for 2nd generation EGFR-TKI include Afatinib and Dacomitinib. 2nd generation EGFR-TKI typically irreversibly bind to the tyrosine kinase of EGFR and other ErbB-family members. Uses for 2nd generation EGFR-TKI include first-line treatment of advanced NSCLC harboring activating EGFR mutations. 3rd generation EGFR-TKI may include, without limitation, one or more of: osimertinib (AZD9291), Rociletinib (CO-1686), HM61713, Nazartinib (EGF816), Olmutinib (HM61713), PF-06747775, YH5448, afatinib, avitinib (AC0010), and ASP8273. 3rd generation EGFR-TKI generally provide efficacy in patients with acquired resistance to 1st or 2nd-generation TKI. 3rd generation EGFR-TKI are typically EGFR-mutant selective and EGFR wild-type (WT) sparing, i.e. their activity against EGFR mutant cells is greater than against EGFR wildtype (WT) cells, e.g. at least 10, 100, 200 times or more greater activity. Also, 3rd generation EGFR-TKI are active against or inhibit both EGFR-activating and resistance mutations, in particular, e.g., the T790M resistance mutation. For example, 3rd generation EGFR-TKI such as Osimertinib, CO-1686, and HM61713 may selectively and irreversibly target both sensitizing/activating EGFR mutations and T790M resistance mutations, while sparing the wild-type EGFR tyrosine kinase.

Osimertinib is a mono-anilino-pyrimidine that selectively and irreversibly targets both sensitizing EGFR mutations and T790M resistance mutations, while sparing the wild-type EGFR tyrosine kinase. Specifically, Osimertinib is substantially less potent at inhibiting phosphorylation of EGFR in wild-type cell lines, e.g. about 100-200 times greater potency against L858R/T790M than wild-type EGFR, and used to treat cancers that developed resistance, or with a tendency to develop resistance, including in particular non-small cell lung carcinoma (NSCLC).

In embodiments, THSD may be administered as described herein to patients with cancers that have a tendency to develop resistance to TKI, to inhibit the growth of cancer cells while avoiding their development of resistance. Such cancers include advanced EGFR, ALK and/or ROS1 mutation-positive tumors, which are common, e.g., in non-small cell lung cancer (NSCLC).

Thus, patient groups that may particularly benefit from THSD administration may include those with activating mutations of EGFR, ALF or ROS1 and/or resistance mutations of EGFR, ALF, or ROS1, including in particular acquired mutations during treatment with a TKI, EGFR-, ALF- or ROS-TKI, including a 1st, 2nd or 3rd generation TKI or EGFR-TKI. For example, patients with acquired T790M EGFR mutations are common among advanced NSCLC patients who progressed after first line EGFR TKI treatment, e.g. with a 1st or 2nd generation EGFR-TKI.

Specifically, THSD may be administered to patient groups typically treated with ALK-TKI (inhibitors to ALK tyrosine kinase receptor or CD246), to avoid development of resistance. Similar to other TKI, the emergence of resistance upon ALK-TKI is common. Also, THSD may be administered to patients after treatment with an ALK-TKI, to overcome acquired resistance to the ALK-TKI. Patient groups include those with cancers that are positive for genetic ALK aberrations, such as, e.g., metastatic NSCLC. Patient groups with ALK-positive cancers, in particular NSCLC, generally include non-smokers, those of younger age, adenocarcinoma histology, female gender and/or with pathological features that include a solid morphology and/or presence of signet ring cells.

ALK aberrations may include chromosomal rearrangements resulting in fusion genes, as seen, e.g., in ALCL and NSCLC. Other alterations include ALK copy-number gains and activating ALK mutations. Aberrations such as mutations or translocation of ALK are known to occur in various cancers, including, e.g., NSCLC, anaplastic large cell lymphomas (ALCL), inflammatory myofibroblastic tumors, diffuse large B cell lymphoma, colon cancer, renal cell carcinoma, breast carcinoma, esophageal cancer, and neuroblastoma.

Examples of ALK-TKI include, e.g., Brigatinib, Crizotinib, Ceritinib, Alectinib and Entrectinib (RXDX-101). Crizotinib (PF-02341066) is a 1st generation ALK-TKI and used e.g. for ALK-positive NSCLC and ROS1-positive NSCLC, particularly locally advanced and/or metastatic NSCLC. It has a IC₅₀ against EML4-ALK of 250-300 nm. Ceritinib is used e.g. for ALK-positive metastatic NSCLC.

Similarly, THSD may be administered to patient groups generally treated with ROS-TKI (inhibitors to Proto-oncogene tyrosine-protein kinase ROS or ROS1). Patient groups include those with cancers that are positive for genetic ROS1 aberrations such as fusions or mutations, such as, e.g., metastatic NSCLC, and patients who developed resistance to treatment with a ROS-TKI. Cancers that may be positive for ROS1 aberrations include, e.g., without limitation: glioblastoma, lung cancers incl. lung adenocarcinoma, ovarian cancer, ovarian carcinoma, sarcoma, cholangiocarcinoma, cholangiosarcoma, inflammatory myofibroblastic cancer, gastric cancer, colorectal cancer, spitzoid melanoma, angiosarcoma, and others.

Examples of ROS-1 inhibitors include, e.g., Crizotinib, Entrectinib, Lorlatinib (PF-06463922), Ceritinib, TPX-0005, DS-6051b, and Cabozantinib. Crizotinib is used e.g. for metastatic ROS1-positive NSCLC). Cabozantinib is used e.g. for metastatic medullary thyroid cancer and renal cell carcinoma.

Some TKI are suitable to address multiple mechanisms of malignancy, and can be used for multiple patient groups, e.g. those that are EGFR-positive, ALK-positive, or ROS/ROS1-positive (EGFR+, ALK+, ROS+), i.e. that have genetic aberrations affecting these genes encoding the respective tyrosine kinase enzymes. For example, Entrectinib is a TKI for all of three Trk proteins (encoded by the three NTRK genes, respectively) as well as the ROS1, and ALK receptor tyrosine kinases. Similarly, Crizotinib inhibits both ALK and ROS1.

In embodiments, the active ingredients (THSD, and optional secondary drug/active(s) such as chemotherapeutic or anti-androgenic) can be admixed or compounded with a conventional, pharmaceutically acceptable excipient. A mode of administration, vehicle, excipient or carrier should generally be substantially inert with respect to the actives, as will be understood by a person of ordinary skill in the art. Illustrative methods, vehicles, excipients, and carriers are described e.g. in Remington's Pharmaceutical Sciences, 18th ed. (1990), the disclosure of which is incorporated herein by reference. The excipient must be "acceptable" in the sense of being compatible with the other ingredients of the formulation and not deleterious to the recipient thereof.

In embodiments, the THSD (or a combination of an ELSD with an ALSD, co-administered as described above), may be co-administered with further drugs in a co-administered in a coordinated administration schedule either concurrently or subsequently. Such drugs for co-administration with a THSD include in particular drugs that tend to induce drug resistance when administered on their own, for example, chemotherapeutics such as, e.g., cisplatin and its derivatives, gemcitabine, and doxorubicin, anti-androgenic drugs, such as, e.g., Abiraterone and Enzalutamide, and taxane drugs, such as, e.g., Docetaxel and Paclitaxel. Without wishing to be bound by theory, the combination of androgen deprivation and therapy with THSD may provide an additive or synergistic therapeutic effect.

In embodiments, taxanes (also known as taxoids) for co-administration are structurally a class of diterpenes that were originally identified from plants of the genus Taxus (yews) and are drugs used for chemotherapy; they comprise a taxadiene core and typically a 6/8/6 or 6/10/6-membered core ring. Taxanes may include one or more of Docetaxel (Taxotere), Paclitaxel (Taxol), Cabazitaxel. They also may include one or more abeotaxane, i.e. a class of taxoid molecules with an unconventional core 5/7/6 type ring structure, e.g., without limitation, taxchinin A. The core carbon skeleton of a conventional taxane has a 6-membered A ring, 8-membered B ring and a 6-membered C ring, combined with conventional side chains, while abeotaxanes contain three altered ring structures, with a 5-membered A ring, 7-membered B-ring and 6-membered C-ring (combined with conventional side chains). Other a 11 (15→1) abeotaxanes besides taxchinin A include brevifoliol and TPI 287 (formerly ARC-100). Other taxanes include taxchinin B (a 11 (15→1) obeotaxoid with an oxetane ring).

In embodiments, the pharmaceutical formulations may conveniently be made available in a unit dosage form by various methods well known in the pharmaceutical arts, for example by presenting the formulation in a suitable form for delivery, e.g., forming an aqueous suspension, compounding a tablet, or encapsulating a powder into a capsule, e.g. to release the powder at a particular time, stage or location of digestion, and/or protect it from stomach acids. The dosage form may optionally comprise one or more adjuvant or accessory pharmaceutical ingredient for use in the formulation, including, without limitation, mixtures, buffers, and solubility enhancers.

In embodiments, parenteral dosage forms (i.e. that bypass the GI tract) of the pharmaceutical formulations include, but are not limited to, solutions ready for injection, dry products ready to be dissolved or suspended in a pharmaceutically acceptable vehicle for injection, suspensions ready for injection, and emulsions. In addition, controlled-release parenteral dosage forms can be prepared for administration to a patient, including, but not limited to, extended release tablets, pills or capsules, DUROS®-type and other implantable dosage forms, for systemic or tissue-specific delivery.

In embodiments, suitable vehicles that can be used to provide parenteral dosage forms include, without limitation: sterile water; water for injection USP; saline solution; glucose solution; aqueous vehicles (e.g. sodium chloride injection, Ringer's injection, dextrose injection, dextrose and sodium chloride injection, and lactated Ringer's injection); water-miscible vehicles (e.g. ethyl alcohol, polyethylene glycol, and propylene glycol); and non-aqueous vehicles (e.g. corn oil, cottonseed oil, peanut oil, sesame oil, ethyl oleate, isopropyl myristate, and benzyl benzoate). Compounds that alter or modify the solubility of a pharmaceutically acceptable salt of a compound of the invention as disclosed herein can also be incorporated into the parenteral dosage forms of the disclosure, including conventional and controlled-release parenteral dosage forms.

In embodiments, formulations for parenteral administration include aqueous and non-aqueous sterile injection solutions, which may further contain additional agents, such as anti-oxidants, buffers, bacteriostats, and solutes, which render the formulations isotonic with the blood of the intended recipient. The formulations may include aqueous and non- aqueous sterile suspensions, which contain suspending agents and thickening agents.

In embodiments, sterile injectable preparations, for example, injectable aqueous or oleaginous suspensions, can be formulated as is well known in the art, e.g. using suitable dispersing or wetting agents and suspending agents. The injectable preparation includes injectable solutions, suspensions or emulsions in a nontoxic parenterally acceptable diluent or solvent, e.g. a solution in 1,3-butanediol. Acceptable vehicles and solvents include e.g. water, Ringer's solution, U.S.P. and isotonic sodium chloride solution. In addition, sterile, fixed oils are conventionally employed as a solvent or suspending medium. For this purpose any bland fixed oil can be employed, including synthetic mono- or diglycerides. In addition, fatty acids such as oleic acid are used in the preparation of injectables.

In embodiments, compositions for rectal or vaginal administration include suppositories which can be prepared by mixing the actives with suitable non-irritating excipients or carriers which are solid at ambient temperature but liquid at body temperature and therefore melt in the rectum or vaginal cavity (e.g. cocoa butter, polyethylene glycol, or a suppository wax) to release the active.

In embodiments, dosage forms suitable for oral or sublingual administration include tablets, troches, capsules, elixirs, suspensions, syrups, wafers, chewing gum, or the like, prepared as is well known in the art. The amount of active in such dosage forms may be adjusted as will be apparent to a person of ordinary skill, e.g. depending on the frequency of administration desired, and whether an extended release formulation is prepared. A syrup formulation will generally consist of a suspension or solution of the active or its salt in a liquid carrier, for example, ethanol, glycerine or water, with a flavoring or coloring agent.

In embodiments, solid dosage forms for oral administration include, e.g., capsules, tablets, pills, powders, and granules. In such solid dosage forms, the active is mixed with at least one inert a) filler, extender or diluent (e.g. starch, lactose, sucrose, glucose, mannitol, silicic acid, and mixtures thereof), and b) binder (e.g. carboxymethylcellulose, alginate, gelatin, polyvinylpyrrolidinone, sucrose, acacia, and mixtures thereof), c) humectant (e.g. glycerol), d) disintegrating agent (e.g. agar-agar, calcium carbonate, potato starch, tapioca starch, alginic acid, certain silicates, sodium carbonate, and mixtures thereof), e) solution retarding agents (e.g. paraffin), f) absorption accelerators (e.g. quaternary ammonium compounds, and mixtures thereof), g) wetting agents (e.g. cetyl alcohol, glycerol monosterate , and mixtures thereof), and h) absorbents (e.g. kaolin clay, bentonite clay, and mixtures thereof), and i) lubricants (e.g. talc, calcium stearate, magnesium stearate, solid polyethylene glycols, sodium lauryl sulfate, and mixtures thereof), and mixtures thereof. Such dosage forms can also include additional substances, e.g., tableting lubricants and other tableting aids such as magnesium stearate and microcrystalline cellulose, or buffering agents, in particular e.g. in capsules, tablets and pills.

In embodiments, solid compositions of a similar type can also be employed as fillers in soft and hardfilled gelatin capsules using excipients such as, e.g., lactose or milk sugar as well as high molecular weight polyethylene glycols. Alternatively or additionally, the actives can be in micro-encapsulated form with one or more excipients as noted above.

The various solid dosage forms (e.g. tablets, dragees, capsules, pills, and granules) can be prepared with coatings and shells such as enteric coatings, release controlling coatings and other coatings well known in the pharmaceutical formulating art. They can optionally contain opacifying agents and can also be of a composition that they release the active only, or preferentially, in a certain part of the intestinal tract, optionally, in a delayed manner. Examples of embedding compositions that can be used e.g. for delayed or extended release include polymeric substances and waxes.

In embodiments, the actives can be present in form of salts, which may be particularly suitable for use in the treatment of cancer. The salts of the present invention may be administered to the patient in a variety of forms, depending on the route of administration, the salt involved, and the cancer being treated. For example, an aqueous composition or suspension of the salts may be administered systemically or tissue-specifically by injection, e.g. in the form of a pharmaceutical matrix by injection or surgical implantation, at a desired site. The particular technique employed for administering the matrix may depend, for example, on the shape and dimensions of the involved matrix. In some embodiments, the salt is introduced substantially homogeneously in a tumor to minimize the occurrence in the tumor of cold (untreated) areas. In certain embodiments, the salt is administered in combination with a pharmaceutically acceptable carrier. A wide variety of pharmaceutically acceptable carriers are available and can be combined with the salts, as will be apparent to one of ordinary skill in the art.

In embodiments, effective amounts, toxicity, and therapeutic efficacy of the active and/or its dosage form can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dosage can vary depending upon the dosage form employed and the route of administration utilized. The dose ratio between toxic and therapeutic effects is the therapeutic index and can be expressed as the ratio LD₅₀/ED₅₀. A therapeutically effective dose can be estimated initially from cell culture assays. Also, a dose can be formulated in animal models to achieve a circulating plasma concentration range that includes the IC₅₀ (i.e., the concentration of the compound of the invention, which achieves a half-maximal inhibition of symptoms, in case of cancer e.g. inhibition of the growth of the cancer cells) as determined in cell culture, or in an appropriate animal model, in particular mammalian animals, including e.g. mouse, rat, guinea pig, rabbit, pig, dog, and others. Levels in plasma can be measured e.g. by high performance liquid chromatography (HPLC). The effects of any particular dosage can be monitored by a suitable bioassay well known in the pharmaceutical art.

In embodiments, the dosage of a pharmaceutical formulation as described herein can be determined by a physician and adjusted, as necessary, to suit observed effects of the treatment. With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment, or make other alterations to the treatment regimen. The dosing schedule/regimen can vary, e.g. once a week, daily, or in particular predetermined intervals, depending on a number of clinical factors, including e.g. the subject's sensitivity to each of the actives.

In embodiments, a pharmaceutical composition comprising one or more active (i.e. one or more THSD, ALSD, and ELSD, optionally combined with one or more further/secondary drug) can be administered to a patient, or to the patient's tumor, cancer or pre-cancerous cells, either in vivo or in vitro, in an effective dose, in particular, to inhibit or suppress cancer cell growth, or optionally induce apoptosis, and thus treat the cancer or tumor, or to lower the risk of cancer developing or a tumor increasing its growth, e.g. in particular groups of patients at higher than average risk for cancer and/or tumor development. The one or more active may be concurrently administered, or may be administered according to a particular dosing regimen, e.g. as described herein-above. The dosing regimen typically takes into account factors such as the concentration of the active(s) in the blood and the half-life of each active, as will be apparent to a person of ordinary skill.

In embodiments, an effective dose of a pharmaceutical composition comprising the one or more active can be administered to a patient once or repeatedly. The pharmaceutical composition can also be administered over a period of time, such as over a 5 minute, 10 minute, 15 minute, 20 minute, or 25 minute period. If warranted, the administration can be repeated, for example, on a regular basis, such as hourly for 3 hours, 6 hours, 12 hours or longer or such as biweekly (i.e., every two weeks) for one month, two months, three months, four months or longer. In some instances, after an initial treatment regimen, the subsequent treatments can be administered on a less frequent basis. For example, after administration biweekly for three months, administration can be repeated once per month, for six months or a year or longer. Administration of a composition comprising one or more active in a coordinated administration schedule may be adjusted accordingly to ensure exposure to a plurality of actives, e.g. concurrent exposure to two or more actives (e.g. both the ALSD and the ELSD, or one or more THSD and a secondary drug), e.g. so that a reduction of levels of a biomarker or one or more symptom results, e.g. growth inhibition of cancer or tumor cells, growth inhibition of a tumor, reduction of tumor volume/tumor shrinkage, reduction of metastase formation, or reduction of the risk thereof.

In embodiments, two or more actives may be administered sequentially; for example, the ELSD and ALSD may be administered one after the other with a duration of time before administration of the second active, e.g. ELSD before ALSD, or vice versa. Preferably, the ELSD may be administered several days, a day, hours or minutes before administration of the ALSD (or vice versa). For example, this duration of time may be about 20-24 h before, about 15-20 hours before, about 12-15 hours before, about 10-12 hours before, about 8-10 hours before, about 2-8 hours before, about 1-6 hours before, about 1-4 hours before, about 1-3 hours before, about 1-2 hours before, about 0.5-1.5 hours before, or about 45-min. about 30 minutes, or about 15 minutes before administration of the second active. Similarly and/or additionally, one or more THSD (ELSD and/or ALSD, concurrently or sequentially) may be administered before administration of a secondary drug, or vice versa.

In embodiments, the amount and/or concentration of the one or more active may depend on the typical dosage of the particular formulation and its route of administration, and may be adapted to expose the tumor, cancer or pre-cancerous cells to concentrations of, e.g., from about 0.1 to about 100 µM. For example, for an ALSD, e.g. DZ2a, the concentration may be about 0.5 to about 50 µM, e.g. about 16 µM, and may be combined with about 0.5 to about 50 µM of an ELSD (DZ2b), e.g. about 16 µM. All amounts and concentrations will need adaptation to factors including the circumstances of the individual patient, cancer type, and treatment duration, as will be apparent to a person of ordinary skill.

In embodiments, the amount and/or concentration of the one or more active in the pharmaceutical composition can be based on weight, moles, or volume. In embodiments, the pharmaceutical composition may comprise about 0.01%-99%, 0.05%-90%, 0.1%-85%, 0.5%-80%, 1%-75%, 2%-70%, or 3%-65%, 4%-60%, or 5%-50% of the actives. The pharmaceutical composition may comprise at least 0.0001%, at least 0.1%, 0.5%, 1%, 2%, 3%, 4%, 5%, 10%, or at least 15% of each active. Alternatively or additionally, the pharmaceutical composition may comprise a maximum of up to about 0.0001%, 0.1%, 0.5%, 1%, 2%, 3%, 4%, 5%, 10%, or 15% of each active.

In embodiments, provided is a bifunctional method of cancer therapy or cell inhibition (inhibiting the growth or development of cancerous or pre-cancerous cells, shrinkage of tumors) and identification/localization, e.g. by imaging, in particular NIR imaging. Therein, the cancerous cells, pre-cancerous cells, or tumors are additionally identified, imaged and/or localized in a patient in need of such therapy. The method may comprise providing one or more THSD and administering it to a patient, and performing optical imaging for the one or more THSD. The method may comprise providing one or more ELSD and administering it to a patient who is optionally further administered with one or more ALSD concurrently or sequentially; and performing optical imaging for the ELSD or the ALSD, or both. Preferably, both an ALSD and an ELSD are administered in a coordinated administration schedule, and optical imaging is performed after administration of at least one of the ALSD and ELSD. More preferably, the first loading dose is a dose of the ELSD, and imaging may be performed after ELSD administration, or may be performed after one or more subsequent dose of the ELSD or the optional ALSD. This allows to visually follow the progress of cell growth inhibition or treatment (e.g. growth arrest, disappearance or shrinkage of tumors, cancer cells, or pre-cancerous lesions), which in turn allows to adjust dosage of the one or more THSD and optional secondary actives, and/or to determine the location of tumor(s) and/or metastase(s) within the NIR spectral region of the THSD. In various embodiments, imaging may be performed, for example, about 6 to 48 hours post administration, e.g., without limitation, by injection. Imaging may be performed by comparing NIR signals of cancer/tumor cells to a background signal determined by imaging normal tissue/cells.

In embodiments, provided is a bifunctional method of conducting in situ pharmacokinetic and pharmacodynamic analyses of the actives in a tumor, cancer or tumor cells, or normal cells or tissue. The method can comprise providing the one or more THSD; contacting it with the cancer/tumor cells or tumor, or with normal cells or normal tissue; and subsequently imaging the cells exposed to the THSD, followed by pharmacokinetic and/or pharmacodynamics analyses, e.g. determining the fluorescence (or changes thereof) over time.

### DETAILED DESCRIPTION OF THE DRAWINGS

Turning to **FIG. 1A-I**, shown are the chemical formulae of an ELSD (top) and an ALSD (bottom) conjugate that are conjugated via the dihydroxyheptanoic acid unit (DHHA) of a statin to the remainder of the statin residue. In the middle on the left, a Rapid Release Ester ("RRE") Conjugate which is conjugated via a lactone-forming DHHA unit is shown for comparison; Simvastatin with its DHHA is shown as an example statin (middle, right). As apparent, in the ELSD and the ALSD, the DHHA is fixated in its open chain form.
**FIG. 1A-II** illustrates a reaction scheme for synthesis of a HMCD dye comprising a reactive carboxyl group for linking the dye. Shown here is the synthesis of DZ1 (compound **4**) wherein R₁ and R₂ are H, and R₃ is -(CH₂)₄SO₃H and R_{4*} (which will become part of the L_{E}/L_{A} linker) is -(CH₂)₅COOH. To form HMCD (and upon further reactions THSD) having different residues for R₁, R₂ and R₃, the educts are chosen accordingly, with the respective desired residues.
**FIG. 1A-III** illustrates a reaction scheme for synthesis of a DZ1-based ALSD (here with Simvastatin as statin) using a diamine to link the resulting statin intermediate (compound **6**) to the dye's carboxyl group and form a substantially stable "no release" ALSD (compound **7,** "DZ2a").
**FIG. 1A-IV** illustrates a reaction scheme for synthesis of DZ1-based ELSD (here with Simvastatin as statin) using ethanolamine to link the resulting DZ1 intermediate (compound **9**) to a Simvastatin intermediate (compound **12**) to form an ELSD (compound **14,** "DZ2b").
**FIG. 1A-V** illustrates a reaction scheme for synthesis of a Rapid Release Ester ("RRE"), here compound **8** ("DZ2c"), a DZ1-SIM RRE, for comparative examples. The RRE is less stable and more easily degrades compared to the ELSD which is stable and not subject to degradation when exposed to serum at body temperature for at least 3 hours.
In **FIG. 1B1**, shown are the chemical formulae of drugs compared in FIG. 1B2 and C-E; these include an illustrative example of a THSD which comprises an HMCD dye and a statin (here: Simvastatin) amide-linked via the L_{E}/L_{A} linker (here an illustrative example of an L_{A2} linker in a compound of formula FIId, "DZ2a", compound 7); an unconjugated dye ("DZ1"), a statin (Simvastatin), and a chemotherapeutic cancer drug (Docetaxel, commonly used for treatment prostate cancer, also known as Taxotere™), see results in FIG. IE).
In **FIG. 1B2** it is shown that the THSD (here e.g. formula FIId, "DZ2a", compound 7) provides improved cancer cell growth inhibition compared to both the unconjugated statin drug itself ("Simvastatin"), and compared to the unconjugated dye ("DZ1"). This is illustrated by the results of 22RV1 prostate cancer cells (ATCC® CRL-2505™, a human prostate carcinoma cell line of epithelial morphology). The results show that the IC₅₀ of the ALSD for reduction of cell viability/growth inhibition of the cancer cells is between 3-4 µM, which is significantly improved compared to both the unconjugated statin (>60 µM), and to the unconjugated dye DZ1.
In **FIG. 1C****,** it is shown that the THSD (here e.g. DZ2a) provides significantly improved cancer cell growth inhibition in various different cancer cell lines (LNCaP, C4-2B, 22RV1). This is illustrated by the results of 22RV1 (ATCC® CRL-2505™, a human prostate carcinoma cell line of epithelial morphology), LNCaP clone FGC (ATCC® CRL-1740™, human, prostate; derived from metastatic site: left supraclavicular lymph node) and C4-2B (ATCC® CRL-3315™, human prostate cancer cells of epithelial morphology) cancer cells, treated with the THSD (e.g. an ALSD of formula FIId, "DZ2a", compound 7, as illustrated here). These results show that the ALSD is similarly effective in different types of cancer cells.
In **FIG. 1D****,** uptake and retention in human tumors is shown for the THSD (here an ASLD of formula formula FIId, "DZ2a", compound 7); the human tumors are grown subcutaneously in mice and monitored by NIR emission.
In **FIG. 1E** it is shown that the THSD (here e.g. "DZ2a", an ALSD of formula FIId, "DZ2a", compound 7) provides significantly improved *in vivo* tumor shrinkage compared to the unconjugated statin drug itself ("SIM"), compared to the unconjugated dye ("DZ1"), and compared to the chemotherapeutic Docetaxel ("DOC"). "Vehicle" is a negative control of PBS buffer. The results show an increase in tumor volume for DZ1, SIM and DOC that is similar to the control, i.e. no tumor suppressive activity. In contrast, the THSD (ALSD with or without DOC) inhibited tumor growth as shown by a significantly lower increase in tumor volume. Co-administration of DOC together with the THSD does not further improve tumor shrinkage of the THSD. The human tumors are grown subcutaneously in mice, and tumor volumes are determined at intervals as shown.
In **FIG. 2****,** it is shown that the THSD (here an ALSD of formula formula FIId, "DZ2a", compound 7) co-localizes with mitochondriae and lysosomes and can thus interfere with various mitochondrial and lysosomal functions in cancer cells. The images of the fluorescent staining show the cell nuclei of human prostate cancer cells (ARCaPM, Androgen Repressed Metastatic Human Prostate Cancer Cell Line, M 'Mesenchymal' Clone) stained blue by DAPI, while the red fluorescence of the THSD co-localizes with that of green fluorescence of the MitoTracker™ and LysoTracker™ fluorescent dyes in the cytosolic fraction of the cancer cells, as shown by the yellow fluorescence in the composite.
In **FIG. 3A** it is shown that the THSD can greatly lower the mitochondrial oxygen consumption rate ("OCR") of cancer cells compared to the unconjugated statin drug, the unconjugated dye, or controls. The figure shows the OCR of C2-4B MDVR drug resistant cancer cells exposed to the following drugs: negative/blank control ("NT"), the unconjugated dye (here "DZ1"), the unconjugated statin drug (here Simvastatin or "SIM"), the THSD (here an ALSD of formula formula FIId, "DZ2a", compound 7), and Cholesterol ("Chole"). As illustrated, the rate drops down from about 800-900 pmol/min to less than 200 pmol/min for the THSD, while few of the other drugs lower it even below 700 pmol/min, and none lowers it below 600 pmol/min. Compared to all other drugs, the THSD thus significantly lowers the OCR starting its effect within about 250 minutes and continuing to lower further until about 700-800 minutes and longer (and eventually reaching a sustained lowered OCR); this significant lowering of the mitochondrial OCR is part of the effect on mitochondrial function that THSD exhibit, partly due to their co-localization with these cell organelles.
In **FIG. 3B****,** it is shown that a THSD exhibits less decrease of the extracellular acidification rate ("ECAR") compared to the corresponding unconjugated statin and corresponding unconjugated dye and to controls. The ECAR is compared for a negative/blank control ("NT"), a negative/vehicle control ("DMSO"), the unconjugated dye ("DZ1"), the unconjugated statin Simvastatin (SIM), the THSD (here an ALSD of formula FIId, "DZ2a", compound 7), and Cholesterol ("Chole"). The ECAR of "DZ2a" decreased less compared to all other drugs and the controls, and shows a bump of increased ECAR starting at about 500 minutes which signifies a switch to the use of glucose. In contrast all other samples show a gradual and stronger decrease, with that of the control being the graph with the lowest ECAR, Simvastatin having the highest ECAR of the gradual curves, and the remainder (DMSO, Chole) hidden behind these curves and showing a similar trend with gradual decrease of similar extent.
In **FIG. 3C** it is shown that the proteins present in cytosol or mitochondriae of C2-4B MDVR drug resistant cancer cells that are bound by the THSD, and the unconjugated dye, respectively, differ in strength of binding and/or identity. This is apparent by additional/missing bands in the denaturing gel when comparing the dye ("DZ1") with the THSD ("DZ2a"). The THSD thus binds to multiple mitochondrial and cytosolic proteins in the cytosolic and mitochondrial lysates tested, and binds more strongly and to additional proteins as compared to the corresponding unconjugated dye. This result provides a biological basis for the THSD's interference with various mitochondrial and lysosomal functions of cells, and particular of cancer cells.
**FIG. 3D** illustrates that a THSD prevents the removal of polyubiquinated proteins in 22Rv1 cancer cells through altered autophagic processes which involve defective lysosomal functions. In the lane of the THSD (here an ALSD of formula FIId, "DZ2a", compound 7), an intense dark "smear" is apparent when compared to marker, control, the corresponding unconjugated statin (Simvastatin), and to the corresponding unconjugated dye ("DZ1"), all of which have much weaker signals and less of a smear. The signal that is seen corresponds to antibody-detection of ubiquitin, which is a protein attached by cells to incorrectly folded proteins to designated them for removal from the lysosomes. When the removal mechanism is compromised, as here by DZ2a, polyubiquinated proteins are not removed but instead accumulate.
In **FIG. 4A** it is shown that a THSD achieves complete growth suppression and at moderate concentration of about 16 µM and within only 8 hours can reduce the growth of cancer cells to 0% cell survival rate. The figure illustrates the results of an 8h assay with C4-2B parental cells, which are non-resistant cancer cells not previously exposed to a cancer drug. The cells are exposed to a THSD (here an ALSD of formula FIId, "DZ2a", compound 7) in amounts of 2, 4, 8 or 16 µM. The cell survival rate decreases to 0% after 8h exposure to 16 µM of the THSD, less than about 50% survive after 3h exposure, and less than about 25% of cells survive after 4h exposure. If less THSD is used, more cells survive during the period tested, e.g. 8 µM of the THSD reduced the survival rate to below about 25-50% within about 4-8 hours, while lower amounts take longer to reduce the cell survival rate. This shows that in contrast to other drugs, the THSD is able to provide much more rapid growth inhibition, with 100% inhibition (0% cell survival) within less than 24 hours, e.g. as shown here, achieved within about 8 hours at concentrations of about 16 µM.
In **FIG. 4B** it is shown that a THSD achieves complete growth suppression for cancer cells resistant to Abiraterone acetate ("AbiR"), and at moderate concentration of about 16 µM, and within only 8 hours, can reduce the growth of the drug resistant cancer cells to a 0% cell survival rate. The figure illustrates the results of an 8h assay with "C4-2B AbiR" cells which are resistant cells previously exposed to Abiraterone acetate. The cells are exposed to a THSD (here an ALSD of formula FIId, "DZ2a", compound 7) in amounts of 2, 4, 8 or 16 µM. The cell survival rate decreases to 0% after 8h exposure to 16 µM of the THSD, less than about 50% survive after 3h exposure, and less than about 25% of cells survive after 4h exposure. If less THSD is used, more cells survive during the period tested, e.g. 8 µM of the THSD reduced the survival rate to below about 25-50% within about 4-8 hours, while lower amounts take longer to reduce the cell survival rate. This shows that in contrast to other drugs, the THSD is able to provide much more rapid growth inhibition, with 100% inhibition (0% cell survival) within less than 24h, e.g. as shown here, achieved within about 8h at concentrations of about 16 µM.
In **FIG. 4C** it is shown that a THSD achieves complete growth suppression for cancer cells resistant to Enzalutamide ("MDVR"), and at moderate concentration of about 16 µM, and within only 8 hours, can reduce the growth of the drug resistant cancer cells to a 0% cell survival rate. The figure illustrates the results of an 8h assay with Enzalutamide resistant ("C4-2B MDVR") cells which are resistant cells previously exposed to Enzalutamide. The cells are exposed to a THSD (here an ALSD of formula FIId, "DZ2a", compound 7) in amounts of 2, 4, 8 or 16 µM. The cell survival rate decreases to 0% after 8h exposure to 16 µM of the THSD, less than about 50% survive after 3h exposure, and less than about 25% of cells survive after 4h exposure. If less THSD is used, more cells survive during the period tested, e.g. 8 µM of the THSD reduced the survival rate to below about 25-50% within about 4-8 hours, while lower amounts take longer to reduce the cell survival rate. This shows that in contrast to other drugs, THSD is able to provide much more rapid growth inhibition, with 100% inhibition (0% cell survival) within less than 24 hours, e.g. as shown here, achieved within about 8 hours at concentrations of about 16 µM.

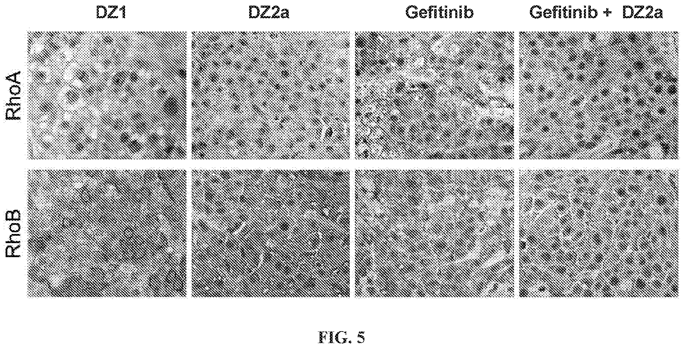

**FIG. 5** shows RhoA/B staining of human tumors in a H-1975 human lung cancer cell mouse model. Staining persists in the membranes of DZ1- and Gefitinib-treated tumors, but not in ALSD- and ALSD/Gefitinib-treated tumors. This demonstrates that a THSD can prevent the anchoring of RhoA/B onto cell membranes, likely due to inhibition of protein prenylation and its downstream cell signaling by the THSD.
In **FIG. 6B1** it is shown that THSD have a half-life of about 0.5-2 hours in mammals (here rats). After intravenous administration of 1 mg/kg of a THSD (here an ALSD, DZ1-SIM amide of formula FIId, "DZ2a"), blood samples are taken from the animals in intervals and measured. Graphs of the results show the mean concentration for female and male rats, respectively.
In **FIG. 6B2** it is shown that THSD have a half-life of about 0.5-2 hours in mammals (here dogs). After intravenous administration of 1 mg/kg of a THSD (here an ALSD, DZ1-SIM amide of formula FIId, "DZ2a"), blood samples are taken from the animals in intervals and measured. Graphs of the results show the mean concentration for female and male dogs, respectively.
In **FIG. 7A** it is shown that an ALSD and an ELSD (amide-linked and ester-linked tumor homing statins, respectively) provide improved cell growth inhibition of cancer cells, and an improved/lowered IC₅₀, compared to the unconjugated statin drug itself. The figure illustrates the IC₅₀ (µM) graphs of the THSDs (here an ALSD of formula FIId, compound **7** or "DZ2a", designated "DZ-amide" in this figure, and an ELSD of formula FId, compound **14** or "DZ2b", designated "DZ-ester" in this figure and FIG. 7B-C) compared to Simvastatin in human 22RV1 cancer cells. The IC₅₀ of the THSDs are similar, with that of the ALSD at 7.8 µM, and of the ELSD at 10.8 µM; the IC₅₀ of the unconjugated statin is much higher at 38.7 µM (i.e. more unconjugated statin is needed for the same effect on cell viability). Thus both ALSD and ELSD reduce cell viability and inhibit the growth of human prostate cancer cells better than the unconjugated statin.
In **FIG. 7B** it is shown that an ALSD and an ELSD (compounds as in FIG. 7A above) provide improved cell growth inhibition of cancer cells, and an improved/lowered IC₅₀, compared to Simvastatin in MDVR (Enzalutamide-resistant C4-2B) cancer cells; also compare description of FIG. 7A. The IC₅₀ of the THSD are 5.6 µM and 3.8 µM, respectively, while the IC₅₀ of the unconjugated statin is much higher at 25.5 µM. Thus both ALSD and ELSD show improved growth inhibition of the human prostate cancer cells.
In **FIG. 7C** it is shown that an ALSD and an ELSD (compounds as in FIG. 7A above) provide improved cell growth inhibition of cancer cells, and an improved/lowered IC₅₀, compared to Simvastatin in PC3 cancer cells; also compare description of FIG. 7A. The IC₅₀ of the THSD are 3.5 µM and 6.0 µM, respectively, while the IC₅₀ of the unconjugated statin is much higher at 14.8 µM. Thus both ALSD and ELSD show improved growth inhibition of the human prostate cancer cells.
In **FIG. 7D** it is shown that an ALSD and an ELSD provide improved cell growth inhibition of cancer cells, and an improved/lowered IC₅₀, compared to the unconjugated statin drug itself and compared to the unconjugated dye. The figure illustrates the IC₅₀ (µM) graphs of the THSD (here an ALSD of formula FIId, compound **7,** or "DZ2a", designated "DZ-SIM-amide" in this figure, and an ELSD of formula FId or compound **14** ("DZ2b"), designated "DZ-SIM-ester" in this figure and FIG. 7E-I) compared to Simvastatin and the unconjugated DZ1 dye in A549 lung cancer cells. The IC₅₀ of the THSD are 5.7 µM and 5.4 µM, respectively, while the IC₅₀ of the unconjugated statin and the unconjugated dye are much higher at >50 µM. Thus both ALSD and ELSD show improved growth inhibition of the lung cancer cells. As shown, the dose-response curve of the ELSD is less steep.
In **FIG. 7E** it is shown that an ALSD and an ELSD provide improved cell growth inhibition of cancer cells, and an improved/lowered IC₅₀ in A549DDP lung cancer cells; also compare description of FIG. 7D. The IC₅₀ of the THSD are 6.1 µM and 2.1 µM, respectively, while the IC₅₀ of the unconjugated statin and the unconjugated dye are much higher at 30 µM and >50 µM, respectively. Thus both ALSD and ELSD show improved growth inhibition of the lung cancer cells. As shown, the dose-response curve of the ELSD is less steep.
In **FIG. 7F** it is shown that an ALSD and an ELSD provide improved cell growth inhibition of cancer cells, and an improved/lowered IC₅₀ in H1975 lung cancer cells; also compare description of FIG. 7D. The IC₅₀ of the THSD are 2.9 µM and 6.4 µM, respectively, while the IC₅₀ of the unconjugated statin and the unconjugated dye are much higher at >50 µM. Thus both ALSD and ELSD show improved growth inhibition of the lung cancer cells.
In **FIG. 7G** it is shown that an ALSD and an ELSD provide improved cell growth inhibition of cancer cells, and an improved/lowered IC₅₀ in H1650 lung cancer cells; also compare description of FIG. 7D. The IC₅₀ of the THSD are 3.6 µM and 11.8 µM, respectively, while the IC₅₀ of the unconjugated statin and the unconjugated dye are much higher at 33.3 µM and >50 µM, respectively. Thus both ALSD and ELSD show improved growth inhibition of the lung cancer cells.
In **FIG. 7H** it is shown that an ALSD and an ELSD provide improved cell growth inhibition of cancer cells, and an improved/lowered IC₅₀ in PC9 lung cancer cells; also compare description of FIG. 7D. The IC₅₀ of the THSD are 9.6 µM and 5.6 µM, respectively, while the IC₅₀ of the unconjugated statin and the unconjugated dye are much higher at 33 µM and >50 µM, respectively. Thus both ALSD and ELSD show improved growth inhibition of the lung cancer cells. As shown, the dose-response curve of the ELSD is less steep.
In **FIG. 7I** it is shown that an ALSD and an ELSD provide improved cell growth inhibition of cancer cells, and an improved/lowered IC₅₀ in H446 lung cancer cells; also compare description of FIG. 7D. The IC₅₀ of the THSD are 3.7 µM and 3.4 µM, respectively, while the IC₅₀ of the unconjugated statin and the unconjugated dye are much higher at 39 µM and >50 µM, respectively. Thus both ALSD and ELSD show improved growth inhibition of the lung cancer cells.
In **FIG. 8** it is shown that an ALSD is a stable non-hydrolyzing drug and provides its growth inhibitory action as such, rather than through release of an active from a pro-drug (i.e. unlike the partially/slowly hydrolyzing ELSD and the rapidly hydrolyzing RRE). In case of hydrolysis and release of an active over time, IC₅₀ curves for drug exposures of different lengths of time (24, 72 and 120h) differ significantly. However, as shown, the dose-response curves of the ALSD (here an ALSD of formula FIId, "DZ2a", compound **7**) are nearly identical irrespective of length of drug exposure.
In **FIG. 9A1****,** a comparative in vivo test in a mouse xenograft model for human drug resistant cancer is shown. The test compares THSD with the unconjugated statin, the unconjugated dye, and the EGFR-TKI drug. The human cancer cells are PC9AR lung cancer cells that are resistant to 3rd generation epidermal growth factor receptor tyrosine kinase inhibitor drugs (EGFR-TKI), in particular AZD9291 resistant. The image shows a photograph of the human cancer cell derived tumors after removal from the host mice after 16 days of treatment. For each row the agent that the tumor was treated with is indicated on the left (from top to bottom): control/no drug (CON), unconjugated dye (DZ1), unconjugated dye Simvastatin (SIM), THSD conjugate (here an ALSD of formula FIId, compound 7 or "DZ2a", designated "DZ-SIM" in this figure and FIG. 9A2, A3 and A4), EGFR-TKI (AZD9291), and both DZ-SIM and AZD9291 (COMBI in the figure). A dramatic growth suppression of the tumor is apparent for the DZ-SIM row and the COMBI row, but not the AZD9291 row. A few tumors, see last two rows on the right, appear to also respond, to a much lesser degree, to the unconjugated dye and to AZD9291.
In **FIG. 9A2**, a bar chart corresponding to the same experiment as described for FIG. 9A1 is shown. Each bars shows the mean tumor weight (g) for the agents as identified for FIG. 9A1, with standard deviation and confidence intervals indicating the significance of the results. The results show a dramatic growth inhibition (almost no increase in tumor weight) in the bars corresponding to the THSD treatment ("DZ-SIM", compound as in FIG. 9A1 above), and the combination of the THSD with the AZD9291 TKI) compared to the remaining agents (control, unconjugated agents, and the TKI alone), which show a strong increase in tumor weight. The standard deviation and confidence level are as indicated (mean ± S.D. (n = 6); *P<0.05, **P<0.01, ***P<0.0005, **** P<0.0001).
In **FIG. 9A3**, a graph corresponding to the same experiment as described for FIG. 9A1 is shown. The graph shows the mean tumor volume (mm³), calculated from caliper measurements over a period of 16 days of treatment for the agents as identified for FIG. 9A1, with standard deviation and confidence intervals indicating the significance of the results (**** P<0.0001 for day 16). The results show a dramatic growth inhibition (almost no increase in tumor volume) in the graphs corresponding to THSD treatment ("DZ-SIM", compound as in FIG. 9A1 above) and the combination of the THSD with the AZD9291 TKI) compared to the remaining agents (control, unconjugated agents, and the TKI alone), whose graphs in contrast show a steep increase in tumor volume.
In **FIG. 9A4**, a graph corresponding to the same experiment as described for FIG. 9A1 is shown. The graph shows the mean total body weight of the six groups of mice during treatment, the standard deviation from the mean is indicated. Each graph corresponds to one of the agents as identified for FIG. 9A1, as indicated. The results show comparatively small changes in weight; the absence of significant weight loss shows a lack of acute toxicity.
In **FIG. 10** a comparative *in vivo* test in a mouse xenograft model using H446 human small cell lung cancer (SCLC) cells is shown. The test compares THSD with the unconjugated statin, the unconjugated dye DZ1, and a non-statin dye-conjugated drug (dihydroartemisinin conjugated to DZ1, structure shown in FIG. 11); in the figure, DZ1-conjugates are designated "DZ-". The graph shows the mean tumor volume (mm³), calculated from caliper measurements over a period of 52 days of treatment for the agents, with standard deviation and confidence intervals indicating the significance comparing the results for day 52 (*P<0.05 or ***P<0.0005, respectively). The results show a dramatic growth inhibition (almost no increase in tumor volume) in the graphs corresponding to THSD treatment (here an ALSD of formula FIId, compound **7,** or "DZ2a", designated "DZ-SIM" in this figure) compared to the unconjugated statin ("SIM") and unconjugated DZ1, whose graphs in contrast show a steep increase in tumor volume. In the human lung cancer model, both THSD provide an earlier and stronger growth inhibition when compared to the non-statin conjugate (DZ1-DHA). The THSD ester outperforms the amide, providing an earlier onset of growth inhibition that is also stronger.
In **FIG. 11** the chemical structure of DZ1-DHA, a non-statin conjugate, is shown (compare comparative results in FIG. 10).

### EXEMPLARY EMBODIMENTS

Methods of synthesis for illustrative embodiments of the ALSD and ELSD are described in **examples 1A-II-IV** below, including synthesis and of an HMCD dye and its conjugation with a statin, e.g. of DZ1 conjugated to Simvastatin forming a L_{E}/L_{A} linker in the resulting THSD (DZ1-SIM ALSD, DZ1-SIM ELSD). For the comparison example with the ELSD, in example 1A-V, the synthesis of a Rapid Release Ester, DZ1-SIM RRE (without L_{E}/L_{A} linker, and attached to the statin allowing lactone-formation) is described. Alternative to DZ1, another HMCD corresponding to the dye moiety of formula FIa or FIIa may be chosen, as will be apparent to a person of ordinary skill. Similarly, Simvastatin may be replace by another statin, and alternative reagents may be chosen to result in alternative stable L_{E}/L_{A} linkers, as will be apparent to a person of ordinary skill. Reaction schemes corresponding to the described methods of synthesis are illustrated in **FIG. 1A-II, A-III** **and** **A-IV**.

**Example 1A-I/General methodology synthesis:** Simvastatin is purchased from Ark Pharm, Inc. (Arlington Heights, IL). All other chemicals and reagents are purchased from standard sources such as Sigma-Aldrich and are of highest quality available. Deionized water (18.2 Ω) used for making solutions is obtained from Milli-Q Direct Ultrapure Water System from Millipore (Billerica, MA, USA). All intermediates are characterized by 1H NMR and mass analysis and the purity of compounds are analyzed by HPLC. 1H NMR data is collected on Bruker 400MHz spectrometers using standard parameters; chemical shifts are reported in ppm (δ) in reference to residual non-deuterated solvent. ESI mass spectroscopy analysis is performed on new compounds at Mass Spectrometry and Biomarker Discovery Core facility using a Thermo Fisher LTQ Orbitrap Elite system. In the following, the following abbreviations or formulae are used: acetic acid ("HOAc" or "CH₃COOH"), ethanol ("EtOH"), methanol ("MeOH"), equivalent ("eq."), room temperature ("RT"), sodium acetate ("NaOAc" or "CH₃COONa"), sodium bicarbonate ("NaHCO₃"), hydroxybenzotriazole ("HOBt"), 3-1-ethyl-3-(3-dimethylaminopropyl)carbodiimide ("EDC"), 4-Dimethylaminopyridine ("DMAP"), Dichloromethane ("CH₂Cl₂"), and p-Toluenesulfonic acid monohydrate ("tosylic acid monohydrate" or "TsOH").

**Example 1A-II:** Synthesis of HMC Dye (illustrated for DZ1). An exemplary reaction scheme is shown in **FIG. 1A-II****.** A HMCD (here: DZ1) comprising a reactive carboxyl group for linking the dye is shown. For DZ1, residues R₁ and R₂ are H, R₃ is-(CH₂)₄SO₃H, and R_{4*} (which will become part of the L_{E}/L_{A} linker) is -(CH₂)₅COOH. To produce THSD having different residues for R₁, R₂ and R₃, a different HMCD educt can be chosen, and modified educts can be chosen accordingly as per the reaction scheme, comprising the corresponding desired residues.

Synthesis of compound **1a** (compound **1** wherein R₃ = -(CH₂)₄SO₃H): A mixture of 2, 3, 3-trimethylindolenine (5 g, 31.4 mmol) and 1,4-butane sultone (5.1 g, 37.7 mmol) is heated while stirring at 120°C under argon for 5h. The resulting reaction mixture is cooled to RT and the solid is dissolved in 50 ml of methanol. Ethyl ether (200 ml) is added to the methanol solution, and the precipitate is collected and washed with ethyl acetate (15ml, 3 times) and dried under vacuum to afford the desired product (compound **1a,** 6.8 g, yield 73%) as a white solid.

Synthesis of compound **1b** (compound **1** wherein R = -(CH₂)₅COOH): To 6-bromohexanoic acid (2.5 g, 13.0 mmol), added is 2, 3, 3-trimethylindolenine (2.5 g, 15.7 mmol). The reaction mixture is heated while stirring at 110 °C under argon for 8 h. The resulting dark red solid is dissolved in 50 ml of methanol. Ethyl ether (150 ml) is added. The precipitate is filtered and washed with ether (15 ml, 3 times) followed by acetone (15 ml, 3 times). The product is obtained as a white solid (compound **1b,** 2.7 g, 58%).

Synthesis of compound **3:** To the mixture of **1a** (2 g, 6.78 mmol) and compound **2** (3 g, 8.36 mmol) in EtOH (100 ml), added is CH₃COONa (0.56 g, 6.78 mmol). The resulting mixture is heated to reflux for 3 h. The reaction mixture is poured into 200 ml of ice-water. The formed precipitate is collected and crystallized from ethanol-acetone, to afford the desired product as a dark blue solid (compound 3, 2.1 g, yield 58%). Mass spectrum (ESI) 525.19 [M+H]⁺.

Synthesis of compound **4** ("DZ1"): To a mixture of **1b** (0.67 g, 1.9 mmol) and compound **3** (1.0 g, 1.9 mmol) in EtOH (20 ml), added is CH₃COONa (156 mg, 1.9 mmol). The resulting solution is heated to reflux for 3 h. The heated mixture is poured into 100 ml of ice water. The solid is filtered and crystallized from methanol-water, to afford the desired product as a dark green solid (compound **4, 0.99** g, yield 74%). Mass spectrum (ESI) 705.31 [M+H]⁺.

**Example 1A-III:** Synthesis of stable ALSD (here: DZ1-SIM of formula FIId or "DZ2a", compound **7**): An illustrative reaction scheme is shown in **FIG. 1A-III****.** An ALSD (here using DZ1/compound **4** for the HMCD dye, and Simvastatin/compound **5** for the statin) can be formed reacting a diamine (e.g. propane-1,3-diamine) with the statin to link the resulting statin intermediate (e.g. compound **6**) to the dye's carboxyl group, and form a substantially stable "no release" ALSD (compound **7**). To form alternative ALSD of formula FIIa, i.e. having different residues for R₁, R₂, R₃ and R_{4*} (which will become the L_{E}/L_{A} linker) as desired, the educts are chosen accordingly to form the respective residues as desired, as will be apparent to a person of ordinary skill.

Synthesis of statin intermediate (compound **6**): To a solution of Simvastatin (compound **5,** 1 g, 2.39 mmol, 1 eq.) in acetonitrile, added is propane-1,3-diamine (1 ml, 11.95 mmol, 5 eq.). The mixture is heated to reflux stirring continuously for 4 h. The solvent is removed under reduced pressure and the product is dried under high vacuum overnight. The resulting product (compound **6**) is used without further purification.

Synthesis of DZ1-SIM ALSD ("DZ2a", compound 7): An HMCD having a carboxyl group, e.g. DZ1 (compound **4,** 500 mg, 0.71 mmol), 1-ethyl-3-(3-dimethyllaminopropyl) carbodiie hydrochloride (204 mg, 1.07 mmol), and 1-hydroxy-7-azabenzotriazole (115 mg, 0.85 mmol) are dissolved in 10.0 ml CH₂Cl₂ solution to form a mixture. The mixture is stirred, e.g. for about 15 min, then compound **6** (350 mg, 0.71 mmol) is added and stirred, e.g. for an additional 2 hours at RT. The solvent is removed under reduced pressure and the product is purified, e.g. by C18-RP reverse phase silica chromatography elution with methanol-water, to afford desired product **7** as a dark green solid (compound 7, 327 mg, 39%). Mass spectrum (ESI) 1179.65 [M+H]⁺.

**Example 1A-IV:** Synthesis of DZ1-based ELSD linked to statin (Simvastatin) by reaction of the carboxyl group of dye (here: DZ1) with ethanolamine. An illustrative reaction scheme is shown in **FIG. 1A-IV****.** An ELSD (here using DZ1/compound **4** for the dye, and Simvastatin/compound **5** for the statin) can be formed reacting an amine (e.g. ethanolamine) with the dye's carboxyl group to form a dye intermediate (e.g. compound **9**). The dye intermediate can then be reacted to the dihydroxyheptanoic acid (DHHA, -CO-CH₂-CHOH-CH₂-CHOH-CH₂-CH₂) unit of a statin (in case of Simvastatin/compound **5** after conversion of its closed ring lactone form of the DHHA into its open chain carboxylic acid form with two hydroxyl groups protected, i.e. compound **12**), to form the substantially stable "slow release" ELSD (compound **14,** "DZ2b"). Alternative statins may be reacted with the dye intermediate accordingly, e.g. statins having an open chain carboxylic acid DHHA (as shown in compound **12,** compare in **FIG. 1A-IV**) may be directly reacted with the dye intermediate. To form alternative ELSD of formula FIa, i.e. having different residues for R₁, R₂, R₃ and R_{4*} (which will become part of the L_{E}/L_{A} linker) as desired, different educts are chosen accordingly to form the respective residues as desired, as will be apparent to a person of ordinary skill.

Synthesis of DZ1-hydroxyethylamide (compound **9**): The dye (here: DZ1, compound **4,** 600 mg, 0.85 mmol), 1-ethyl-3-(3-dimethyllaminopropyl) carbodiie hydrochloride (245 mg, 1.28 mmol) and 1-hydroxy-7-azabenzotriazole (138 mg, 1.02 mmol), are dissolved in 10.0 ml CH₂Cl₂ solution. The resulting mixture is stirred for 15 min, then ethanolamine (57 mg, 0.94 mmol) is added and stirred for an additional 2 hours at RT. The solvent is removed under reduced pressure and the product is purified by C18-RP reverse phase silica chromatography elution with methanol-water to afford desired product (compound **9**) as a dark green solid 331 mg (52%). Mass spectrum (ESI) 749.34 [M+H]⁺.

Synthesis of a statin methyl ester (here: a methyl derivative of Simvastatin, compound **10**): Sulfuric acid 200µl is added to methanol solution (20 ml) of Simvastatin (compound **5,** 418 mg, 1 mmol), at 0°C. The solution is stirred for 5h at RT, and 10 ml of 10% NaHCO₃ is added to quench the reaction. The resulting mixture is extracted with ethyl acetate. The organic layer is washed with saturated brine, dried (Na₂SO₄), and the solvent is removed under reduced pressure. The crude product is purified using silica gel chromatography (ethyl acetate: hexane=2:1) to give the product, compound **10** (387 mg, 86% yield).

Synthesis of compound **11:** 2,2-Dimethoxypropane (1.3 ml, 1108 mg, 10.639 mmol) is added to a solution of compound **10** (300 mg, 0.666 mmol), *p*-toluenesulfonic acid monohydrate (13 mg, 0.067 mmol) in dichloromethane (10 ml). This solution is stirred for 2 h at reflux, and then 10% NaHCO₃ (5 ml) is added to quench the reaction. The resulting mixture is extracted with dichloromethane. The organic layer is washed with saturated brine, dried (Na₂SO₄), and the solvent is removed under reduced pressure. The crude product is purified using silica gel chromatography (ethyl acetate:hexane= 1:2) to provide the product, compound **11** (301 mg, 92% yield).

Synthesis of statin derivate with carboxyl group, compound **12:** NaOH (1M, 1ml) is added to a tetrahydrofuran solution (4 ml) of compound **11** (300 mg, 0.600 mmol). The resulting mixture is stirred for 18h at RT. Then 1M HCl (1.2 ml) is added, followed by extraction with diethyl ether. The organic layer is washed with saturated brine, dried (Na₂SO₄), and evaporated to give the product, compound **12** (274 mg, 94% yield). Mass spectrum (ESI) 499.30 [M+Na]⁺

Synthesis of an ELSD (here: DZ1-SIM ELSD, compound **14,** "DZ2b"): The dye-intermediate with hydroxyl group (compound **9,** 25 mg, 0.033 mmol) and the statin derivate with carboxyl group (SIM derivate compound **12,** 16 mg, 0.034 mmol) are dissolved in 3ml of anhydrous CH₂Cl₂. To the resulting solution, EDC (9.5 mg, 0.049 mmol) is added, followed by DMAP (2 mg, 0.017 mmol), and the resulting mixture is stirred at RT for 18h. Then this reaction mixture is crashed out in ether and the leftover residue is treated with 80% aqueous HOAc (3 ml) for 2h. The crude product is subjected to purification by semi-preparative HPLC, to give the product, compound **14** (14 mg, 36%). Mass spectrum (ESI) m/z 1166.62 [M+H]⁺.

**Comparative example 1A-V:** Synthesis of DZ1-SIM rapid release ester (RRE) and comparison of stability to an ELSD (here: DZ1-SIM). A rapid release ester (RRE) is synthesized as described below, linking DZ1 to Simvastatin (DZ1-SIM RRE or "DZ2c", compound **8**) and is tested in comparison to the ELSD (here: DZ1-SIM or "DZ2b", compound **14,** synthesized as described above). Both compounds are incubated in mouse blood serum at 37°C for 3 hours, after which samples are taken and tested for degradation of the conjugates by testing for the unconjugated statin as described in example 6 hereinbelow (LC-MS/MS-025 with positive ion, ESI, MRM detection and HPLC). The ELSD exhibits substantially higher stability (i.e. less degradation/release of the statin from the conjugate) compared to **8** (RRE); specifically the amount of the ELSD is measured at time 0 and time 3h. The value at time 0 for each of the samples is 100. At 3h the value for the ELSD is 100 (i.e. no degradation/release), while the value for the RRE at 3h is 10 (i.e. degradation of 90%).

Synthesis of DZ1-SIM RRE (compound **8,** DZ2c): DZ1 (compound **4,** 500 mg, 0.71 mmol) and Simvastatin (compound **5,** 356 mg, 0.85 mmol) are dissolved in 10 ml of anhydrous CH₂Cl₂. To the resulting mixture, added is EDC (204 mg, 1.06 mmol), followed by DMAP (40 mg, 0.33 mmol), and the resulting EDC/DMAP mixture is stirred at RT for 18 h. The reaction mixture is crashed out in ether and the leftover residue is dissolved in 2 ml of methanol. The crude product is subjected to purification by C18-RP reverse phase silica chromatography elution with methanol-water, to afford the desired DZ1-SIM RRE product (compound **8,** 329mg, 42%). Mass spectrum (ESI) m/z 1105.57 [M+H]⁺.

The formulae of the drugs tested in **examples 1B-E** described herein below are shown in **FIG. 1A-IV**. In summary, the experiments described in the examples below demonstrate an improved growth inhibition of various cancer cells *in vitro* and *in vivo,* in particular tumor shrinkage/volume reduction of human tumors grown in mice, by an ALSD (here of formula formula FIId, "DZ2a", compound **7**), when compared to a statin (here Simvastatin or "SIM") or to an unconjugated dye (here "DZ1").

### Methodology examples 1B-E:

**Cell culture:** In the following examples, unless otherwise specified, all cell lines are purchased from American Type Culture Collection and cultured in American Type Culture Collection (ATCC)-recommended media, with fetal bovine serum (FBS) to a final concentration of 10 % and 1× penicillin/streptomycin at 37°C with 5% CO₂ in a cell culture incubator, unless otherwise specified. Unless otherwise specified, culture is 2D. Where culture is 3D, low attachment plates are used, with the same media. **C4-2B** (parental cell line and drug-resistant cells derived therefrom) are cultured in RPMI-1640 with 10% FBS. **MDVR cells** (an Enzalutamide-resistant variety of C4-2B prostate cancer cells formed as described in **Example 4** below) are cultured as indicated for the parental C4-2B cells. **22RV1 Prostate Cancer (PC) cells (ATCC® CRL-2505™,** a human prostate carcinoma cell line of epithelial morphology) are cultured in RPMI-1640 with 10% FBS. **A549** cells (ATCC® CCL-185™, a human lung carcinoma cell line of epithelial morphology) are cultured in F-12K medium with 10% FBS. **A549**/DDP cells (Cells were described in DOI: 10.3892/mmr.2014.2163 and in doi: 10.7150/jca.19426; cisplatin resistant cells may be established from exposing parental A549 cells to cisplatin for an extended time). MDR A549/cisplatin lung adenocarcinoma cell line, drug resistant) are cultured in F-12K medium with 10% FBS. **H1975** cells (ATCC® CRL-5908™, a human adenocarcinoma non-small cell lung cancer cell line of epithelial morphology) are cultured in RPMI-1640 medium with 10% FBS; H1975 cells are a cell model for human lung cancer that is resistant to Gefitinib (an EGFR inhibitor drug used for treatment of certain breast, lung and other cancers). **H1650** cells (ATCC® CRL-5883™, a adenocarcinoma/bronchoalveolar carcinoma lung cancer cell line of epithelial morphology) are cultured in RPMI-1640 medium with 10% FBS. **PC9** cells (ECACC® 90071810, formerly known as PC-14, a pulmonary adenocarcinoma/Non-Small Cell Lung Carcinoma (NSCLC) cell line with EGFR mutation of mixed morphology that includes round and spindle shaped cells) are cultured in RPMI 1640 medium with 2mM Glutamine and with 10% FBS. **PC9AR** cells (an Osimertinib (AZD9291)-resistant cell line) were published (DOI: 10.1158/1078-0432.CCR-17-1574) and may be obtained by the authors, or formed from the parental cell line by exposure of PC9 cells to Osimertinib (also known as AZD9291), in gradually increased concentrations from 10 nmol/L to 500 nmol/l over a period of about 6 months; the resulting resistant cells are cultured as described for the parental PC9 cells. **H446** cells (ATCC® HTB-171™, a human small cell carcinoma SCC lung cancer cell line derived from a metastatic site) are cultured as described for 22RV1 cells. **PC3** cells (ATCC® CRL-1435™, a human prostate cancer cell line of epithelial morphology initiated from a bone metastasis of a grade IV prostatic adenocarcinoma) are cultured in F-12K with 10% FBS. **LNCaP** cells (LNCaP clone FGC, ATCC® CRL-1740™, a prostate carcinoma cell line of epithelial morphology) are cultured in RPMI-1640 with 10% FBS.

**Cell and tissue uptake:** In the following examples, unless otherwise specified, cell and tissue uptake of THSD or HMCD controls is determined as follows. Cells (1 × 10⁴ per well) are seeded on vitronectincoated four-well chamber slides (Nalgen Nunc) and incubated with the respective growth medium (e.g. for C4-B2 cells: T-medium) containing 5% fetal bovine serum for 24 hours. After the cells are attached to the chamber slides, the cells are washed with PBS and exposed to the THSD/HMCD at a concentration of 20 µmol/l in the medium. The slides are incubated at 37°C for 30 minutes and washed twice with PBS to remove excess dyes, and cells are fixed with 10% formaldehyde at 4°C. The slides are then washed twice with PBS and covered with glass coverslips with an aqueous mounting medium (Sigma-Aldrich). Images are recorded by confocal laser microscopy (Zeiss LSM 510 META) using a 633-nm excitation laser and 670 to 810 nm long pass filter or a fluorescence microscope (Olympus 1 × 71) equipped with a 75-W Xenon lamp and an indocyanine green filter cube (excitation, 750-800 nm; emission, 820-860 nm; Chroma). To determine dye uptake in tissues, tissues isolated from tumor-bearing mice are placed in OCT (optimal cutting temperature) medium and frozen at -80°C. Frozen 5-µm tissue sections are prepared for histopathologic observation using the microscope as described above.

**Subcellular localization (mitochondria, lysosomes):** In the following examples, unless otherwise specified, uptake of THSD or HMCD controls into mitochondria and lysosomes of cancer cells, is determined as follows by virtue of each compound constituting a dye or comprising a dye moiety. Cells are plated on live-cell imaging chambers (World Precision Instrument) overnight. Cells are exposed to the dyes (i.e. THSD/HMCD) at different concentrations, and dye uptake is evaluated by a Perkin-Elmer Ultraview ERS spinning disc confocal microscope. This system is mounted on a Zeiss Axiovert 200 m inverted microscope equipped with a 37°C stage warmer, incubator, and constant CO₂ perfusion. A 63× or 100× Zeiss oil objective (numerical aperture, 1.4) is used for live cell images, and a Z-stack is created using the attached piezoelectric z-stepper motor. The 633-nm laser line of an argon ion laser (set at 60% power) is used to excite the THSD/HMCD. Light emission at 650 nm, although not optimal for these dyes, is detected and found to correlate directly with the dye concentrations in the cells. For comparative studies, the exposure time and laser intensity are kept identical for accurate intensity measurements. Pixel intensity is quantified using Metamorph 6.1 (Universal Imaging), and the mean pixel intensity is generated as gray level using the Region Statistics feature on the software. To determine the dye uptake by mitochondria, the mitochondrial tracking dye MitoTracker™ Green FM (Invitrogen™ Molecular Probes™) is used. To determine dye localization in lysosomes, lysosome-tracking dye LysoTracker™ Green DND-26 (Invitrogen™ Molecular Probes™) are used. Imaging of mitochondrial and/or lysosome localization of the dyes (THSD, HMCD) is conducted under confocal microscopy.

**Uptake and accumulation in tumors *in vivo:*** In the following examples, unless otherwise specified, the uptake and accumulation of THSD/HMCD in tumors of mice is determined *in vivo* as follows. Human cancer cells are implanted (1 × 106) either s.c., orthotopically, or intraosseously into 4- to 6-week-old athymic nude mice (National Cancer Institute). When tumor sizes reach between 1 and 6 mm in diameter, as assessed by X-ray or by palpation, mice are injected i.v. or i.p. with HMCD at a dose of 0.375 mg/kg or 10 nmol/20 g mouse body weight. Whole body optical imaging is taken at 24 hours or as indicated using a Kodak Imaging Station 4000 MM equipped with fluorescent filter sets (excitation/emission, 800:850 nm), with a field of view of 120 mm in diameter, a frequency rate for NIR excitation light of 2 mW/cm2, and the following camera settings: maximal gain, 2 × 2 binning, 1,024 × 1,024 pixel resolution, exposure time of 5 seconds. Live mice are alternatively/additionally imaged by an Olympus OV100 Whole Mouse Imaging System (excitation, 762nm; emission, 800 nm; Olympus Corp.), containing a MT-20 light source (Olympus Biosystems) and DP70 CCD camera (Olympus). Before imaging, mice are anesthetized with ketamine (75 mg/kg), and maintained in an anesthetized state during imaging.

**Example 1B:** Prostate Cancer (PC) cells (22RV1, ATCC® CRL-2505™, a human prostate carcinoma cell line) are cultured according to the standard protocol, and exposed for 24-48 hours, to 0-50 µM of the respective compound (THSD, unconjugated dye). Cell viability is determined by counting viable cells and dead cells after treatment of the cells with trypan blue dye to distinguish viable from dead cells. The results are shown in **FIG. 1B2** with the y-axis indicating % cell viability. It is shown that the IC₅₀ of the ALSD of formula FIId, "DZ2a", compound 7, for inhibition of the cancer cells is between about 3-4 µM, which is significantly improved over the unconjugated statin (>50 µM), and an unconjugated dye (here: "DZ1"). For chemical formulae of tested compounds, see **FIG. 1B1**.

**Example 1C:** PC (22RV1), LNCaP (LNCaP clone FGC (ATCC® CRL-1740™, human, prostate; derived from metastatic site: left supraclavicular lymph node) and C4-2B (ATCC® CRL-3315™, human prostate cancer cells of epithelial morphology) cancer cells are cultured as follows: 22RV1 and LNCaP are cultured according to the protocol described above using RPMI; C4-2B are cultured in RPMI with 10% FBS as described above. The cultured cells are exposed for about 1 h to 0-50 µM of the ALSD of formula FIId, "DZ2a", compound 7. Cell viability is determined as described in example 1B. The results are shown in **Fig. 1C** with the y-axis indicating % cell viability, and the x-axis indicating the dose of the drug in µM. It is shown that ALSD is similarly effective in different types of cancer cells, with an of about 3-4 µM.

**Example 1D:** 22Rv1 human prostate carcinoma cells are cultured as described above, separated from the medium, resuspended in 50% phosphate buffered saline (PBS) and 50% BD Matrigel™ (BD Biosciences, CA), and then the re-suspended cells are injected and grown subcutaneously in mice. Uptake and retention of the drug is monitored by NIR emission; the tumors showed uptake and retention of the ALSD for at least about 2 to 4 weeks or longer, especially in solid non-necrotic tumors/non-necrotic parts of the tumor, see **FIG. 1D****.** The decrease of the THSD in the tumors at 2 and 4 weeks, respectively, as determined by NIR signal, is less than about 30%.

**Example 1E:** 22Rv1 human prostate carcinoma cells are cultured, injected and grown subcutaneously in mice as described above in example 1D. Tumor volume is monitored by measuring the size by caliper and calculating volume as described in example 9 below. The mice are intravenously injected with the following: ALSD of formula FIId, "DZ2a", compound 7, the corresponding unconjugated dye (here: DZ1), unconjugated Simvastatin, a statin drug (SIM), and docetaxel (DOC), a cancer drug, and a mixture of the ALSD with DOC. Each active was administered in an amount of 5 mg/kg for the ALSD, DZ1, and SIM, except, in case of DOC, 8 mg/kg. The results are shown in **FIG. 1E****.** A slower rate of increased tumor volume indicates tumor suppressive activity. "Vehicle PBS" serves as a negative control, and thus shows the tumor growth without suppression. DZ1, SIM and DOC showed no significant tumor suppressive activity; in contrast, the ALSD showed tumor suppressive activity and significantly decreased tumor growth, as shown by a much slower rate of tumor volume increase. Addition of DOC combined with ALSD did not further increase the ALSD's tumor growth suppressive effect.

**Example 2:** 22Rv1 human prostate carcinoma cells are cultured as described above, and treated with DAPI (blue fluorescence of nuclei), the ALSD of formula FIId"DZ2a", compound 7, (yellow fluorescence), MitoTracker™ (green fluorescence), and LysoTracker™ (green fluorescence) as described herein-above. As shown in the composite, **see** **FIG. 2****,** the cell nuclei are stained blue by DAPI, while the red fluorescence of the ALSD co-localizes with that of green fluorescence of the MitoTracker™ and LysoTracker™ in the cytosolic fraction of the cancer cells, as indicated by the yellow fluorescence in the composite; the co-localization thus allows the ALSD to interfere with mitochondrial and lysosomal functions of the cancer cells.

**Example 3A:** The metabolic phenotype, and change thereof, as apparent from mitochondrial oxygen consumption rate (OCR), is determined for C2-4B MDVR cells upon exposure to different drugs and a control as described below. Drugs/controls include: Negative control/medium only ("NT"), the unconjugated dye ("DZ1", 6 µM), Simvastatin, an unconjugated statin drug ("SIM"), and Cholesterol ("Chole", 20 µM). The OCR results are shown in **FIG. 3A****,** and the ECAR results (with a different control) are described and shown in **example 3B** and **FIG. 3B****.** OCR and ECAR correspond to the two major energy pathways of the cell, mitochondrial respiration and glycolysis, with OCR serving as an indicator of mitochondrial respiration (oxidative phosphorylation), and ECAR as an indicator of glycolysis and lactate production. The initial OCR for all drugs is within 650-1000 pmol/min, with SIM having the highest, DZ1 having the lowest, and the other drugs having intermediate rates. Compared to all other drugs, the ALSD of formula FIId ("DZ2a", compound 7) significantly lowers the OCR; after about 200-300 minutes the rates drops lower than that of either NT, SIM, DZ1, or Cholesterol, and continues to decrease rapidly within 600-800 minutes to a low rate below 200-300 pmol/min (compared to 650-950 pmol/min for the other drugs), with the decrease continuing.

OCR and ECAR of live cells are determined in a metabolic chamber in a 24-well plate format using a Seahorse XF analyzer (Seahorse XFe24, Agilent, CA). Reagents (drugs/controls) are added to the wells just before start of the recording at time 0, and the effect of the drugs on the basal mitochondrial function is recorded by the analyzer for about 12 hours (720 minutes). All reagents used are Agilent reagents. Cells are seeded and cultured in Seahorse XF RPMI Medium, pH 7.4. The seeding rate is 8x10⁵ for the cells to reach about 90% confluency within about 24 hours when the analysis is started. The protocol is essentially performed as per the standard protocol of the manufacturer, removing the medium, washing the cells once with XF Real-Time ATP Rate Assay Medium, then starting the measurement as per the instrument's pre-programming and subsequent real-time measurements of OCAR/ECAR.

**Example 3B:** The Extracellular acidification rate ("ECAR") which indicates glucose consumption is determined as described in example 3A above. The results show an upwards bump at around 500 minutes in the curve for DZ2a, indicating a switch of the cell's metabolism to glucose.

**Example 3C:** C2-4B MDVR cells are used and cultured as described above. The cells are denatured with beta-mercapto-ethanol as a denaturing agent and a 20 µg portion is loaded into a lane of a denaturing gels to separate the proteins by size, the gel is run and the separated proteins are transferred from the gel onto a nitrocellulose membrane, and the proteins are identified by their NIR signal. The ALSD (here of formula FIId, "DZ2a", compound 7) is compared to an unconjugated dye (DZ1). The results are shown in **FIG. 3C****.** The figure shows that the ALSD binds to multiple mitochondrial and cytosolic proteins in the cytosolic and mitochondrial lysates, likely the binding is covalently. Compared to the unconjugated dye (DZ1), the strength of the signal may indicate higher affinity to some of the proteins, as well as additional proteins bound in case of additional bands appearing.

**Example 3D:** Proteins are denatured and separated as described in **example 3C;** on the membrane a western blot is performed using an antibody that targets ubiquitin for detection of polyubiquitinated proteins; the probes include a molecular weight marker ("Marker"), DMSO (vehicle which serves as a control), an unconjugated statin (Simvastatin), an unconjugated dye (DZ1), and the THSD (here ALSD of formula FIId, "DZ2a", compound **7**). The results are shown in **FIG. 3D****.** The strong signal of a smear of proteins of multitude sizes (rather than single/discrete bands) in the DZ2a lane indicates an accumulation of polyubiquitinated proteins, i.e. unfolded protein not cleared from the cell. Thus it is shown that THSD prevent the removal of polyubiquitinated proteins by cancer cells through altered autophagic processes which involve defective lysosomal functions.

**Example 3E:** Whole cells and mitochondria are prepared/treated as follows. 22RV1 prostate cancer cells are cultured as described herein-above. The cells are harvested, treated with Lithium dodecyl sulfate (LDS) detergent, then a whole cell fraction and an isolated mitochondrial fraction provided by centrifugation and the pellets are processed to determine their cholesterol levels as follows: cholesterol is extracted with hexane/methanol mixture (7:1, v/v), into which an appropriate amount of stable isotope-labeled d7-cholesterol standard (Avanti Polar Lipids) is spiked. Samples are sonicated in a water-bath, vortexed, and centrifuged at 16,000× g for 5 min at RT. The upper layers are dried down in a SpeedVac at RT. Then samples are incubated with 200 µL 10 mg/mL 4-(dimethylamino)phenyl isocyanate (DMAPI) in dichloromethane and 30 µL triethylamine at 65°C for 1.5 h in a ThermoMixer. The reaction is quenched by phosphate buffer (pH 8.0), followed by extraction of DMAPI-labeled cholesterol with hexane and vacuum concentration in a SpeedVac. The dry residues are reconstituted in acetonitrile/isopropanol (1:1) and analyzed by liquid chromatography-selected ion monitoring (LC-SIM) using an Ultimate 3000 XRS liquid chromatography system connected to an Orbitrap Fusion Lumos mass spectrometer (Thermo Scientific). Following separation on a 5-cm Hypersil GOLD C18 column, DMAPI-derivatized endogenous cholesterol (m/z 549.442) and d7-cholesterol (m/z 556.485) are analyzed by time-scheduled SIM analysis on an Orbitrap Fusion Lumos. The acquired LC-SIM data are analyzed by Skyline for cholesterol quantification.

The quantified cholesterol is shown in the table below.

| **Cancer cell materials:** | **Vehicle (Control)** | **SIM-treated** | **ALSD-treated** |
|---|---|---|---|
| Whole cells (nmol Cholesterol per 10⁶ cells) | 116 (100%) | 102 (88%) | 67 (58%) |
| Mitochondria (nmol Cholesterol per 10⁷ cells) | 108 (100%) | 56 (51%) | 34 (31%) |

The results show that ALSD (here of formula FIId, "DZ2a", compound **7**) is able to significantly lower cholesterol levels to about 58% in cancer cells or 34% in their mitochondria, respectively, compared to the control (see results shown in the table below). The statin Simvastatin lowers cholesterol less compared to the ALSD (to 88% in cancer cells or 51% in their mitochondria, respectively).

**Example 4:** A cell assay is performed exposing cells cultured as described above to various drugs indicated below for 72 hours. The following cells are used: Parental C4-2B cells, and resistant C4-B2 cells: C4-2B AbiR (resistant to Abiraterone acetate, an antiandrogen drug used in hormone treatment of prostate cancer) and Enzalutamide resistant cells ("MDVR"). The cells designated "C4-2B parental" are non-resistant C4-2B cells not previously exposed to a cancer drug. All C4-B2 based cells are cultured as described herein-above for the parental cells. Drug-resistant C4-B2 cells are created by exposure to the relevant drug. The cells are exposed to an ALSD (here of formula FIId, "DZ2a", compound **7**) in amounts of 2, 4, 8 or 16 µM. The results are shown in **FIG. 4A** (non-resistant parental C4-2B), **FIG. 4B** (Abiraterone acetate resistant or AbiR), and **FIG. 4C** (Enzalutamide resistant or MDVR). For all cell types, the cell survival rate decreases to 0% after 8 h exposure to 16 µM of the ALSD, less than 50% survive after 3 h exposure, and less than 25% of cells survive after 4 h exposure. If less ALSD is used, more cells survive during the period tested, e.g. 8 µM of the ALSD reduced the survival rate to below 25-50% within 4-8 hours, while lesser amounts take longer to reduce the cell survival rate. The results indicate that the ALSD decreases the cell survival rate and inhibits the growth of Abiraterone acetate-resistant and Enzalutamide-resistant cells, as well as that of non-resistant "parental" C4-2B cells. Amounts of about 4-5 µM ALSD are typically sufficient for decreased cell survival/growth inhibition of each of these resistant cells, compare e.g. **FIG. 4B and FIG. 4C****.**

Further, preliminary results indicate that the ALSD in amounts of about 5-20 µM, e.g. 14 µM or less, decreases the cell survival rate and is able to inhibit the growth of Docetaxel-resistant cells.

Compared to other drugs including e.g. Abiraterone acetate, Enzalutamide, and Docetaxel, the ALSD is able to provide much more rapid growth inhibition. For example, inhibition is achieved typically within less than 8-24 h, generally starting within less than about two hours and having a significant effect of decrease of cell survival to below 50% within the first about 3-8 hours, e.g. at 8-16 µM, compare **FIG. 4A-C**. Complete inhibition (100%) may be achieved within 8-18 hours, particularly at higher concentrations of e.g. about 16 µM. As the results indicate, the ALSD provides rapid and complete (0% cell survival or 100% growth inhibition) within less than 8-24 h, depending on dosage. Due to slow inactivation, amounts of e.g. about 4-5 µM ALSD or less may be sufficient for decreased cell survival/growth inhibition of either non-resistant or resistant cells over a longer time period, e.g. 16-48 hours, or about 24-32 hours. For Docetaxel-resistant cells higher amounts may be required, e.g. about 5 to about 20 µM or less, e.g. about 14 µM, to decrease the cell survival rate and inhibit the growth of (data not shown). Similarly, the ELSD may provide a rapid growth inhibition, as apparent from its less steep dose-response curve, compare e.g. **example 7D and E.**

**Example 5:** This example shows that a THSD can prevent the anchoring of RhoA/B onto cell membranes, likely due to inhibition of protein prenylation and its downstream cell signaling by the THSD. RhoA/RhoB membrane staining of tumor tissue of a H-1975 human lung cancer cell mouse model is performed as described below, and the tumor-bearing mice are treated with a THSD (here an ALSD of formula FIId, "DZ2a", compound 7) in comparison to the corresponding unconjugated dye ("DZ1") and to Gefinitib (an EGFR inhibitor drug used for treatment of certain breast, lung and other cancers). The results show that staining persists in the membranes of DZ1- and Gefitinib-treated tumors, but not in ALSD- and ALSD/Gefitinib-treated tumors **(see** **FIG. 5****).** This demonstrates that ALSD prevents the anchoring of RhoA/B onto cell membranes, likely due to inhibition of protein prenylation by the ALSD.

H-1975 cells, a cell model for human lung cancer that is resistant to Gefitinib (an EGFR inhibitor drug used for treatment of certain breast, lung and other cancers) are cultured as described above and injected subcutaneously into mice to develop tumors. The mice and the tumors that are formed are systemically exposed to an ALSD (here of formula FIId, "DZ2a", compound 7) by intravenous injection. The tumor-bearing mice are treated with either Gefitinib (20 mg/kg), or with an ALSD (here of formula FIId, "DZ2a", compound 7, 5 mg/kg), an unconjugated dye of formula III ("DZ1", 5 mg/kg) or a combination of the ALSD with Gefitinib. Upon ALSD exposure, the tumors show significant shrinkage of tumor volume as determined by visual inspection after removal, and regular tumor measurements calculating tumor size and/or volume by calipers (data not shown).

For immunostaining of tumor tissue, the animals are sacrificed by prescribed and approved euthanasia techniques. Tumor tissue (maximum thickness 3 mm) is sliced, formalin fixated, embedded into paraffin blocks which are sectioned and processed into paraffin section slides. For immunohistochemical staining, the slides are deparaffinized and re-hydrated (heat, xylene, alcohol, 3% H₂O₂ solution in methanol, optional exposure to Retrievagen A (pH 6.0), BD Pharmingen™, Cat. #550524). Slides are exposed to RhoA (26C4) monoclonal antibody 1:1000, (Santa Cruz, CAT# SC-418). Alternatively one or more other Rho A and/or Rho B monoclonal antibody may be used. An HRP-based procedure (Hores Raddish Peroxidase, to catalyze a chromogenic substrate for detection by microscopy) may be employed after blocking of endogenous peroxidase. The primary antibody and a negative control antibody are applied (e.g. RhoA1: 1000 and neg. control, e.g. Mouse IgG1 negative control, Agilent Dako, CA, 1:100). After exposure and rinsing, a biotinylated secondary antibody (e.g. LSAB2 Kit, Agilent Dako, CA) is applied, then Streptavidin LSAB2, then DAB chromogen with enhancer (Signet) 20ul/ml, and a counter stain with Hematoxylin 50. The stained slides are dehydrated and cleared with graded alcohols and xylene, then mounted and examined under a microscope suitable for the chromophore used. For the discussion of results see above.

**Example 6A: In vivo administration of THSD to mammals (rat, dog).** A THSD (here an ALSD, DZ1-SIM amide of formula FIId or "DZ2a") is administered to 4 animals (N=2M+2F), 4 beagle dogs (7.38-8.59 kg) and 4 Wistar rats (220-235 g), respectively. Administration is IV, 1 mg/kg (1 ml/kg), via cephalic vein injection (dogs) or via foot dorsal vein injection (rats), respectively. The animals are given free access to feed and water. Samples are taken at time points (hr) 0.083, 0.25, 0.5, 1, 2, 4, 8, 24. For blood collection at the designated time points, each animal is manually restrained, and approximately 150 µl of blood is collected via cephalic vein puncture (dogs) or via tail vein (rats) for serial bleeding into EDTA-K2 sodium tubes. The blood samples are maintained in wet ice and within 15 minutes post sampling centrifuged (2000g, 4°C, 5 min) to obtain plasma. All plasma samples are diluted 2 fold with the same volume of 0.5% FA in water, and stored at approximately -70°C until analysis. The concentration of the THSD and the unconjugated statin (Simvastatin) are analytically determined by LCMSMS-025 (AB SCIEX 6500 LC/MS/MS System). MS performed is positive ion, ESI. MRM detection is performed. HPLC conditions are H₂O-0.1%FA and ACN-0.1%FA, on the following column: Poroshell 120, EC-C18, 50^{∗}2.1mm, 2.7 µm, with a flow rate of 0.50 ml/min, and a column temperature of 45 °C. Calibration curves with internal standards are established (1.00-1000 ng/ml THSD, 2.00-2000 ng/ml Simvastatin). The matrix of the sample is animal blood plasma mixed with equal volume of 0.5% formic acid in water solution. An aliquot of 30.0 µl sample is added to 200 µl IS in ACN containing 0.1%FA. The mixture is vortexed for 5 min and centrifuged at 13000 rpm for 5 min. The 2.00 µl mixture is injected into LC-MS/MS.

Representative results are shown in the tables below. For both dog and rat samples, the THSD is determined in significant amounts within 5 minutes and peak concentration is reached within about 5-15 (dog) or 15-30 (rat) minutes, which then decreases over time to 0 (no peak) after about 8 hours. In contrast, the unconjugated statin is either not determined, or determined only in trace amounts, over the whole sample period of 5 min to 24 hours, and the highest trace concentration determined well below 1 ng/ml (highest concentration rat 0.094 ng/ml, and dog 0.46 ng/ml). The results indicate that the THSD is not releasing the statin, as it is not determined in the blood plasma.

| **Rat** | | |
|---|---|---|
| **Sample time (h)** | **THSD (ng/ml)** | **Simvastatin (ng/ml)** |
| 0.083 | 1,095.67 | 0.094*, 0.024, 0.019 or <0 |
| 0.25 | 3,662.8 | 0.016*, no peak, or <0 |
| 0.5 | 9,468.8 | no peak or <0 |
| 1 | 161.3 | no peak or <0 |
| 2 | 14.8 | no peak or <0 |
| 4 | 3.9 | no peak or <0 |
| 8 | 1.1 | no peak or <0 |
| 24 | no peak | no peak or <0 |

| | | |
|---|---|---|
| *highest value determined | | |

| **Dog** | | |
|---|---|---|
| **Sample time (h)** | **THSD (ng/ml)** | **Simvastatin (ng/ml)** |
| 0.083 | 11,134.2 | no peak |
| 0.25 | 6,976.9 | no peak |
| 0.5 | 2,855.9 | 0.32-0.46* |
| 1 | 1,419.4 | 0.27-0.37* |
| 2 | 320.3 | 0.37*, 0.3, or no peak |
| 4 | 16.8 | 0.26* or no peak |
| 8 | 1.75 | 0.29* or no peak |
| 24 | 0.34 | no peak |

| | | |
|---|---|---|
| *highest value determined | | |

**Example 6B: In vivo administration of THSD to mammals (rat, dog) and determination of plasma concentration over time.** The experiment is performed as described above in example 6A, with intravenous administration of 1 mg/kg of a THSD (here an ALSD, DZ1-SIM amide of formula FIId or "DZ2a"); blood samples are taken from the animals in intervals as above for 24 hours total, and for each sample, the THSD is quantitatively measured as described above, calculating its plasma concentration. Graphs of the results are shown in **FIG. 6B1** (mean concentration for female and male rats, respectively) and **FIG. 6B2** (mean concentration for female and male dogs, respectively), with the measurement being below the quantifiable limit at 24 hours (not shown). The graphs show a half-life of about 1 hour, here about 0.6 to about 1.7 in the mammals, e.g. from about half an hour in female dogs, to less than about 2 hours in male rats. The related PK parameters are listed below.

| PK parameters | Unit | Rats, male (mean) | Rats, female (mean) | Dogs, male (mean) | Dogs, female (mean) |
|---|---|---|---|---|---|
| CL | L/hr/kg | 0.154 | 0.190 | 0.103 | 0.0710 |
| Vₛₛ | L/kg | 0.0477 | 0.0446 | 0.0531 | 0.0375 |
| Regression time | hr | NA | NA | NA | NA |
| Terminal T_{1/2} | hr | 1.70 | 1.21 | 0.87 | 0.620 |
| AUCₗₐₛₜ | hr*ng/mL | 6514 | 5401 | 9705 | 14099 |
| AUC_{INF} | hr*ng/mL | 6523 | 5409 | 9710 | 14103 |
| NMT_{INF} | hr | 0.311 | 0.235 | 0.517 | 0.529 |

**Example 7:** The experiment is performed by cell assay essentially as described above in **example 4.** All cell lines are human cancer cell lines and are cultured as described herein-above; they include: 22RV1 (a prostate carcinoma cell line), MDVR (an EZ-resistant variety of C4-2B prostate cancer cells formed as described in **Example 4** herein-above), PC3 (a prostate cancer cell line initiated from a bone metastasis of a grade IV prostatic adenocarcinoma (AC)), A549 cells (a lung carcinoma cell line), A549/DDP cells (MDR A549/cisplatin lung adenocarcinoma cell line, drug resistant), H1975 (an AC/NSCLC cell line and a cell model for TKI-resistant cancer, particularly Gefitinib resistance), H1650 (an AC/NSCLC cell line), PC9 (an AC/NSCLC lung cancer cell line with EGFR mutation of mixed morphology), PC9AR cells (an osimertinib/AZD9291-resistant cell line which may be formed from the parental cell line by exposure of PC9 cells to osimertinib (also known as AZD9291) and H446 (a SCLC cell line).

The cell lines PC9, H1975 and H1650 have the following EGFR-activating mutations: PC9 has the EGFR-activating mutation T790M. H1975 has L858R/T790M double mutations, and H1650 has an EGFR exon 19 deletion (Del). Other mutations include the following. In H446 cells, c-myc DNA sequences are amplified about 20 fold, and there is a 15 fold increase in c-myc RNA relative to normal cells.

The cell lines are cultured according to the protocols as described above, then each of the cell lines are treated for 48 h either with an ELSD (here of formula FId, "DZ2b" or compound **14**) or with an ALSD (here of formula FIId, "DZ2a" or compound **7**). For each cell line, the is determined for each drug, i.e. the ELSD, the ALSD, and for the corresponding unconjugated statin (Simvastatin), in concentrations from 0.1-100 µM.

Cell viability (IC₅₀) is determined by an MTT assay as follows: 1×10⁴/ml cells in 100 µl are treated with increasing concentrations of the drug or a control (THSD, DZ1, SIM) for 24 hours. In the controls (not shown in the figures), the cells are exposed to DMSO (vehicle) for a final concentration that equals the highest concentration of the drug tested, to a maximum concentration less than 0.1% v/v. 4 hours before culture end/SDS addition, 10 µL MTT (3-(4,5-Dimethyl-2-thiazolyl)-2,5-diphenyl-2H-tetrazolium bromide, Sigma-Aldrich) is added to wells containing the cells. At the end of culture, 100 µl 10% SDS is added and then the plate containing the cells is placed in a 37°C cell culture incubator for 8 hours. The absorbance density of the supernatant is read on a 96-well microplate reader at wavelength 595 nm. All IC₅₀ are relative IC50, and based on the curves fitted as shown in the figures.

The results are indicated in the table below, and the corresponding curves are shown in **FIG. 7A-I**.

| Cell lines (PC-Prostate Cancer; LC-Lung cancer) | **IC50 (µM) of ELSD** | **IC50 (µM) of ALSD** | **IC50 (µM) of Unconjugated statin (SIM)** | **IC50 (µM) of Unconjugated Dye (DZ1)** |
|---|---|---|---|---|
| **22RV1 (PC)** | 7.8 | 10.8 | 38.7 | n/a |
| **EZ-resistant C4-2B (PC)** | 3.8 | 5.6 | 25.5 | n/a |
| **PC3 (PC)** | 6.0 | 3.5 | 14.8 | n/a |
| **A549 (LC)** | 5.4 | 5.7 | >50 | >50 |
| **A549DDP (LC)** | 2.1 | 6.1 | 30 | >50 |
| **H1975 (LC)** | 6.4 | 2.9 | >50 | >50 |
| **H1650 (LC)** | 11.8 | 3.6 | 33.3 | >50 |
| **PC9 (LC)** | 5.6 | 9.6 | 33 | >50 |
| **H446 (LC)** | 3.4 | 3.7 | 39 | >50 |

For the three different cancer cell lines, when comparing the of the THSD to that of the unconjugated statin or dye, the trend is similar: the for the THSD (ELSD and ALSD) range from about 2 to about 12 µM, while the of the unconjugated statin is much higher and ranges from about 15 to about >50 µM (i.e. more unconjugated statin is needed for the same effect on cell viability). Thus both ALSD and ELSD reduce cell viability and inhibit the growth of human prostate cancer cells better than the unconjugated statin (also compare the graphs in **FIG. 7A-I**).

**Example 8:** The example is performed essentially as described in example 7 above, using A549 cells (a lung carcinoma cell line), and treated with various concentrations of a ALSD (shown here for an ALSD of formula FIId, "DZ2a", compound **7**) for 24, 72, or 120 hours respectively, followed by detection of cell viability by the MTT assay described below. Results (compare graphs shown in **FIG. 8**) indicate a strong inhibition of growth (IC₅₀ from about 5.7 µM to 5.9 µM) regardless of the time of incubation (24 to 120 hours), which indicates that the ALSD is a drug (rather than a pro-drug) which directly inhibits tumor growth without the requirement of enzymatic release of the statin from its amide linkage to the dye moiety of the ALSD. Similarly, the slow release ELSD only minimally releases the statin (data not shown).

**Example 9:** TKI-resistant cell lines are established by exposing sensitive cell lines to the TKI, here, for example, the 3^{rd} generation EGFR-TKI AZD9291, or existing sensitive cell lines may be used. Other TKI and EGFR-TKI may be established and tested accordingly. AZD9291 is purchased from Active Biochemicals. "DZ-SIM" amide, a THSD of formula FId (compound **7,** an ALSD) is tested *in vivo* in a mouse xenograft model for human drug resistant prostate cancer, in comparison to the unconjugated statin, the unconjugated dye, the EGFR-TKI drug, and a control as described below. The human prostate cancer cells are PC9AR cells that are resistant to third generation epidermal growth factor receptor (EGFR) tyrosine kinase inhibitor (TKI) drugs (EGFR-TKI). The PC9AR cells are cultured as described above. 5^{∗}10⁶ PC9AR cells with one third matrigel are inoculated subcutaneously on the flanks of 5 week-old female mice. After 7 days of inoculation, when tumor size went to about 50 mm³, mice are treated three times a week for 16 days total with the following: vehicle/neg. control (1% Tween80, i.p.), DZ1 (5 mg/kg, i.p.), Simvastatin (5 mg/kg, i.p.), DZ1-SIM (5 mg/kg, i.p.), AZD9291 (10 mg/kg, o.g.), and a combination of DZ1-SIM (5 mg/kg, i.p.) and the 3^{rd} generation EGFR-TKI AZD9291 (10 mg/kg, o.g.). Tumor volumes are measured using caliper measurements and calculated with the formula 2a×b×0.5, with a and b being the two main dimensions (width, length) of the tumor and a < b. After treatment for 16 days, the mice are killed and the tumor tissue is separated, weighed and imaged. Photographs, weight and volume of the tumors and the weight of the mice are shown in **FIG. 9A1-4****.** The standard deviation and confidence level are indicated in the figures as follows: Mean ± S.D. (n = 6). *P<0.05, **P<0.01, ***P<0.0005, **** P<0.0001. The results are indicated in detail in **FIG. 9A1-4** and their description herein-above. In summary, a dramatic growth inhibition as shown by tumor size, weight and volume measurements over time was determined in human cancer cell derived tumors treated with the THSD, but not in tumors treated with any other agent. Combination of the THSD with the TKI did not increase THSD growth inhibition.

**Example 10:** THSD (ALSD and ELSD, respectively) are tested *in vivo* in a mouse xenograft model for human lung cancer. The example is essentially performed as described for example 9 with the following modifications: human SCLC H446 cancer cells are used, and the tested agents include two statin conjugates (compounds of formula Fid/compound **14** or "DZ2b" and FIId/compound **7** or "DZ2a", respectively, designated "DZ-SIM-Ester" or "DZ-SIM-Amide" in **FIG. 10**), and a non-statin conjugate ("DZ-DHA"). SCLC H446 cells (1.5x10⁶) are subcutaneously implanted on the bilateral flanks of athymic nude mice at age of 5 weeks. 10 days post cell implantation, mice are treated with DZ1 (2.8 mg/kg), SIM (2 mg/kg), DZ1-SIM-Amide/compound **7** or DZ1-SIM-Ester/compound **14** (4 mg/kg), and DZ1-DHA (4 mg/kg, DZ1-dihydroartemisinin ester "DZ1-DHA", as shown in **FIG. 11**), twice a week via i.p. injection. (A) Tumor sizes are measured by caliper every four days during the treatment period of 52 days, and volumes are calculated using formula 0.5x2axb as described in example 9 above. Confidence levels are indicated as *P<0.05, **P<0.01, ***P<0.0005. The results are shown in **FIG. 10****.** In summary, both THSD provide dramatically increased growth inhibition in the human cancer tissue when compared to all other agents; the growth inhibition is slightly increased also when compared to the non-statin dye conjugate.

It should be noted that the features illustrated in the drawings are not necessarily drawn to scale, and features of one embodiment may be employed with other embodiments as apparent to a person of ordinary skill, even if not explicitly stated herein. Descriptions of various well-known components and techniques are omitted or shortened to avoid obscuring the embodiments.

While multiple embodiments are disclosed, still other embodiments of the present invention will become apparent to those of ordinary skill from this detailed description. The invention is capable of myriad modifications in various aspects apparent to a person of ordinary skill, all without departing from the spirit and scope of the present invention. Accordingly, the drawings and descriptions are to be regarded as illustrative in nature rather than restrictive.

Further aspects and/or embodiments of the invention are described in the following clauses:
Clause 1: An ester-linked statin derivative shown in formula FIa below: 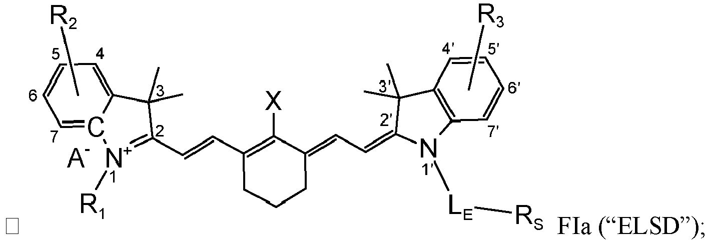
   wherein X is a halogen residue,
   wherein R₁ is a residue selected from the group consisting of C₁-C₂₅ alkyl, C₅-C₂₅ aryl, C₅-C₂₅ indolyl, C₅-C₂₅ thienyl, C₅-C₂₅ phenyl, C₅-C₂₅ napththyl, C₁-C₂₅ aralkyl, C₁-C₂₅ alkylsulphonate, C₁-C₂₅ alkylcarboxyl, C₁-C₂₅ alkylamino, C₁-C₂₅ ω-alkylaminium, C₁-C₂₅ ω-alkynyl, a PEGyl polyethylene chain with (-CH₂-CH₂-O-)₂₋₂₀, a PEGylcarboxylate with (-CH₂-CH₂-O-)₂₋₂₀, a ω-PEGylaminium with (-CH₂-CH₂-O-)₂₋₂₀, a ω-acyl-NH, a ω-acyl-lysinyl-, a ω-acyl-triazole, a ω-PEGylcarboxyl-NH- with (-CH₂-CH₂-O-)₂₋₂₀, a ω-PEGylcarboxyl-lysinyl with (-CH₂-CH₂-O-)₂₋₂₀, and a ω-PEGylcarboxyl-triazole with (-CH₂-CH₂-O-)₂₋₂₀;
   wherein R₂ and R₃ are residues independently selected from the group consisting of: a hydrogen, an C₁-C₂₀ alkyl, a sulphonate, a C₁-C₂₀ alkylcarboxyl, C₁-C₂₀ alkylamino, C₁-C₂₀ aryl, -SO₃H, -PO₃H, -OH, -NH₂, and a halogen residue;
   and wherein R₂ and R₃ are attached at a ring position selected from the group consisting of 3, 3', 4, 4', 5, 5', 6, and 6';
   wherein the A⁻ group is a pharmaceutically acceptable negatively charged anion;
   wherein an ester linker L_{E} is linked to a statin residue R_{S} by an ester bond;
   wherein L_{E} is selected from the group consisting of L_{E1} and L_{E2}, wherein L_{E1} is -(CH₂)ₙ-O-, and wherein L_{E2} is -(CH₂)ₙ-CO-NH-(CH₂)ₘ-O-, and wherein n=4-9, and m=1-4;
   wherein Rs is a residue of a statin or of a statin derivative,
   wherein R_{S} comprises a dihydroxyheptanoic acid unit (DHHA) that is connected to residue R_{S}* which is the remainder of the statin,
   and wherein Rs is linked to L_{E} via its DHHA in its open chain form which is -CO-CH₂-COH-CH₂-CHOH-R_{S*}.
Clause 2: The ELSD of clause 1 wherein the statin is selected from the group consisting of: Simvastatin, Mevastatin, Lovastatin, Pravastatin, Atorvastatin, Fluvastatin, Rosuvastatin, Cerivastatin, Pitavastatin, and derivatives thereof which retain binding to the statin's active binding site.
Clause 3: The ELSD of clause 1 wherein the statin is Simvastatin and wherein the ester linker L_{E} is L_{E2}, as shown in Formula FIb below: 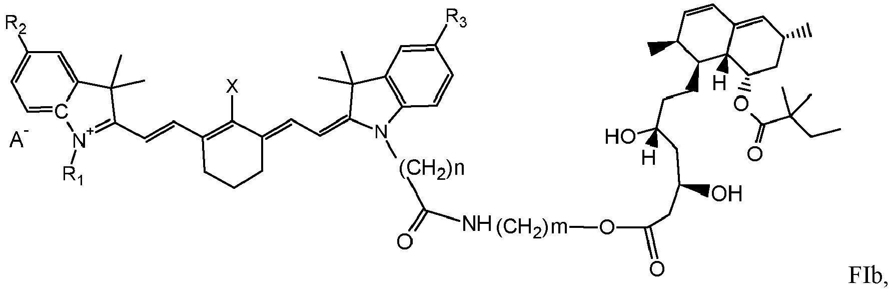 and wherein A⁻, X, R₁, R₂, R₃, and n and m of the L_{E2} linker are defined as for FIa.
Clause 4: The ELSD of clause 1, wherein X is Cl.
Clause 5: The ELSD of clause 1, wherein R₁ is a -(CH₂)n-SO₃⁻ alkylsulphonate residue, and wherein n of R₁ is selected from 2, 3, 4, 5, 6, 7 and 8.
Clause 6: The ELSD of clause 1, wherein R₁ is a -(CH₂)₄-SO₃⁻ alkylsulphonate residue.
Clause 7: The ELSD of clause 1, wherein R₂ and R₃ are H.
Clause 8: A pharmaceutical composition comprising an ELSD and one or more pharmaceutical excipient, wherein the ELSD is selected from the group consisting of:
   (i) An ester-linked statin derivative shown in formula FIa below:, 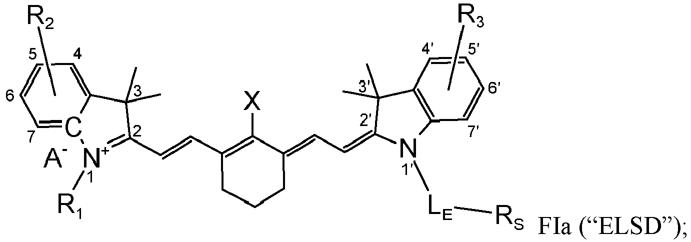
      wherein X is a halogen residue,
      wherein R₁ is a residue selected from the group consisting of C₁-C₂₅ alkyl, C₅-C₂₅ aryl, C₅-C₂₅ indolyl, C₅-C₂₅ thienyl, C₅-C₂₅ phenyl, C₅-C₂₅ napththyl, C₁-C₂₅ aralkyl, C₁-C₂₅ alkylsulphonate, C₁-C₂₅ alkylcarboxyl, C₁-C₂₅ alkylamino, C₁-C₂₅ ω-alkylaminium, C₁-C₂₅ ω-alkynyl, a PEGyl polyethylene chain with (-CH₂-CH₂-O-)₂₋₂₀, a PEGylcarboxylate with (-CH₂-CH₂-O-)₂₋₂₀, a ω-PEGylaminium with (-CH₂-CH₂-O-)₂₋₂₀, a ω-acyl-NH, a ω-acyl-lysinyl-, a ω-acyl-triazole, a ω-PEGylcarboxyl-NH- with (-CH₂-CH₂-O-)₂₋₂₀, a ω-PEGylcarboxyl-lysinyl with (-CH₂-CH₂-O-)₂₋₂₀, and a ω-PEGylcarboxyl-triazole with (-CH₂-CH₂-O-)₂₋₂₀;
      wherein R₂ and R₃ are residues independently selected from the group consisting of: a hydrogen, an C₁-C₂₀ alkyl, a sulphonate, a C₁-C₂₀ alkylcarboxyl, C₁-C₂₀ alkylamino, C₁-C₂₀ aryl, -SO₃H, -PO₃H, -OH, -NH₂, and a halogen residue;
      wherein R₂ and R₃ are attached at a ring position selected from the group consisting of 3, 3', 4, 4', 5, 5', 6, and 6';
      wherein the A⁻ group is a pharmaceutically acceptable negatively charged anion;
      wherein an ester linker L_{E} is linked to a statin residue R_{S} by an ester bond;
      wherein L_{E} is selected from the group consisting of L_{E1} and L_{E2}, wherein L_{E1} is -(CH₂)ₙ-O-, and wherein L_{E2} is -(CH₂)ₙ-CO-NH-(CH₂)ₘ-O-, and wherein n=4-9, and m=1-4;
      wherein Rs is a residue of a statin or of a statin derivative,
      wherein R_{S} comprises a dihydroxyheptanoic acid unit (DHHA) that is connected to residue R_{S*} which is the remainder of the statin,
      and wherein Rs is linked to L_{E} via its DHHA in its open chain form which is -CO-CH₂-COH-CH₂-CHOH-R_{S*}.
   (ii) the ELSD of FIa as defined in (i), wherein the statin to form R_{S} is selected from the group consisting of: Simvastatin, Mevastatin, Lovastatin, Pravastatin, Atorvastatin, Fluvastatin, Rosuvastatin, Cerivastatin, Pitavastatin, and derivatives thereof which retain binding to the statin's active binding site;
   (iii) an ELSD of formula FIb, wherein the statin is Simvastatin and the ester linker L_{E} is L_{E2} (-(CH₂),-CO-NH-(CH₂)ₘ-O-) as shown below: 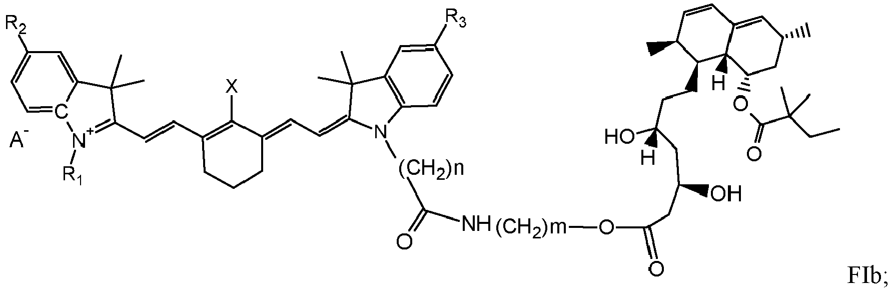 and wherein A⁻, X, R₁, R₂, R₃, and n and m of the L_{E2} linker, are defined as for FIa in (i) above;
   (iv) an ELSD as defined in (i), wherein X is Cl;
   (v) an ELSD as defined in (ii), wherein X is Cl;
   (vi) an ELSD as defined in (iii), wherein X is Cl;
   (vii) an ELSD as defined in (i), wherein R₁ is a -(CH₂)n-SO₃⁻ alkylsulphonate residue and wherein n of R₁ is selected from 2, 3, 4, 5, 6, 7 and 8;
   (viii) an ELSD as defined in (ii), wherein R₁ is a -(CH₂)n-SO₃⁻ alkylsulphonate residue and wherein n of R₁ is selected from 2, 3, 4, 5, 6, 7 and 8;
   (ix) an ELSD as defined in (iii), wherein R₁ is a -(CH₂)n-SO₃⁻ alkylsulphonate residue and wherein n of R₁ is selected from 2, 3, 4, 5, 6, 7 and 8;
   (x) an ELSD as defined in (i), wherein R₁ is a -(CH₂)₄-SO₃⁻ alkylsulphonate residue;
   (xi) an ELSD as defined in (ii), wherein R₁ is a -(CH₂)₄-SO₃⁻ alkylsulphonate residue;
   (xii) an ELSD as defined in (iii), wherein R₁ is a -(CH₂)₄-SO₃⁻ alkylsulphonate residue.
Clause 9: The pharmaceutical composition of clause 8 comprising one or more ELSD and further comprising an ALSD formula FIIa below: 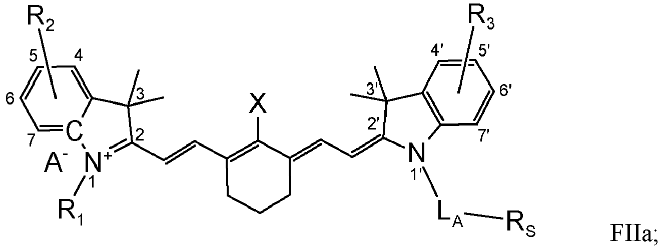
   wherein X is a halogen residue;
   wherein R₁ is a residue selected from the group consisting of C₁-C₂₅ alkyl, C₅-C₂₅ aryl, C₅-C₂₅ indolyl, C₅-C₂₅ thienyl, C₅-C₂₅ phenyl, C₅-C₂₅ napththyl, C₁-C₂₅ aralkyl, C₁-C₂₅ alkylsulphonate, C₁-C₂₅ alkylcarboxyl, C₁-C₂₅ alkylamino, C₁-C₂₅ ω-alkylaminium, C₁-C₂₅ ω-alkynyl, a PEGyl polyethylene chain with (-CH₂-CH₂-O-)₂₋₂₀, a PEGylcarboxylate with (-CH₂-CH₂-O-)₂₋₂₀, a ω-PEGylaminium with (-CH₂-CH₂-O-)₂₋₂₀, a ω-acyl-NH, a ω-acyl-lysinyl-, a ω-acyl-triazole, a ω-PEGylcarboxyl-NH- with (-CH₂-CH₂-O-)₂₋₂₀, a ω-PEGylcarboxyl-lysinyl with (-CH₂-CH₂-O-)₂₋₂₀, and a ω-PEGylcarboxyl-triazole with (-CH₂-CH₂-O-)₂₋₂₀;
   wherein R₂ and R₃ are residues independently selected from the group consisting of: a hydrogen, an C₁-C₂₀ alkyl, a sulphonate, a C₁-C₂₀ alkylcarboxyl, C₁-C₂₀ alkylamino, C₁-C₂₀ aryl, -SO₃H, -PO₃H, -OH, -NH₂, and a halogen residue;
   wherein R₂ and R₃ are attached at a ring position selected from the group consisting of 3, 3', 4, 4', 5, 5', 6, and 6';
   wherein the A⁻ group is a pharmaceutically acceptable negatively charged anion;
   wherein an amide linker L_{A} is linked to a statin residue R_{S} via an amide bond;
   wherein L_{A} is a linker selected from the group consisting of L_{A1} and L_{A2}, wherein L_{A1} is-(CH₂)ₙ-NH-, and wherein L_{A2} is -(CH₂)ₙ-CO-NH-(CH₂)ₘ-NH- and wherein n=4-9, and m=1-4;
   wherein Rs is a residue of a statin or of a statin derivative,
   wherein R_{S} comprises a dihydroxyheptanoic acid unit (DHHA) that is connected to residue R_{S*} which is the remainder of the statin,
   and wherein Rs is linked to L_{A} via its DHHA in its open chain form which is -CO-CH₂-COH-CH₂-CHOH-R_{S*}.
Clause 10: A method of treating cancer wherein one or more ELSD, and optionally one or more ALSD, are administered to a patient in need thereof in amounts sufficient to inhibit cancer cell or pre-cancerous cell growth or induce apoptosis in cancer or pre-cancerous cells in the patient,
   wherein the one or more ELSD is selected from the group consisting of:
   (i) An ester-linked statin derivative shown in formula FIa below:, □ 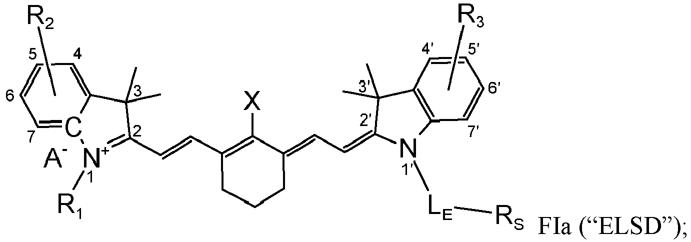
      wherein X is a halogen residue,
      wherein R₁ is a residue selected from the group consisting of C₁-C₂₅ alkyl, C₅-C₂₅ aryl, C₅-C₂₅ indolyl, C₅-C₂₅ thienyl, C₅-C₂₅ phenyl, C₅-C₂₅ napththyl, C₁-C₂₅ aralkyl, C₁-C₂₅ alkylsulphonate, C₁-C₂₅ alkylcarboxyl, C₁-C₂₅ alkylamino, C₁-C₂₅ ω-alkylaminium, C₁-C₂₅ ω-alkynyl, a PEGyl polyethylene chain with (-CH₂-CH₂-O-)₂₋₂₀, a PEGylcarboxylate with (-CH₂-CH₂-O-)₂₋₂₀, a ω-PEGylaminium with (-CH₂-CH₂-O-)₂₋₂₀, a ω-acyl-NH, a ω-acyl-lysinyl-, a ω-acyl-triazole, a ω-PEGylcarboxyl-NH- with (-CH₂-CH₂-O-)₂₋₂₀, a ω-PEGylcarboxyl-lysinyl with (-CH₂-CH₂-O-)₂₋₂₀, and a ω-PEGylcarboxyl-triazole with (-CH₂-CH₂-O-)₂₋₂₀;
      wherein R₂ and R₃ are residues independently selected from the group consisting of: a hydrogen, an C₁-C₂₀ alkyl, a sulphonate, a C₁-C₂₀ alkylcarboxyl, C₁-C₂₀ alkylamino, C₁-C₂₀ aryl, -SO₃H, -PO₃H, -OH, -NH₂, and a halogen residue;
      wherein R₂ and R₃ are attached at a ring position selected from the group consisting of 3, 3', 4, 4', 5, 5', 6, and 6';
      wherein the A⁻ group is a pharmaceutically acceptable negatively charged anion;
      wherein an ester linker L_{E} is linked to a statin residue R_{S} by an ester bond;
      wherein L_{E} is selected from the group consisting of L_{E1} and L_{E2}, wherein L_{E1} is -(CH₂)ₙ-O-, and wherein L_{E2} is -(CH₂)ₙ-CO-NH-(CH₂)ₘ-O-, and wherein n=4-9, and m=1-4;
      wherein the statin residue R_{S} is a residue of a statin or of a statin derivative that is linked to L_{E} via its dihydroxyheptanoic acid unit (DHHA) in its open chain form which is -CO-CH₂-COH-CH₂-CHOH-R_{S*} wherein R_{s*} is the remainder of the statin residue R_{S}.
   (ii) the ELSD of FIa as defined in (i), wherein the statin to form R_{S} is selected from the group consisting of: Simvastatin, Mevastatin, Lovastatin, Pravastatin, Atorvastatin, Fluvastatin, Rosuvastatin, Cerivastatin, Pitavastatin, and derivatives thereof which retain binding to the statin's active binding site;
   (iii) an ELSD of formula FIb, wherein the statin is Simvastatin and the ester linker L_{E} is L_{E2} (-(CH₂)ₙ-CO-NH-(CH₂)ₘ-O-) as shown below: 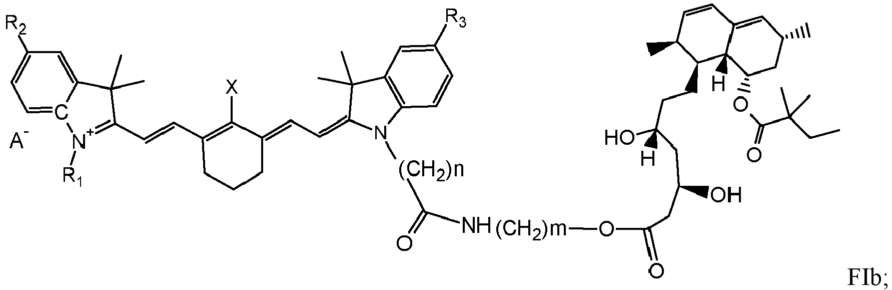 and wherein A⁻, X, R₁, R₂, R₃, and n and m of the L_{E2} linker, are defined as for FIa in (i) above;
   (iv) an ELSD as defined in (i), wherein X is Cl;
   (v) an ELSD as defined in (ii), wherein X is Cl;
   (vi) an ELSD as defined in (iii), wherein X is Cl;
   (vii) an ELSD as defined in (i), wherein R₁ is a -(CH₂)n-SO₃⁻ alkylsulphonate residue and wherein n of R₁ is selected from 2, 3, 4, 5, 6, 7 and 8;
   (viii) an ELSD as defined in (ii), wherein R₁ is a -(CH₂)n-SO₃⁻ alkylsulphonate residue and wherein n of R₁ is selected from 2, 3, 4, 5, 6, 7 and 8;
   (ix) an ELSD as defined in (iii), wherein R₁ is a -(CH₂)n-SO₃⁻ alkylsulphonate residue and wherein n of R₁ is selected from 2, 3, 4, 5, 6, 7 and 8;
   (x) an ELSD as defined in (i), wherein R₁ is a -(CH₂)₄-SO₃⁻ alkylsulphonate residue;
   (xi) an ELSD as defined in (ii), wherein R₁ is a -(CH₂)₄-SO₃⁻ alkylsulphonate residue;
   (xii) an ELSD as defined in (iii), wherein R₁ is a -(CH₂)₄-SO₃⁻ alkylsulphonate residue;
   and wherein the optional one or more ALSD is selected from the group consisting of:
   (xiii) an ALSD of formula FIIa as shown below: 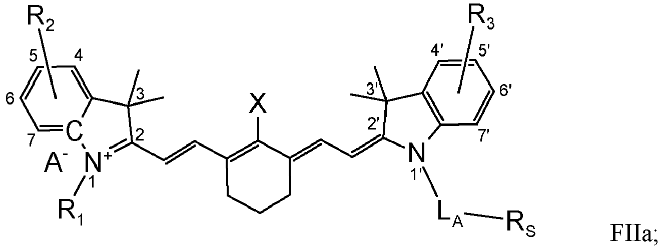
      wherein an amide linker L_{A} is linked to a statin residue R_{S} via an amide bond;
      wherein L_{A} is a linker selected from the group consisting of L_{A1} and L_{A2}, wherein L_{A1} is-(CH₂)ₙ-NH-, and wherein L_{A2} is -(CH₂)ₙ-CO-NH-(CH₂)ₘ-NH- and wherein n=4-9, and m=1-4;
      wherein Rs is a residue of a statin or of a statin derivative,
      wherein R_{S} comprises a dihydroxyheptanoic acid unit (DHHA) that is connected to residue R_{S*} which is the remainder of the statin,
      wherein Rs is linked to L_{A} via its DHHA in its open chain form which is -CO-CH₂-COH-CH₂-CHOH-R_{S*},
      and wherein A⁻, X, R₁, R₂, R₃, and n and m of the L_{A2} linker, are as defined in (i) for FIa;
   (xiv) an ALSD of formula FIIb as shown below, wherein the statin is Simvastatin and the linker is L_{A2} (-(CH₂)ₙ-CO-NH-(CH₂)ₘ-NH-): 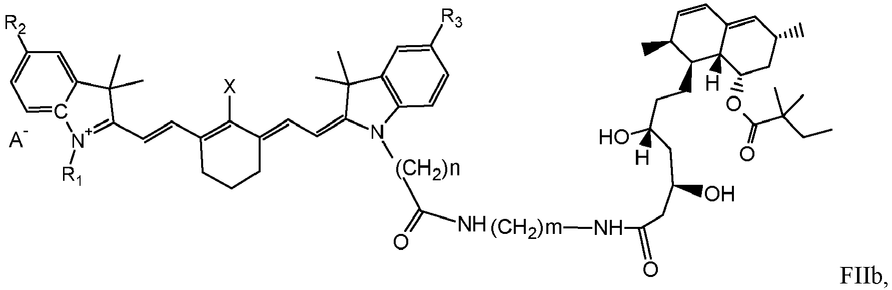 and wherein A⁻, X, R₁, R₂, R₃, and n and m of the L_{A2} linker, are as defined in (i) for FIa.
Clause 11: The method of clause 10, wherein the one or more ELSD and the one or more ALSD are co-administered to the patient in a coordinated administration schedule of one or more combined dosage forms, wherein each dosage form comprises the one or more ELSD and the one or more ALSD and one or more pharmaceutical excipient.
Clause 12: The method of clause 10, wherein the one or more ELSD is co-administered in a coordinated administration schedule with the one or more ALSD;
   wherein the schedule includes administration of a loading dose administered at least one hour or more prior to administration of one or more maintenance dose;
   wherein the loading dose consists of a separate dosage form that comprises the one or more ELSD and one or more pharmaceutical excipient, and does not comprise the one or more ALSD;
   and wherein the one or more maintenance dose consists of a dosage form that comprises the one or more ALSD and one or more pharmaceutical excipient, and optionally comprises the ELSD.
Clause 13: The method of clause 10, wherein the one or more ELSD is co-administered in a coordinated administration schedule together with one or more secondary drug, and wherein the one or more secondary drug is selected from the group consisting of: a hormonal antagonist, an anti-androgenic drug, Abiraterone, Enzalutamid, a chemotherapeutic drug, Docetaxel, Paclitaxel, and Cabazitaxel.
Clause 14: The method of clause 10, wherein one or more combined dosage form, or one or more separate dosage forms of a loading dose and one or more maintenance doses, are co-administered in a coordinated administration schedule;
   wherein the combined dosage form comprises the one or more ELSD, the one or more ALSD, and the one or more pharmaceutical excipient,
   wherein the loading dose consists of a separate dosage form that comprises the one or more ELSD and one or more pharmaceutical excipient, and does not comprise the one or more ALSD;
   and wherein the one or more maintenance dose consists of a dosage form that comprises the one or more ALSD and one or more pharmaceutical excipient, and optionally comprises the ELSD;
   wherein the co-administered dosage forms are further co-administered in a coordinated administration schedule together with one or more secondary drug,
   and wherein the one or more secondary drug is selected from the group consisting of:
   a hormonal antagonist, an anti-androgenic drug, Abiraterone, Enzalutamid, a chemotherapeutic drug, Docetaxel, Paclitaxel, and Cabazitaxel.
Clause 15: The method of clause 10, wherein the one or more ELSD is administered to a patient whose cancer cells, pre-cancerous lesions, tissues, tumors or metastases are identified to carry one or more genetic aberration in one or more gene encoding for one or more tyrosine kinase receptor, selected from the group comprising: epidermal growth factor receptor tyrosine kinase (EGFR), Anaplastic lymphoma kinase receptor (ALF), and Proto-oncogene tyrosine-protein kinase (ROS or ROS1).
Clause 16: The method of clause 10, wherein the one or more ELSD is administered to a patient whose cancer cells, pre-cancerous lesions, tumors or metastases have acquired resistance to one or more tyrosine kinase inhibitor (TKI), including a patient who received prior TKI treatment with one or more TKI prior to ELSD administration and whose response to the prior TKI treatment is therapeutically insufficient.
Clause 17: The method of clause 16, wherein the TKI is selected from the group consisting of an epidermal growth factor receptor tyrosine kinase inhibitor (EGFR-TKI), an ALK tyrosine kinase receptor inhibitor (ALK-TKI), and an inhibitor to Proto-oncogene tyrosine-protein kinase ROS (ROS-TKI).
Clause 18: The method of clause 16, wherein the EGFR-TKI is selected from the group consisting of: Gefitinib, Icotinib, Erlotinib, Brigatinib, Dacomitinib, Lapatinib, Vandetanib, Afatinib, Osimertinib (AZD9291), CO-1686, HM61713, Nazartinib (EGF816), Olmutinib, PF-06747775, YH5448, Avitinib (AC0010), Rociletinib, and Cetuximab.
Clause 19: The method of clause 10, wherein the patient is suffering from a drug-resistant cancer as determined by drug exposure or genetic testing, the drug-resistant cancer selected from the group comprising: prostate cancer, pancreatic cancer, lung cancer, non-small cell lung carcinoma (NSCLC; NSCLC may include squamous-cell carcinoma, adenocarcinoma (mucinous cystadenocarcinoma), large-cell lung carcinoma, rhabdoid carcinoma, sarcomatoid carcinoma, carcinoid, salivary gland-like carcinoma, adenosquamous carcinoma, papillary adenocarcinoma, giant-cell carcinoma), SCLC (small cell lung carcinoma), combined small-cell carcinoma, non-carcinoma cancers of the lung (sarcoma, lymphoma, immature teratoma, and melanoma), kidney cancer, lymphoma, colorectal cancer, skin cancer, HCC cancer, and breast cancer, squamous-cell carcinoma of the lung, anal cancers, glioblastoma, epithelian tumors of the head and neck, and other cancers.
Clause 20: The method of clause 10, wherein the patient is a patient suffering from a drug-resistant lung cancer as determined by drug exposure or genetic testing, the drug resistant lung cancer selected from the group comprising: small cell carcinoma lung cancer (SCCLC), non-small cell lung carcinoma (NSCLC), combined small-cell carcinoma, squamous-cell carcinoma, adenocarcinoma (AC, mucinous cystadenocarcinoma, MCACL), large-cell lung carcinoma, rhabdoid carcinoma, sarcomatoid carcinoma, carcinoid, salivary gland-like carcinoma, adenosquamous carcinoma, papillary adenocarcinoma, giant-cell carcinoma, non-carcinoma cancer of the lung, sarcoma, lymphoma, immature teratoma, and melanoma.

## Claims

1. An ester-linked statin derivative shown in formula FIa below: 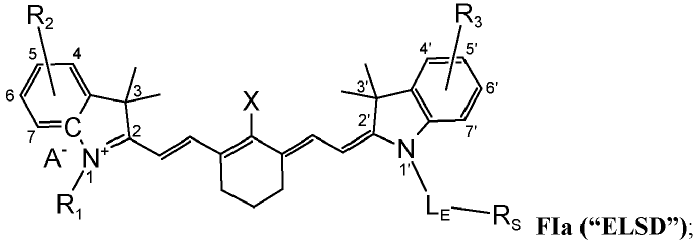
wherein X is a halogen residue,
wherein R₁ is a residue selected from the group consisting of C₁-C₂₅ alkyl, C₅-C₂₅ aryl, C₅-C₂₅ indolyl, C₅-C₂₅ thienyl, C₅-C₂₅ phenyl, C₅-C₂₅ napththyl, C₁-C₂₅ aralkyl, C₁-C₂₅ alkylsulphonate, C₁-C₂₅ alkylcarboxyl, C₁-C₂₅ alkylamino, C₁-C₂₅ ω-alkylaminium, C₁-C₂₅ ω-alkynyl, a PEGyl polyethylene chain with (-CH₂-CH₂-O-)₂₋₂₀, a PEGylcarboxylate with (-CH₂-CH₂-O-)₂₋₂₀, a ω-PEGylaminium with (-CH₂-CH₂-O-)₂₋₂₀, a ω-acyl-NH, a ω-acyl-lysinyl-, a ω-acyl-triazole, a ω-PEGylcarboxyl-NH- with (-CH₂-CH₂-O-)₂₋₂₀, a ω-PEGylcarboxyl-lysinyl with (-CH₂-CH₂-O-)₂₋₂₀, and a ω-PEGylcarboxyl-triazole with (-CH₂-CH₂-O-)₂₋₂₀;
wherein R₂ and R₃ are residues independently selected from the group consisting of: a hydrogen, an C₁-C₂₀ alkyl, a sulphonate, a C₁-C₂₀ alkylcarboxyl, C₁-C₂₀ alkylamino, C₁-C₂₀ aryl, -SO₃H, -PO₃H, -OH, -NH₂, and a halogen residue;
and wherein R₂ and R₃ are attached at a ring position selected from the group consisting of 3, 3', 4, 4', 5, 5', 6, and 6';
wherein the A⁻ group is a pharmaceutically acceptable negatively charged anion;
wherein an ester linker L_{E} is linked to a statin residue R_{S} by an ester bond;
wherein L_{E} is selected from the group consisting of L_{E1} and L_{E2}, wherein L_{E1} is-(CH₂)ₙ-O-, and wherein L_{E2} is -(CH₂)ₙ-CO-NH-(CH₂)ₘ-O-, and wherein n=4-9, and m=1-4;
wherein Rs is a residue of a statin or of a statin derivative,
wherein R_{S} comprises a dihydroxyheptanoic acid unit (DHHA) that is connected to residue R_{S*} which is the remainder of the statin,
and wherein Rs is linked to L_{E} via its DHHA in its open chain form which is -CO-CH₂-COH-CH₂-CHOH-R_{S*}.

2. An ester-linked statin derivative as claimed in claim 1, wherein the statin is selected from the group consisting of: Simvastatin, Mevastatin, Lovastatin, Pravastatin, Atorvastatin, Fluvastatin, Rosuvastatin, Cerivastatin, Pitavastatin, and derivatives thereof which retain binding to the statin's active binding site.

3. An ester-linked statin derivative as claimed in claim 1, wherein the statin is Simvastatin and wherein the ester linker L_{E} is L_{E2}, as shown in Formula FIb below: 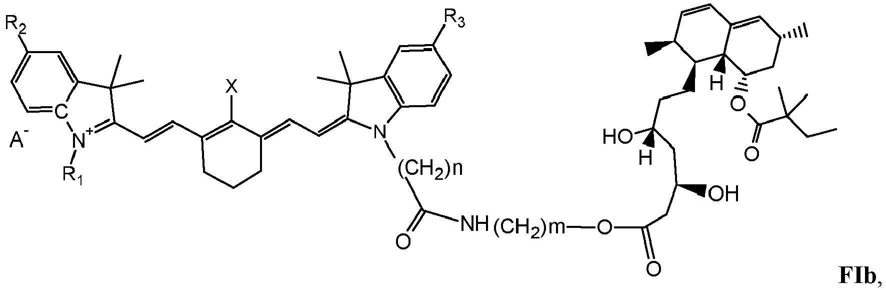 and wherein A⁻, X, R₁, R₂, R₃, and n and m of the L_{E2} linker are defined as for FIa.

4. An ester-linked statin derivative as claimed in any one of claims 1 to 3, wherein X is Cl.

5. An ester-linked statin derivative as claimed in any one of claims 1 to 4, wherein R₁ is a -(CH₂)n-SO₃⁻ alkylsulphonate residue, and wherein n of R₁ is selected from 2, 3, 4, 5, 6, 7 and 8.

6. An ester-linked statin derivative as claimed in any one of claims 1 to 5, wherein R₂ and R₃ are H.

7. An ester-linked statin derivative as claimed in any one of claims 1 to 6, wherein an ELSD and one or more pharmaceutical excipient is comprised within a pharmaceutical composition.

8. An ester-linked statin derivative as claimed in any one of claims 1 to 7 in the form of a composition comprising one or more ELSD and further comprising an ALSD formula FIIa below: 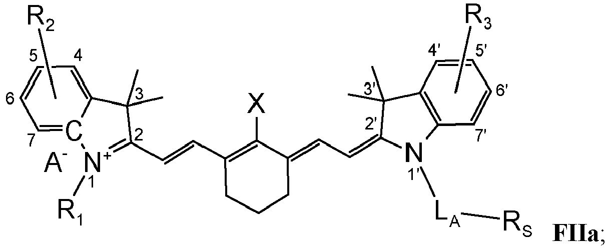
wherein X is a halogen residue;
wherein R₁ is a residue selected from the group consisting of C₁-C₂₅ alkyl, C₅-C₂₅ aryl, C₅-C₂₅ indolyl, C₅-C₂₅ thienyl, C₅-C₂₅ phenyl, C₅-C₂₅ napththyl, C₁-C₂₅ aralkyl, C₁-C₂₅ alkylsulphonate, C₁-C₂₅ alkylcarboxyl, C₁-C₂₅ alkylamino, C₁-C₂₅ ω-alkylaminium, C₁-C₂₅ ω-alkynyl, a PEGyl polyethylene chain with (-CH₂-CH₂-O-)₂₋₂₀, a PEGylcarboxylate with (-CH₂-CH₂-O-)₂₋₂₀, a ω-PEGylaminium with (-CH₂-CH₂-O-)₂₋₂₀, a ω-acyl-NH, a ω-acyl-lysinyl-, a ω-acyl-triazole, a ω-PEGylcarboxyl-NH- with (-CH₂-CH₂-O-)₂₋₂₀, a ω-PEGylcarboxyl-lysinyl with (-CH₂-CH₂-O-)₂₋₂₀, and a ω-PEGylcarboxyl-triazole with (-CH₂-CH₂-O-)₂₋₂₀;
wherein R₂ and R₃ are residues independently selected from the group consisting of: a hydrogen, an C₁-C₂₀ alkyl, a sulphonate, a C₁-C₂₀ alkylcarboxyl, C₁-C₂₀ alkylamino, C₁-C₂₀ aryl, -SO₃H, -PO₃H, -OH, -NH₂, and a halogen residue;
wherein R₂ and R₃ are attached at a ring position selected from the group consisting of 3, 3', 4, 4', 5, 5', 6, and 6';
wherein the A⁻ group is a pharmaceutically acceptable negatively charged anion;
wherein an amide linker L_{A} is linked to a statin residue R_{S} via an amide bond;
wherein L_{A} is a linker selected from the group consisting of L_{A1} and L_{A2}, wherein L_{A1} is -(CH₂)ₙ-NH-, and wherein L_{A2} is -(CH₂)ₙ-CO-NH-(CH₂)ₘ-NH- and wherein n=4-9, and m=1-4;
wherein Rs is a residue of a statin or of a statin derivative,
wherein R_{S} comprises a dihydroxyheptanoic acid unit (DHHA) that is connected to residue R_{S*} which is the remainder of the statin,
and wherein Rs is linked to L_{A} via its DHHA in its open chain form which is -CO-CH₂-COH-CH₂-CHOH-R_{S*}.

9. An ester-linked statin derivative as defined in any one of claims 1 to 8 for use in treating cancer, wherein one or more ELSD, and optionally one or more ALSD, are prepared to be administered to a patient in need thereof in amounts sufficient to inhibit cancer cell or pre-cancerous cell growth or to induce apoptosis in cancer or pre-cancerous cells in the patient.

10. An ester-linked statin derivative as claimed in claim 9, wherein the one or more ELSD and the one or more ALSD are prepared to be co-administered to the patient in a coordinated administration schedule of one or more combined dosage forms, wherein each dosage form comprises the one or more ELSD and the one or more ALSD and optionally one or more pharmaceutical excipient.

11. An ester-linked statin derivative as claimed in claim 9, wherein the one or more ELSD is prepared to be co-administered in a coordinated administration schedule with the one or more ALSD;
wherein the schedule includes administration of a loading dose administered at least one hour or more prior to administration of one or more maintenance dose;
wherein the loading dose consists of a separate dosage form that comprises the one or more ELSD and optionally one or more pharmaceutical excipient, and does not comprise the one or more ALSD;
and wherein the one or more maintenance dose consists of a dosage form that comprises the one or more ALSD and optionally one or more pharmaceutical excipient, and optionally comprises the ELSD.

12. An ester-linked statin derivative as claimed in any one of claims 9 to 11, wherein the one or more ELSD is prepared to be co-administered in a coordinated administration schedule together with one or more secondary drug, and wherein the one or more secondary drug is selected from the group consisting of: a hormonal antagonist, an anti-androgenic drug, Abiraterone, Enzalutamid, a chemotherapeutic drug, Docetaxel, Paclitaxel, and Cabazitaxel.

13. An ester-linked statin derivative as claimed in any one of claims 9 to 12, for use in one or more of:
(i) a patient whose cancer cells, pre-cancerous lesions, tissues, tumors or metastases carry one or more genetic aberration in one or more gene encoding for one or more tyrosine kinase receptor, selected from the group comprising: epidermal growth factor receptor tyrosine kinase (EGFR), Anaplastic lymphoma kinase receptor (ALF), and Proto-oncogene tyrosine-protein kinase (ROS or ROS1);
(ii) a patient whose cancer cells, pre-cancerous lesions, tumors or metastases have acquired resistance to one or more tyrosine kinase inhibitor (TKI);
(iii) a patient who received prior TKI treatment with one or more TKI prior to ELSD administration and whose response to the prior TKI treatment is therapeutically insufficient; and
(iv) a patient who is suffering from a drug-resistant cancer.

14. An ester-linked statin derivative as claimed in claim 13, wherein the TKI is selected from the group consisting of:
(i) an epidermal growth factor receptor tyrosine kinase inhibitor (EGFR-TKI),
(ii) an ALK tyrosine kinase receptor inhibitor (ALK-TKI),
(iii) an inhibitor to Proto-oncogene tyrosine-protein kinase ROS (ROS-TKI), and
(iv) an EGFR-TKI, wherein the EGFR-TKI is selected from the group consisting of: Gefitinib, Icotinib, Erlotinib, Brigatinib, Dacomitinib, Lapatinib, Vandetanib, Afatinib, Osimertinib (AZD9291), CO-1686, HM61713, Nazartinib (EGF816), Olmutinib, PF-06747775, YH5448, Avitinib (AC0010), Rociletinib, and Cetuximab.

15. An ester-linked statin derivative as claimed in any one of claims 9 to 14, wherein the patient is suffering from a drug-resistant cancer, the drug-resistant cancer selected from the group comprising: prostate cancer, pancreatic cancer, lung cancer, non-small cell lung carcinoma (NSCLC; NSCLC may include squamous-cell carcinoma, adenocarcinoma (mucinous cystadenocarcinoma), large-cell lung carcinoma, rhabdoid carcinoma, sarcomatoid carcinoma, carcinoid, salivary gland-like carcinoma, adenosquamous carcinoma, papillary adenocarcinoma, giant-cell carcinoma), SCLC (small cell lung carcinoma), combined small-cell carcinoma, non-carcinoma cancers of the lung (sarcoma, lymphoma, immature teratoma, and melanoma), kidney cancer, lymphoma, colorectal cancer, skin cancer, HCC cancer, and breast cancer, squamous-cell carcinoma of the lung, anal cancers, glioblastoma, epithelian tumors of the head and neck, a drug resistant lung cancer, a drug resistant lung cancer selected from the group comprising: small cell carcinoma lung cancer (SCCLC), non-small cell lung carcinoma (NSCLC), combined small-cell carcinoma, squamous-cell carcinoma, adenocarcinoma (AC, mucinous cystadenocarcinoma, MCACL), large-cell lung carcinoma, rhabdoid carcinoma, sarcomatoid carcinoma, carcinoid, salivary gland-like carcinoma, adenosquamous carcinoma, papillary adenocarcinoma, giant-cell carcinoma, non-carcinoma cancer of the lung, sarcoma, lymphoma, immature teratoma, and melanoma, and other cancers.
